# EUROPEAN PATENT APPLICATION

(11) **EP 4 365 177 A1**
(43) Date of publication of application: **08.05.2024**
(21) Application number: 22830373.1
(22) Date of filing: 20.06.2022
(51) Int. Cl.: C07D 519/00, C08G 61/12

(54) **ORGANIC PHOTODETECTOR, AND ORGANIC SEMICONDUCTOR COMPOUND USED FOR SAME**

(30) Priority: 29.06.2021 JP 2021108045
(71) Applicant: TOYOBO CO., LTD., Osaka-shi, Osaka 530-0001 (JP)
(72) Inventor: TOYAMA, Taichi, Otsu-shi, Shiga 520-0292 (JP); IMANISHI, Yoshiki, Otsu-shi, Shiga 520-0292 (JP); TANAKA, Hikaru, Otsu-shi, Shiga 520-0292 (JP); HAGIYA, Kazutake, Otsu-shi, Shiga 520-0292 (JP)
(74) Representative: Müller-Boré & Partner Patentanwälte PartG mbB
(86) International application number: PCT/JP2022/024509
(87) International publication number: WO 2023/276753

(57) **Abstract**

It is provided that an organic photodetector that exhibits high conversion efficiency. An organic photodetector comprising a structure in which a base material, a cathode, an active layer, and an anode are disposed in order, respectively, wherein the active layer includes a polymer compound having a benzo(bis)thiazole structural unit represented by chemical formula (1), 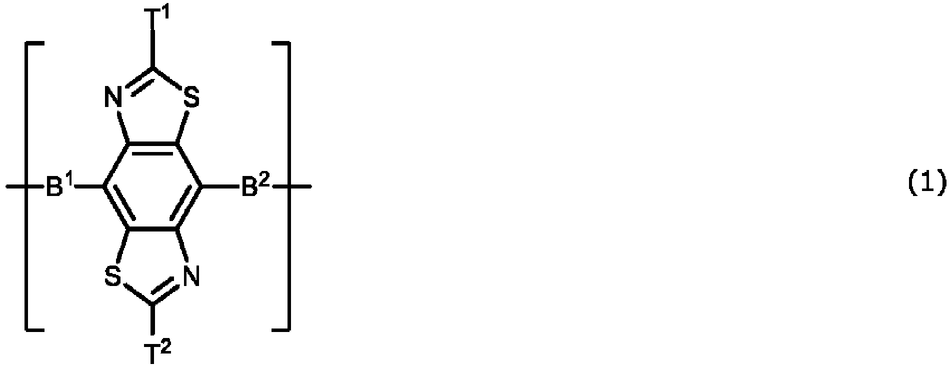 [in formula (1), T¹ and T² each independently represent an alkoxy group, a thioalkoxy group, a thiophene ring that is optionally substituted with a hydrocarbon group or an organosilyl group, a thiazole ring that is optionally substituted with a hydrocarbon group or an organosilyl group, or a phenyl group that is optionally substituted with a hydrocarbon group, an alkoxy group, a thioalkoxy group, an organosilyl group, a halogen atom, or a trifluoromethyl group, and B¹ and B² each represent a thiophene ring that is optionally substituted with a hydrocarbon group, a thiazole ring that is optionally substituted with a hydrocarbon group, or an ethynylene group.].

## Description

### TECHNICAL FIELD

The present invention relates to an organic photodetector that has a structure in which a base material, a cathode, an active layer, and an anode are disposed in order, respectively, and that contains a polymer compound having a specific benzo(bis)thiazole backbone structural unit.

### BACKGROUND ART

Organic semiconductor compounds are among the most important materials in the field of organic electronics, and can be classified into electron-donating p-type organic semiconductor compounds and electron-accepting n-type organic semiconductor compounds. Various elements can be produced by appropriately combining p-type organic semiconductor compounds and n-type organic semiconductor compounds, and these elements are applied to, for example, organic electroluminescence that allows light emission by the action of excitons formed by recombination of electrons and holes, organic photodetectors and organic thin-film solar cells which convert light into electric power, and organic thin-film transistors which control a current amount and voltage.

Among them, an organic photodetector can detect minute light having various wavelengths, and is thus useful for, for example, health care (sensing in blood, etc.), security (various authentications), and logistics (article management, etc.). Furthermore, an organic photodetector has a simple structure and can be easily produced, and can be downsized and can be formed with flexibility. Therefore, the organic photodetectors can be set with a high degree of freedom and are unlikely to be broken, and are thus excellent. Non-Patent Literature 1 discloses an organic photodetector in which poly(3-hexylthiophene-2,5-diyl) is used as a general-purpose p-type organic semiconductor.

### CITATION LIST

### NON PATENT LITERATURE

[NPL 1] ACS Applied Materials & Interfaces, 2018, the 10th edition, pp. 42733-42739

### SUMMARY OF THE INVENTION

### PROBLEMS TO BE SOLVED BY THE INVENTION

However, in the organic photodetector disclosed in Non-Patent Literature 1, a dark current is 1×10⁻² mA/cm² (at -2 V), and it cannot be said that this is a satisfactory level. The dark current refers to a current density Jd (mA/cm²) at a time when a reverse voltage is applied in a state where light is not applied. Reduction of the dark current is one of important factors for enhancing a sensitivity. This requires a chemical structure of the above-described p-type semiconductor compound which absorbs light to cause excitation to be made appropriate.

An object of the present invention is to provide an organic photodetector that exhibits high conversion efficiency. The ability of the organic photodetector depends on kinds and combination of organic semiconductor compounds, a structure of the organic photodetector, and the like, and is closely related to the chemical structure in the organic semiconductor compound. Therefore, an object of the present invention is to provide an organic photodetector that has a polymer compound into which a wider variety of backbones and substituents can be introduced. In recent years, as described in Non-Patent Literature 1, a reverse-structure type element having an electrode (cathode) for collecting electrons on the base material side because of high stability, has attracted attention, and the element structure needs to be also examined.

### SOLUTION TO THE PROBLEMS

The inventors of the present invention have found that it is useful to make the chemical structure of the organic semiconductor appropriate in order to reduce the dark current. As a result of thorough studies in which attention is focused on correlation between a dark current and a chemical structure in the organic semiconductor compound, it has been found that the dark current is reduced by using an organic semiconductor polymer having a specific structure. The inventors have found that charge separation between a p-type organic semiconductor and an n-type organic semiconductor can be easily caused by using such an organic semiconductor polymer, and have completed the present invention.

That is, the present invention is directed to an organic photodetector that includes a structure in which a base material, a cathode, an active layer, and an anode are disposed in order, respectively, and the active layer includes a polymer compound (hereinafter, may be referred to as "polymer compound (1)") having a specific benzo(bis)thiazole backbone structural unit represented by formula (1), 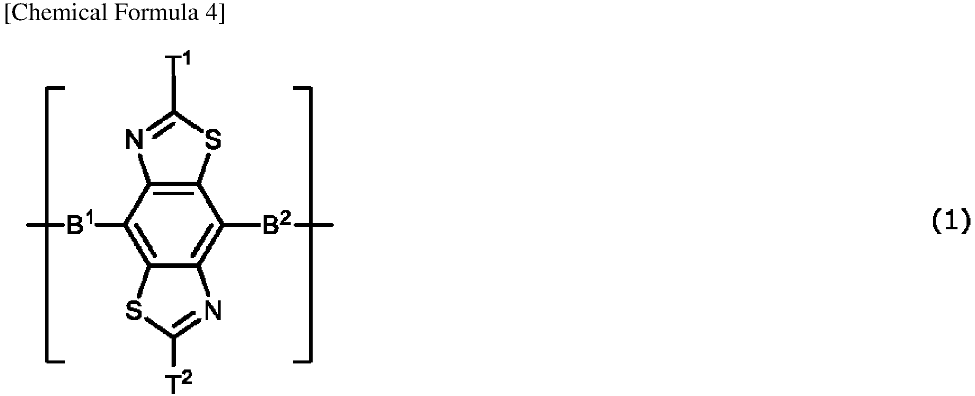 [in formula (1),
T¹ and T² each independently represent an alkoxy group, a thioalkoxy group, a thiophene ring that is optionally substituted with a hydrocarbon group or an organosilyl group, a thiazole ring that is optionally substituted with a hydrocarbon group or an organosilyl group, or a phenyl group that is optionally substituted with a hydrocarbon group, an alkoxy group, a thioalkoxy group, an organosilyl group, a halogen atom, or a trifluoromethyl group, and
B¹ and B² each represent a thiophene ring that is optionally substituted with a hydrocarbon group, a thiazole ring that is optionally substituted with a hydrocarbon group, or an ethynylene group.].

Preferably, in the chemical formula (1), T¹ and T² of the polymer compound each represent a group represented by one of the following formulas (t1) to (t5). 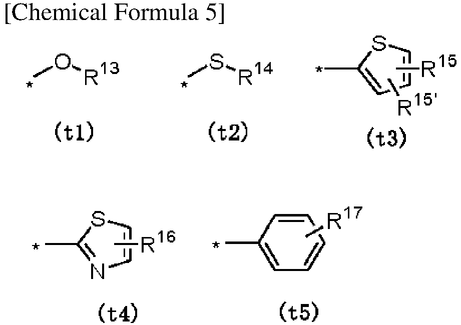 [in formulas (t1) to (t5),
R¹³ to R¹⁴ each independently represent a C6 to C30 hydrocarbon group,
R¹⁵ to R¹⁶ each independently represent a C6 to C30 hydrocarbon group or a group represented by *-Si(R¹⁸)₃, and R^{15'} represents a hydrogen atom, a C6 to C30 hydrocarbon group, or a group represented by *-Si(R¹⁸)₃, R¹⁷ each independently represent a C6 to C30 hydrocarbon group, *-Q-R¹⁹, *-S-R²⁰, *-Si(R¹⁸)₃, or *-CF₃,
R¹⁸ each independently represent a C1 to C20 aliphatic hydrocarbon group or a C6 to C10 aromatic hydrocarbon group, and a plurality of R¹⁸s may be the same or different from each other,
R¹⁹ to R²⁰ each represent a C6 to C30 hydrocarbon group, and
* represents a bond moiety for bonding to a thiazole ring of benzo(bis)thiazole.].

Preferably, in the chemical formula (1), B¹ and B² each represent a group represented by one of the following formulas (b1) to (b3). 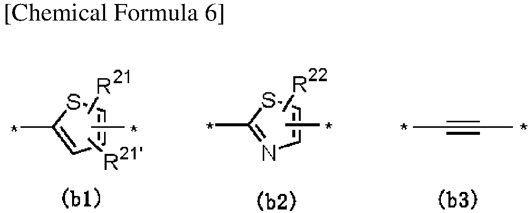 [in formulas (b1) to (b3),
R²¹, R²², and R^{21'} each represent a hydrogen atom or a C6 to C30 hydrocarbon group, * represents a bond moiety, and, in particular, * on a left side represents a bond moiety for bonding to a benzene ring of a benzo(bis)thiazole compound.].

The polymer compound (1) is preferably a donor-acceptor semiconductor polymer. Organic semiconductor material containing the polymeric compound (1) is also included in the technical scope of the present invention.

The active layer further preferably includes an n-type organic semiconductor compound. The n-type organic semiconductor compound is preferably a fullerene or a derivative thereof.

In the organic photodetector of the present invention, an electron transport layer is preferably disposed between the cathode and the active layer, and a hole transport layer is preferably disposed between the anode and the active layer. The cathode is a preferably transparent electrode. The anode is preferably a metal electrode.

### ADVANTAGEOUS EFFECTS OF THE INVENTION

The polymer compound (1) used in the present invention can form a planar cross-shaped backbone under the intramolecular S-N interaction. As a result, a π-conjugation is extended in the planar cross-shaped backbone, and therefore, multi-band light absorptions derived from a plurality of π-π* transitions are enabled, and reaction can be efficiently caused even with minute light. By optimizing a packing structure resulting from the planar cross-shaped backbone, the dark current can be reduced. Various substituents can be introduced into the benzo(bis)thiazole backbone constituting the polymer compound (1) used in the present invention, as substituents, and characteristics (crystallinity, film formability, absorption wavelength), of materials, which exert various influences on organic photodetector characteristics can be controlled. As the element structure, a reverse-structure type element in which electrons are collected on the substrate side can be produced.

### BRIEF DESCRIPTION OF THE DRAWINGS

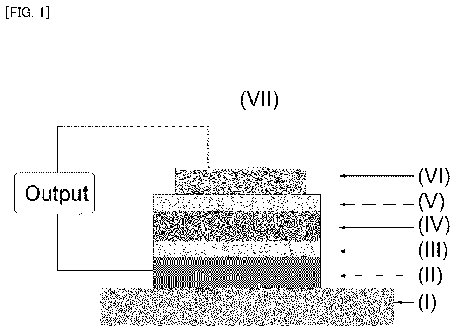

[FIG. 1] FIG. 1 illustrates an element structure of an organic photodetector in which a base material, a cathode, an active layer, and an anode are disposed in order, respectively (reverse-structure type).

### DESCRIPTION OF EMBODIMENTS

An embodiment of the present invention will be described below in detail. The structural requirements described below indicate one example (representative example) of an embodiment of the present invention, and the present invention is not limited to the contents of the example as long as the present invention does not depart from the gist thereof.

### < 1. Organic photodetector>

An organic photodetector according to the present invention is an organic photodetector having a structure in which a base material, a cathode, an active layer, and an anode are disposed in order, respectively, and the active layer contains a polymer compound having a benzo(bis)thiazole structural unit represented by formula (1).

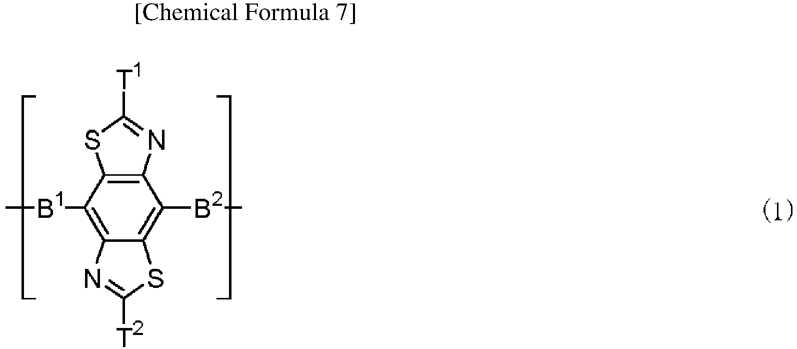

FIG. 1 shows an organic photodetector (VII) according to one embodiment of the present invention. FIG. 1 shows an organic photodetector used for a standard organic thin-film solar cell. However, the organic photodetector according to the present invention is not limited to the structure shown in FIG. 1.

The organic photodetector (VII) has a structure in which a base material (I), an electrode (cathode) (II), an active layer (IV), and an electrode (anode) (VI) are disposed in order, respectively. Preferably, the organic photodetector (VII) further includes a buffer layer (electron transport layer) (III) and a buffer layer (hole transport layer) (V). That is, the organic photodetector (VII) preferably has a structure in which the base material (I), the cathode (II), the buffer layer (electron transport layer) (III), the active layer (IV), the buffer layer (hole transport layer) (V), and the anode (VI) are disposed in order, respectively. However, the organic photodetector according to the present invention may not necessarily have the electron transport layer (III) and the hole transport layer (V). Each of these parts will be described below.

### <1.1 Active layer (IV)>

The active layer (IV) refers to a layer at which photoelectric conversion is performed, and typically includes one or a plurality of p-type semiconductor compounds and one or a plurality of n-type semiconductor compounds. Specific examples of the p-type semiconductor compound include, but are not limited to, the polymer compound (1) and an organic semiconductor compound (11) described below. In the present invention, as the p-type semiconductor compound, at least the polymer compound (1) needs to be used. When the organic photodetector (VII) receives light, the light is absorbed by the active layer (IV), electricity is generated at the interface between the p-type semiconductor compound and the n-type semiconductor compound, and the generated electricity is extracted from the cathode (II) and the anode (VI). In the present invention, the polymer compound (1) is used as the p-type semiconductor compound.

The film thickness of the active layer (IV) is, but is not particularly limited to, preferably 70 nm or more and more preferably 90 nm or more, and may be 200 nm or more. Meanwhile, the film thickness of the active layer (IV) is preferably 2000 nm or smaller, more preferably 1550 nm or smaller, and further preferably 1000 nm or smaller. Particularly, when the film thickness is between 500 nm and 600 nm, reduction of the dark current and exhibition of high photocurrent can be expected.

When the film thickness of the active layer (IV) is 70 nm or more, reduction of the dark current in the organic photodetector (VII) can be expected. The active layer (IV) having the film thickness of 70 nm or more is preferable also in that penetration and short-circuiting in the film can be prevented. The thickness of the active layer (IV) is preferably 2000 nm or smaller since the internal resistance is reduced, a distance between the electrodes (II) and (VI) is not excessively large, and charge diffusion becomes good. Furthermore, the active layer (IV) having the film thickness of 70 nm or more and 2000 nm or smaller, in particular, the active layer (IV) having the film thickness between 500 nm and 600 nm, is preferable in that reproducibility is enhanced in a process of producing the active layer (IV).

In general, the thicker the active layer is, the larger a distance over which charge generated in the active layer is transferred to an electrode, an electron transport layer, or a hole transport layer is, so that transport of the charge to the electrode is hindered. Thus, when the active layer (IV) is thick, although a region in which light can be absorbed is increased, transport of charge generated by the light absorption becomes difficult, so that photocurrent is reduced. Therefore, the film thickness of the active layer (IV) is preferably 70 nm or more and 2000 nm or smaller, and is particularly preferably between 500 nm and 600 nm.

### [1.1.1 Layer structure of active layer]

Examples of the layer structure of the active layer (IV) include a thin film laminate type structure in which the p-type semiconductor compound and the n-type semiconductor compound are stacked, and a bulk heterojunction type structure in which the p-type semiconductor compound and the n-type semiconductor compound are mixed. Among them, a bulk heterojunction type active layer is preferable from the viewpoint of further reducing the dark current.

### Bulk heterojunction type active layer

The bulk heterojunction type active layer has a layer (i layer) in which the p-type semiconductor compound and the n-type semiconductor compound are mixed. The i layer has a structure in which the p-type semiconductor compound and the n-type semiconductor compound are phase-separated, carrier separation occurs at the phase interface, and the generated carriers (holes and electrons) are transported to the electrode.

Usually 50 weight % or more, preferably 70 weight % or more, and more preferably 90 weight % or more of the p-type semiconductor compound included in the i layer is the polymer compound (1) that is formed of the benzo(bis)thiazole structural unit represented by formula (1) and a copolymerization component (2) described below. The polymer compound (1) has suitable properties as the p-type semiconductor compound, and thus, it is particularly preferable that the p-type semiconductor compound merely contains the polymer compound (1).

In the i layer, a weight ratio (the p-type semiconductor compound/the n-type semiconductor compound) of the p-type semiconductor compound to the n-type semiconductor compound is preferably 0.5 or more and more preferably 1 or more, and preferably 4 or smaller, more preferably 3 or smaller, and particularly preferably 2 or smaller from the viewpoint that the dark current is reduced by obtaining a suitable phase-separated structure.

The i layer can be formed by any method including an application method and a vapor deposition method (for example, co-vapor deposition method). The application method is preferably used since the i layer can be more easily formed. The polymer compound (1) according to the present invention is preferable since the polymer compound (1) has solubility with respect to a solvent, and thus has excellent coatability and film formability. In a case where the i layer is formed by an application method, a coating solution containing the p-type semiconductor compound and the n-type semiconductor compound may be prepared and the prepared coating solution may be applied. The coating solution containing the p-type semiconductor compound and the n-type semiconductor compound may be produced by preparing and thereafter mixing a solution containing the p-type semiconductor compound and a solution containing the n-type semiconductor compound, or by dissolving the p-type semiconductor compound and the n-type semiconductor compound in a solvent described below.

The total of concentrations of the p-type semiconductor compound and the n-type semiconductor compound in the coating solution is, but is not particularly limited to, preferably 1.0 weight % or more with respect to the entire coating solution from the viewpoint of forming the active layer having a sufficient film thickness, and preferably 10.0 weight % or smaller with respect to the entire coating solution from the viewpoint of sufficiently dissolving the semiconductor compounds.

Any application method can be used. Examples of the application method include a spin coating method, an ink jet method, a doctor blade method, a drop casting method, a reverse roll coating method, a gravure coating method, a kiss coating method, a roll brushing method, a spray coating method, an air-knife coating method, a wire bar coating method, a pipe doctor method, an impregnating- coating method, a curtain coating method, and a flexo coating method. After the coating solution is applied, a drying process through heating or the like may be performed.

A solvent of the coating solution is not particularly limited as long as the p-type semiconductor compound and the n-type semiconductor compound can be uniformly dissolved. Examples of the solvent include: aliphatic hydrocarbons such as hexane, heptane, octane, isooctane, nonane, and decane; aromatic hydrocarbons such as toluene, xylene, mesitylene, cyclohexylbenzene, chlorobenzene, and ortho-dichlorobenzene; alicyclic hydrocarbons such as cyclopentane, cyclohexane, methylcyclohexane, cycloheptane, cyclooctane, tetralin, and decalin; lower alcohols such as methanol, ethanol, propanol, and anisole; aliphatic ketones such as acetone, methyl ethyl ketone, methyl isobutyl ketone, cyclopentanone, and cyclohexanone; aromatic ketones such as acetophenone and propiophenone; esters such as ethyl acetate, isopropyl acetate, butyl acetate, and methyl lactate; halogen hydrocarbons such as chloroform, methylene chloride, dichloroethane, trichloroethane, and trichloroethylene; ethers such as ethyl ether, tetrahydrofuran, cyclopentyl methyl ether, dibutyl ether, diphenyl ether, and dioxane; and amides such as dimethylformamide, NMP, DMI, and dimethylacetamide.

Among them, aromatic hydrocarbons such as toluene, xylene, mesitylene, cyclohexylbenzene, chlorobenzene, and ortho-dichlorobenzene; alicyclic hydrocarbons such as cyclopentane, cyclohexane, methylcyclohexane, cycloheptane, cyclooctane, tetralin, and decalin; ketones such as acetone, methyl ethyl ketone, cyclopentanone, and cyclohexanone; and ethers such as ethyl ether, tetrahydrofuran, and dioxane are preferable.

In a case where a bulk heterojunction type active layer is formed by the application method, an additive may be further added to the coating solution containing the p-type semiconductor compound and the n-type semiconductor compound. A phase-separated structure of the p-type semiconductor compound and the n-type semiconductor compound in the bulk heterojunction type active layer exerts an influence on a light absorbing process, an exciton diffusion process, an exciton dissociation (carrier separation) process, a carrier transport process, and the like. Therefore, the dark current characteristics are considered to become good by optimizing the phase-separated structure. In a case where the coating solution contains an additive having a high affinity to the p-type semiconductor compound or the n-type semiconductor compound, an active layer having a preferable phase-separated structure is obtained, and the dark current can thus be reduced.

The additive is preferably a solid or has a high boiling point from the viewpoint of inhibiting the additive from being removed from the active layer (IV).

Specifically, in a case where the additive is a solid, the melting point (one atmosphere) of the additive is usually 35°C or higher, preferably 50°C or higher, more preferably 80°C or higher, further preferably 150°C or higher, and particularly preferably 200°C or higher.

In a case where the additive is a liquid, the boiling point (one atmosphere) is 80°C or higher, more preferably 100°C or higher, and particularly preferably 150°C or higher.

In a case where the additive is a solid, examples of the additive include aliphatic hydrocarbons or aromatic compounds each having 10 or more carbon atoms. Specific examples of the additive include naphthalene compounds, and a compound in which a substituent is bonded to naphthalene and the number of the substituents is 1 or more and 8 or smaller, is particularly preferable. Examples of the substituent bonded to naphthalene include a halogen atom, a hydroxy group, a cyano group, an amino group, an amide group, a carbonyloxy group, a carboxyl group, a carbonyl group, an oxycarbonyl group, a silyl group, an alkenyl group, an alkynyl group, an alkoxy group, an aryloxy group, an alkylthio group, an arylthio group, and an aromatic group.

In a case where the additive is a liquid, examples of the additive include aliphatic hydrocarbons or aromatic compounds each having eight or more carbon atoms. Specific examples of the additive include dihalogen hydrocarbon compounds. Particularly, a compound in which a substituent is bonded to octane and the number of the substituents is 1 or more and 8 or smaller, is preferable. Examples of the substituent bonded to octane include a halogen atom, a hydroxy group, a thiol group, a cyano group, an amino group, an amide group, a carbonyloxy group, a carboxyl group, a carbonyl group, and an aromatic group. Other examples of the additive include benzene compounds in which halogen atoms are bonded and the number of the halogen atoms is 4 or more and 6 or smaller.

An amount of the additive that is contained in the coating solution containing the p-type semiconductor compound and the n-type semiconductor compound is preferably 0.1 weight % or more and more preferably 0.5 weight % or more with respect to the entire coating solution. Meanwhile, the amount of the additive is preferably 10 weight % or smaller and more preferably 5 weight % or smaller with respect to the entire coating solution. In a case where the amount of the additive is in this range, a preferable phase-separated structure can be obtained.

### [1.1.2 p-type semiconductor compound]

The active layer (IV) contains at least the polymer compound (1) as the p-type semiconductor compound.

### (Polymer compound (1))

A polymer compound (hereinafter, may be referred to as "polymer compound (1)") used in the organic photodetector of the present invention is one kind of the p-type semiconductor compound, and has a benzo(bis)thiazole structural unit (hereinafter, may be referred to as "structural unit represented by formula (1)") represented by formula (1). 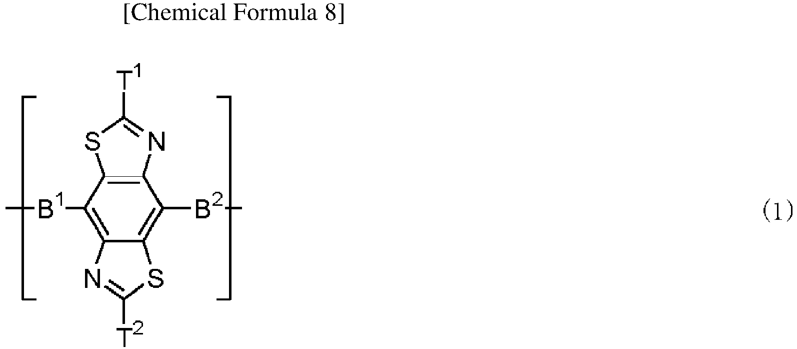 [In formula (1),
T¹ and T² each independently represent an alkoxy group, a thioalkoxy group, a thiophene ring that is optionally substituted with a hydrocarbon group or an organosilyl group, a thiazole ring that is optionally substituted with a hydrocarbon group or an organosilyl group, or a phenyl group that is optionally substituted with a hydrocarbon group, an alkoxy group, a thioalkoxy group, an organosilyl group, a halogen atom, or a trifluoromethyl group.
B¹ and B² each represent a thiophene ring that is optionally substituted with a hydrocarbon group, a thiazole ring that is optionally substituted with a hydrocarbon group, or an ethynylene group.]

The polymer compound used in the present invention has the benzo(bis)thiazole structural unit represented by formula (1), and is thus advantageous in that the dark current is reduced. The polymer compound (1) is preferably a donor-acceptor semiconductor polymer in which the structural unit represented by formula (1) and the copolymerization component (2) described below are copolymerized. The donor-acceptor semiconductor polymer refers to a polymer compound in which donor units and acceptor units alternate. The donor unit refers to an electron-donating structural unit. The acceptor unit refers to an electron-accepting structural unit. The donor-acceptor semiconductor polymer is preferably a polymer compound in which the structural unit represented by formula (1) and the copolymerization component (2) described below alternate. Such a structure is advantageous for widening an absorption wavelength band and further reducing the dark current, and can be suitably used as the p-type semiconductor compound.

In the present specification, an organosilyl group represents a monovalent group in which an Si atom has one or more hydrocarbon groups as a substituent, and the number of the hydrocarbon groups which the Si atom has as substituents is preferably two or more and more preferably three.

In the benzo(bis)thiazole structural unit represented by formula (1), T¹ and T² may be the same or different from each other. However, T¹ and T² are preferably the same from the viewpoint of facilitating the production.

In the benzo(bis)thiazole structural unit represented by formula (1), each of T¹ and T² preferably represents a group represented by one of the following formulas (t1) to (t5). Specifically, as each of T¹ and T², the alkoxy group is preferably a group represented by the following formula (t1), the thioalkoxy group is preferably a group represented by the following formula (t2), the thiophene ring that is optionally substituted with a hydrocarbon group or an organosilyl group is preferably a group represented by the following formula (t3), the thiazole ring that is optionally substituted with a hydrocarbon group or an organosilyl group is preferably a group represented by the following formula (t4), and a phenyl group that is optionally substituted with a hydrocarbon group, an alkoxy group, a thioalkoxy group, an organosilyl group, a halogen atom, or a trifluoromethyl group is preferably a group represented by the following formula (t5). In a case where T¹ and T² are each a group represented by one of the following formulas (t1) to (t5), light having a short wavelength can be absorbed, and, furthermore, planarity is high, and π-π stacking is thus efficiently formed, thereby further reducing the dark current. 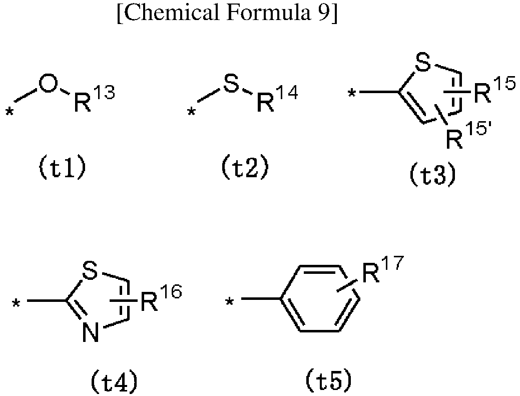 [In formulas (t1) to (t5),
R¹³ to R¹⁴ each independently represent a C6 to C30 hydrocarbon group.
R¹⁵ to R¹⁶ each independently represent a C6 to C30 hydrocarbon group or a group represented by *-Si(R¹⁸)₃. R^{15'} represents a hydrogen atom, a C6 to C30 hydrocarbon group, or a group represented by *-Si(R¹⁸)₃. R¹⁷ each independently represent a C6 to C30 hydrocarbon group, *-Q-R¹⁹, *-S-R²⁰, *-Si(R¹⁸)₃, or *-CF₃.
R¹⁸ each independently represent a C1 to C20 aliphatic hydrocarbon group or a C6 to C10 aromatic hydrocarbon group, and a plurality of R¹⁸s may be the same or different from each other.
R¹⁹ to R²⁰ each represent a C6 to C30 hydrocarbon group.
* represents a bond moiety for bonding to a thiazole ring of benzo(bis)thiazole.]

In formulas (t1) to (t5), the C6 to C30 hydrocarbon group of R¹³ to R¹⁷, R¹⁹ to R²⁰, and R¹⁵ is preferably a branched hydrocarbon group, and more preferably a branched-chain saturated hydrocarbon group. The hydrocarbon group of R¹³ to R¹⁷, R¹⁹ to R²⁰, and R^{15'} is branched, and thus, solubility with respect to an organic solvent can be enhanced, and the polymer compound of the present invention can have appropriate crystallinity. The larger the number of carbon atoms in the hydrocarbon group of R¹³ to R¹⁷, R¹⁹ to R²⁰, and R^{15'} is, the higher the solubility with respect to an organic solvent can be made. However, the excessively large number of carbon atoms causes reduction of reactivity in a coupling reaction described below, so that synthesis of the polymer compound becomes difficult. Therefore, the number of carbon atoms in the hydrocarbon group of R¹³ to R¹⁷, R¹⁹ to R²⁰, and R^{15'} is preferably 8 to 25, more preferably 8 to 20, and further preferably 8 to 16.

Examples of the C6 to C30 hydrocarbon groups represented by R¹³ to R¹⁷, R¹⁹ to R²⁰, and R^{15'} include C6 alkyl groups such as an n-hexyl group; C7 alkyl groups such as an n-heptyl group; C8 alkyl groups such as an n-octyl group, a 1-n-butylbutyl group, a 1-n-propylpentyl group, a 1-ethylhexyl group, a 2-ethylhexyl group, a 3-ethylhexyl group, a 4-ethylhexyl group, a 1-methylheptyl group, a 2-methylheptyl group, a 6-methylheptyl group, a 2,4,4-trimethylpentyl group, and a 2,5-dimethylhexyl group; C9 alkyl groups such as an n-nonyl group, a 1-n-propylhexyl group, a 2-n-propylhexyl group, a 1-ethylheptyl group, a 2-ethylheptyl group, a 1-methyloctyl group, a 2-methyloctyl group, a 6-methyloctyl group, a 2,3,3,4-tetramethylpentyl group, and a 3,5,5-trimethylhexyl group; C10 alkyl groups such as an n-decyl group, a 1-n-pentylpentyl group, a 1-n-butylhexyl group, a 2-n-butylhexyl group, a 1-n-propylheptyl group, a 1-ethyloctyl group, a 2-ethyloctyl group, a 1-methylnonyl group, a 2-methylnonyl group, and a 3,7-dimethyloctyl group; C11 alkyl groups such as an n-undecyl group, a 1-n-butylheptyl group, a 2-n-butylheptyl group, a 1-n-propyloctyl group, a 2-n-propyloctyl group, a 1-ethylnonyl group, and a 2-ethylnonyl group; C12 alkyl groups such as an n-dodecyl group, a 1-n-pentylheptyl group, a 2-n-pentylheptyl group, a 1-n-butyloctyl group, a 2-n-butyloctyl group, a 1-n-propylnonyl group, and a 2-n-propylnonyl group; C13 alkyl groups such as an n-tridecyl group, a 1-n-pentyloctyl group, a 2-n-pentyloctyl group, a 1-n-butylnonyl group, a 2-n-butylnonyl group, a 1-methyldodecyl group, and a 2-methyldodecyl group; C14 alkyl groups such as an n-tetradecyl group, a 1-n-heptylheptyl group, a 1-n-hexyloctyl group, a 2-n-hexyloctyl group, a 1-n-pentylnonyl group, and a 2-n-pentylnonyl group; C15 alkyl groups such as an n-pentadecyl group, a 1-n-heptyloctyl group, a 1-n-hexylnonyl group, and a 2-n-hexylnonyl group; C16 alkyl groups such as an n-hexadecyl group, a 2-n-hexyldecyl group, a 1-n-octyloctyl group, a 1-n-heptylnonyl group, and a 2-n-heptylnonyl group; C17 alkyl groups such as an n-heptadecyl group and a 1-n-octylnonyl group; C18 alkyl groups such as an n-octadecyl group and a 1-n-nonylnonyl group; C19 alkyl groups such as an n-nonadecyl group; C20 alkyl groups such as an n-eicosyl group and a 2-n-octyldodecyl group; C21 alkyl groups such as an n-heneicosyl group; C22 alkyl groups such as an n-docosyl group; C23 alkyl groups such as an n-tricosyl group; and C24 alkyl groups such as an n-tetracosyl group and a 2-n-decyltetradecyl group. The C8 to C28 alkyl group is preferable, the C8 to C24 alkyl group is more preferable, the C8 to C26 branched-chain alkyl group is even more preferable, and a 2-ethylhexyl group, a 3,7-dimethyloctyl group, a 2-n-butyloctyl group, a 2-n-hexyldecyl group, a 2-n-octyldodecyl group, and a 2-n-decyltetradecyl group are particularly preferable. In a case where R¹³ to R¹⁷, R¹⁹ to R²⁰, and R^{15'} represent the above-described groups, the polymer compound of the present invention has enhanced solubility with respect to an organic solvent and has appropriate crystallinity.

In the group represented by *-Si(R¹⁸)₃ of R¹⁵ to R¹⁷ and R^{15'} in formulas (t1) to (t5), the number of carbon atoms of the aliphatic hydrocarbon group of R¹⁸ is preferably 1 to 18 and more preferably 1 to 8. Examples of the aliphatic hydrocarbon group of R¹⁸ include a methyl group, an ethyl group, an isopropyl group, a tert-butyl group, an isobutyl group, an octyl group, and an octadecyl group. The number of carbon atoms of the aromatic hydrocarbon group of R¹⁸ is preferably 6 to 8, more preferably 6 to 7, and particularly preferably 6. Examples of the aromatic hydrocarbon group of R¹⁸ include a phenyl group. Among them, R¹⁸ is preferably an aliphatic hydrocarbon group, more preferably a branched aliphatic hydrocarbon group, and particularly preferably an isopropyl group. The plurality of R¹⁸s may be the same or different from each other. However, the plurality of R¹⁸s are preferably the same. In a case where R¹⁵ to R¹⁷ and R^{15'} are each a group represented by *-Si(R¹⁸)₃, the polymer compound of the present invention has enhanced solubility with respect to an organic solvent.

Specific examples of the group represented by *-Si(R¹⁸)₃ of R¹⁵ to R¹⁷ and R^{15'} in formulas (t1) to (t5) include alkylsilyl groups such as a trimethylsilyl group, a triethyldimethylsilyl group, an isopropyldimethylsilyl group, a triisopropylsilyl group, a tertbutyldimethylsilyl group, a triethylsilyl group, a triisobutylsilyl group, a tripropylsilyl group, a tributylsilyl group, a dimethylphenylsilyl group, and a methyldiphenylsilyl group; and arylsilyl groups such as a triphenylsilyl group and a tert-butylchlorodiphenylsilyl group. Among them, an alkylsilyl group is preferable, and a trimethylsilyl group and a triisopropylsilyl group are particularly preferable.

In a case where R¹⁷ is a halogen atom in formula (t5), any of fluorine, chlorine, bromine, and iodine may be used.

R^{15'} represents a hydrogen atom, a C6 to C30 hydrocarbon group indicated as examples of R¹⁵, or a group represented by *-Si(R¹⁸)₃,

As the electron-donating group of T¹ and T², the groups represented by formulas (t1), (t3), and (t5) are more preferable, the group represented by formula (t3) is even more preferable, and groups represented by the following formulas (t3-1) to (t3-16) are particularly preferable from the viewpoint that planarity is excellent over the entirety of the structural unit represented by formula (1). In the formulas, * represents a bond moiety.

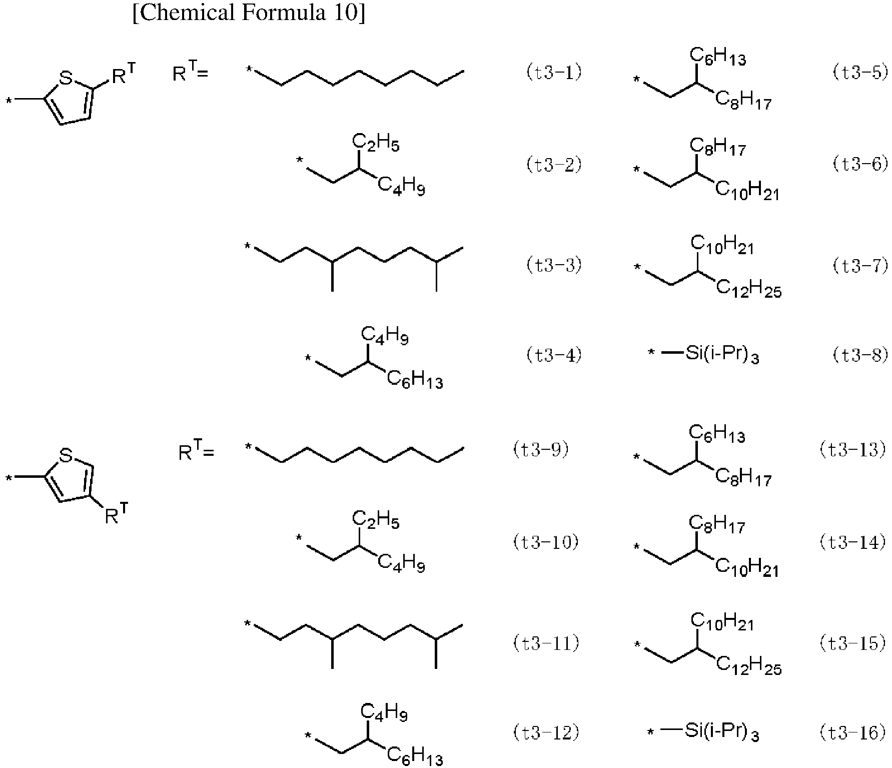

As T¹ and T², an electron-donating group or an electron-attracting group can be used. Examples of the electron-donating group include the groups represented by formulas (t1) to (t3). 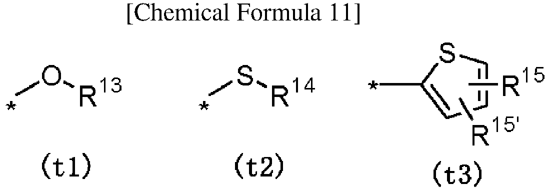 [In formulas (t1) to (t3), * represents a bond moiety, and R¹³ to R¹⁵ and R^{15'} represent groups that are the same as the above-described groups. R¹⁷ each independently represent a C6 to C30 hydrocarbon group, *-O-R¹⁹, or *-S-R²⁰. * represents a bond moiety.]

Examples of an electron-attracting group that can be used as T¹ and T² include groups represented by formulas (t4) to (t5). 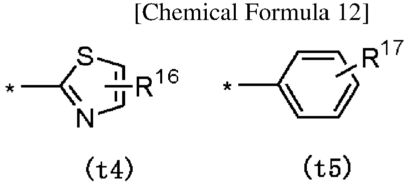 [In formulas (t4) to (t5), R¹⁶ represents groups that are the same as the above-described groups. R¹⁷ represents a halogen atom or a trifluoromethyl group. * represents a bond moiety.]

In the benzo(bis)thiazole structural unit represented by formula (1), B¹ and B² may be the same or different from each other. However, B¹ and B² are preferably the same from the viewpoint of facilitating the production. In the structural unit represented by formula (1), each of B¹ and B² preferably represents a group represented by any one of the following formulas (b1) to (b3). In a case where each of B¹ and B² represents a group represented by any one of the following formulas (b1) to (b3), the obtained polymer compound has good planarity, and the dark current can be further reduced. 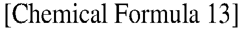 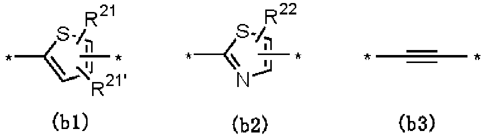
[In formulas (b1) to (b3), R²¹, R²², and R^{21'} each represent a hydrogen atom or a C6 to C30 hydrocarbon group.
* represents a bond moiety, and, in particular, * on the left side represents a bond moiety for bonding to a benzene ring of the benzo(bis)thiazole compound.]

As the C6 to C30 hydrocarbon group of R²¹, R²², and R²¹, groups indicated as examples of the C6 to C30 hydrocarbon groups of R¹³ to R¹⁷, R¹⁹ to R²⁰, and R^{15'} can be preferably used.

R²¹, R²², R^{21'} representing a hydrogen atom is preferable since a donor-acceptor semiconductor polymer is easily formed. R²¹, R²², R^{21'} representing the C6 to C30 hydrocarbon group is preferable since the dark current can be further reduced.

In the benzo(bis)thiazole structural unit represented by formula (1), from the viewpoint that planarity is excellent over the entirety of the structural unit represented by formula (1), and planarity is excellent also over the entirety of the obtained polymer compound, B¹ and B² more preferably represent groups represented by formulas (b1) and (b2). In a case where B¹ and B² represent the groups represented by formulas (b1) and (b2), interaction between S atoms and N atoms is caused in the benzo(bis)thiazole structural unit (1) to further enhance planarity. Specifically, B¹ and B² preferably represent groups represented by the following formulas. In the formulas, * represents a bond moiety, and * on the left side bonds to a benzene ring of benzo(bis)thiazole.

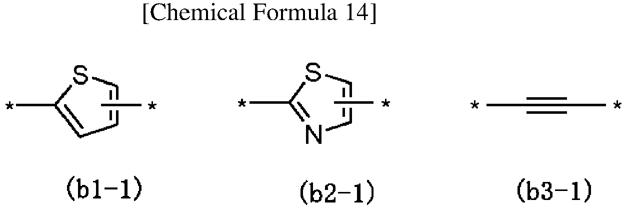

Examples of the structural unit represented by formula (1) include groups represented by the following formulas (1-1) to (1-48).

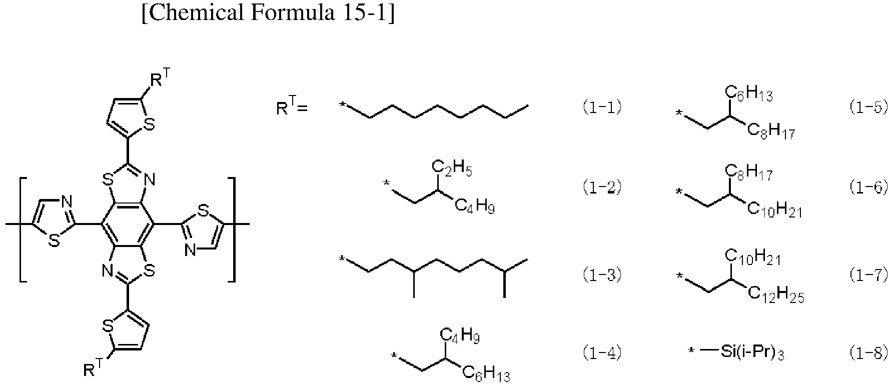

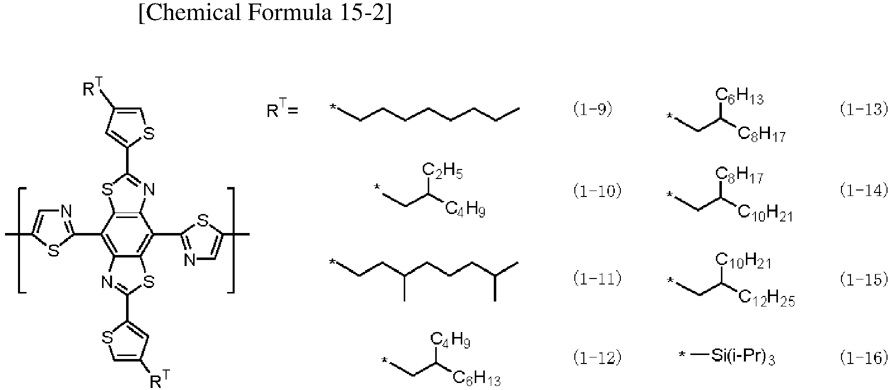

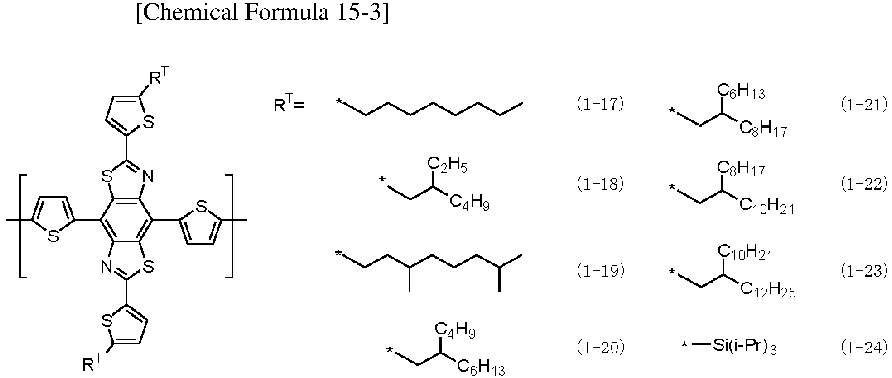

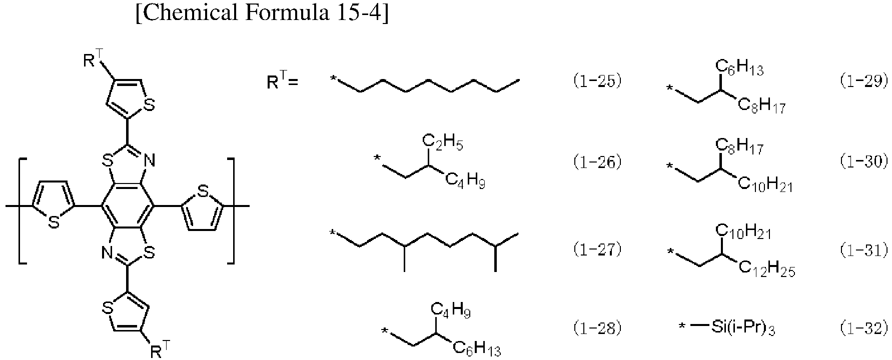

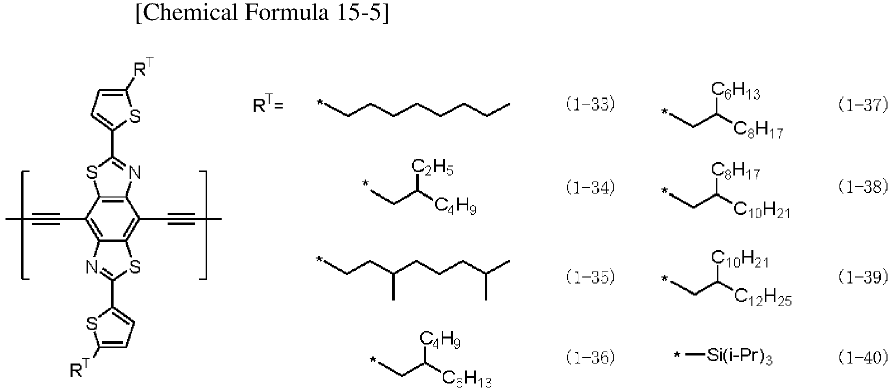

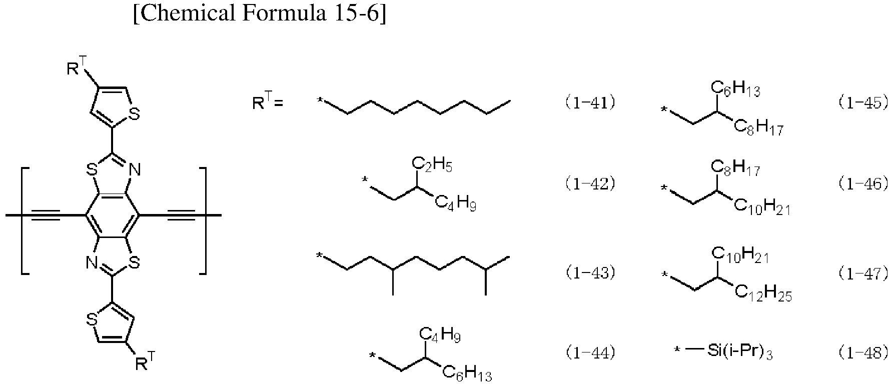

As the copolymerization component (2) (donor unit, acceptor unit) that forms the donor-acceptor semiconductor polymer in combination with the structural unit represented by formula (1), a conventionally known structural unit can be used. Specifically, for example, the following structural unit can be used. 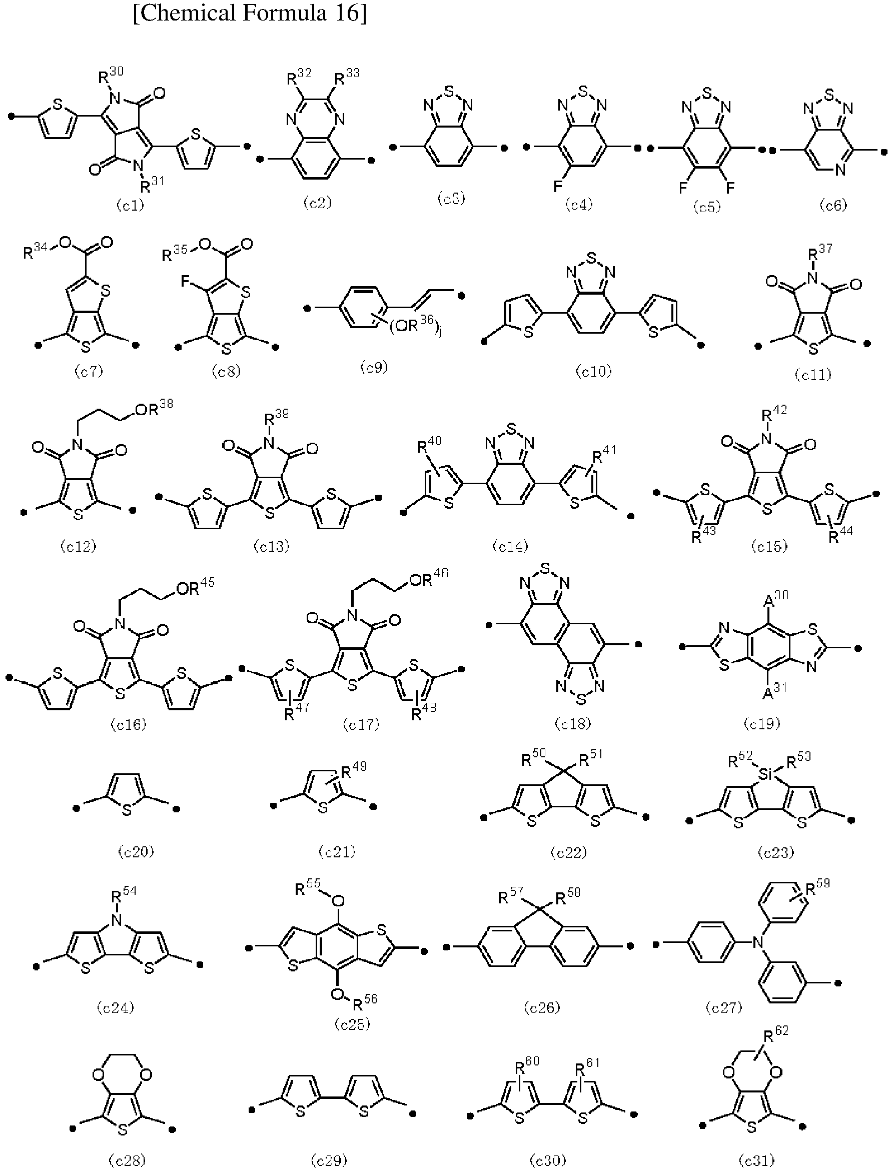 [In formulas (c1) to (c31), R³⁰ to R⁶² each independently represent a group that is the same as the C6 to C30 hydrocarbon group of R¹³ to R¹⁷, R¹⁹ to R²⁰, and R¹⁵, A³⁰ and A³¹ each independently represent a group that is the same as the group of T¹ and T², and j represents an integer of 1 to 4. • represents a bond moiety for bonding to a thiazole ring of the structural unit represented by formula (1).]

The groups represented by formulas (c1) to (c18) each represent a group which acts as an acceptor unit, and the groups represented by formulas (c20) to (c31) each represent a group which acts as a donor unit. The group represented by formula (c19) acts as an acceptor unit or acts as a donor unit depending on the kinds of A³⁰ and A³¹.

A proportion of repeat of the structural unit represented by formula (1) in the polymer compound (1) used in the present invention is, but is not particularly limited to, usually 1 mol% or more, preferably 5 mol% or more, more preferably 15 mol% or more, and further preferably 30 mol% or more. Meanwhile, the proportion of repeat is usually 99 mol% or smaller, preferably 95 mol% or smaller, more preferably 85 mol% or smaller, and further preferably 70 mol% or smaller.

A proportion of repeating units of the copolymerization component (2) in the polymer compound (1) is, but is not particularly limited to, usually 1 mol% or more, preferably 5 mol% or more, more preferably 15 mol% or more, and further preferably 30 mol% or more. Meanwhile, the proportion of the repeating units is usually 99 mol% or smaller, preferably 95 mol% or smaller, more preferably 85 mol% or smaller, and further preferably 70 mol% or smaller.

The alignment of the structural unit represented by formula (1) and the copolymerization component (2) in the repeating unit of the polymer compound (1) according to the present invention may be in any one of an alternating, block, and random states. That is, the polymer compound (1) according to the present invention may be any of an alternating copolymer, a block copolymer, and a random copolymer. The structural unit represented by formula (1) and the copolymerization component (2) preferably alternate.

The polymer compound (1) may contain one kind of the structural unit represented by formula (1) and one kind of the copolymerization component (2). Alternatively, two or more kinds of the structural units represented by formula (1) may be contained, and two or more kinds of the copolymerization components (2) may be contained. The number of kinds of each of the structural unit represented by formula (1) and the copolymerization component (2) is, but is not limited to, usually eight or smaller and preferably five or smaller. The polymer compound (1) particularly preferably contains one kind of the structural unit represented by formula (1) and one kind of the copolymerization component (2) alternately, and the polymer compound (1) most preferably contains only one kind of the structural unit represented by formula (1) and only one kind of the copolymerization component (2) alternately.

Preferable specific examples of the polymer compound (1) are indicated below. However, the polymer compound (1) according to the present invention is not limited to the following examples. In the following specific examples, R^{T} represents an n-octyl group, a 2-ethylhexyl group, a 3,7-dimethyloctyl group, a 2-n-butyloctyl, a 2-n-hexyldecyl group, a 2-n-octyldodecyl group, a 2-n-decyltetradecyl group, or a triisopropylsilyl group. R³⁰ to R⁶² each represent an n-octyl group, a 2-ethylhexyl group, a 3,7-dimethyloctyl group, a 2-n-butyloctyl group, and a 2-n-hexyldecyl group. In a case where the polymer compound (1) includes a plurality of repeating units, the proportion among the numbers of the repeating units is discretionary.

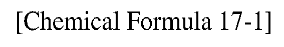

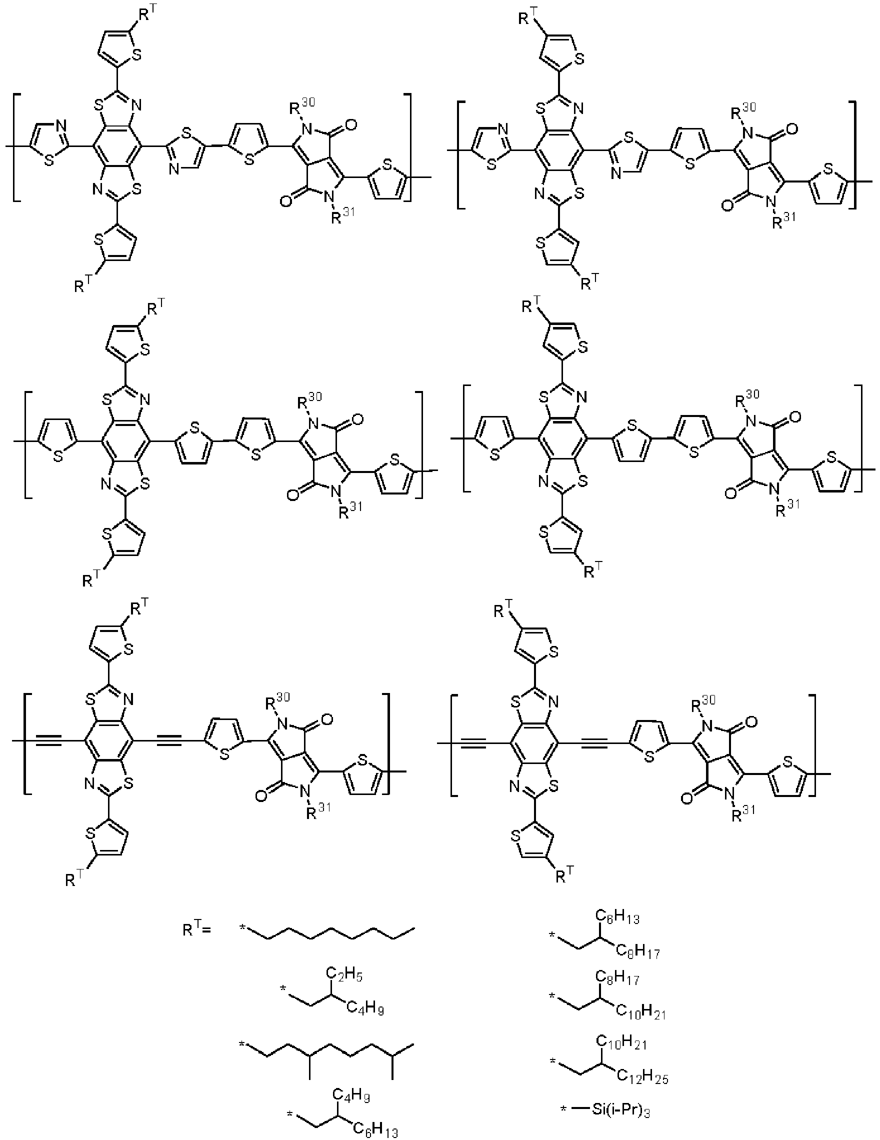

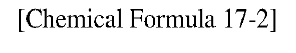

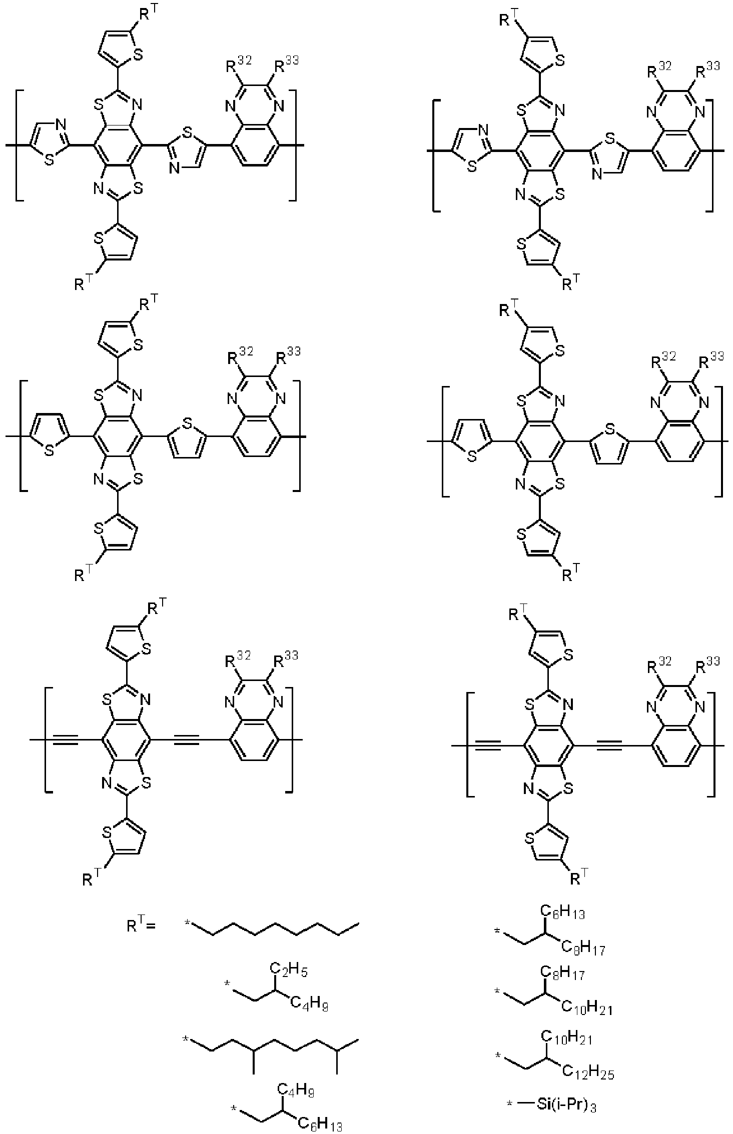

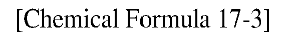

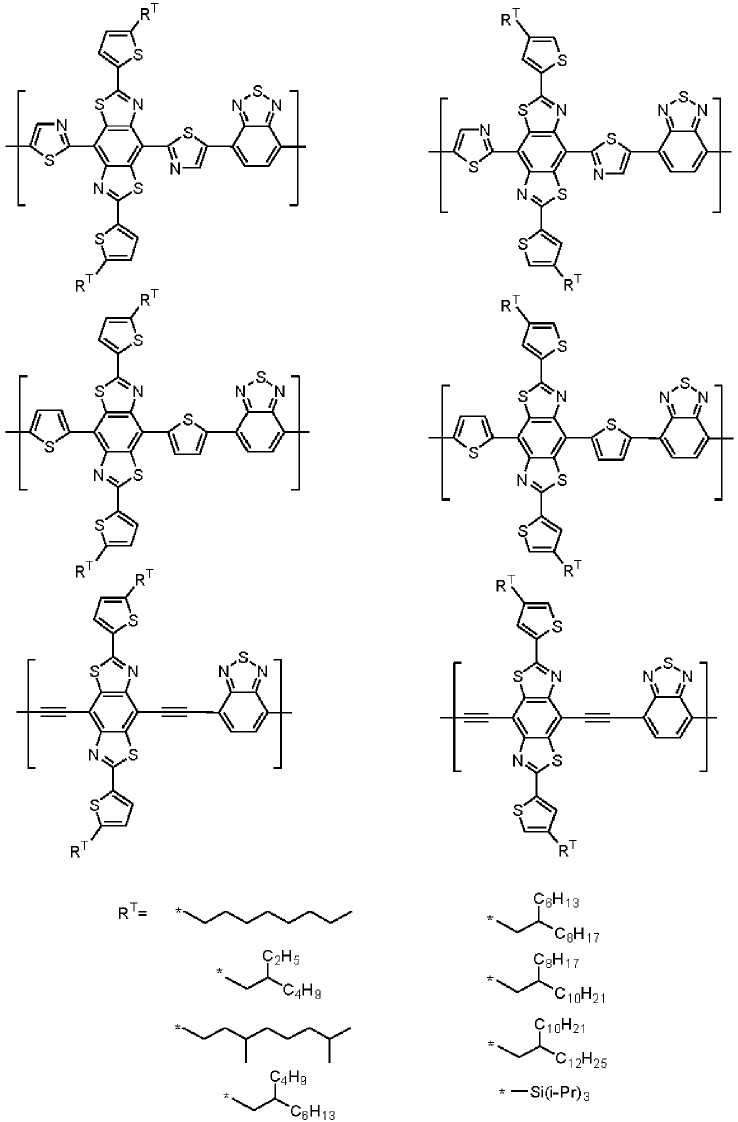

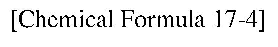

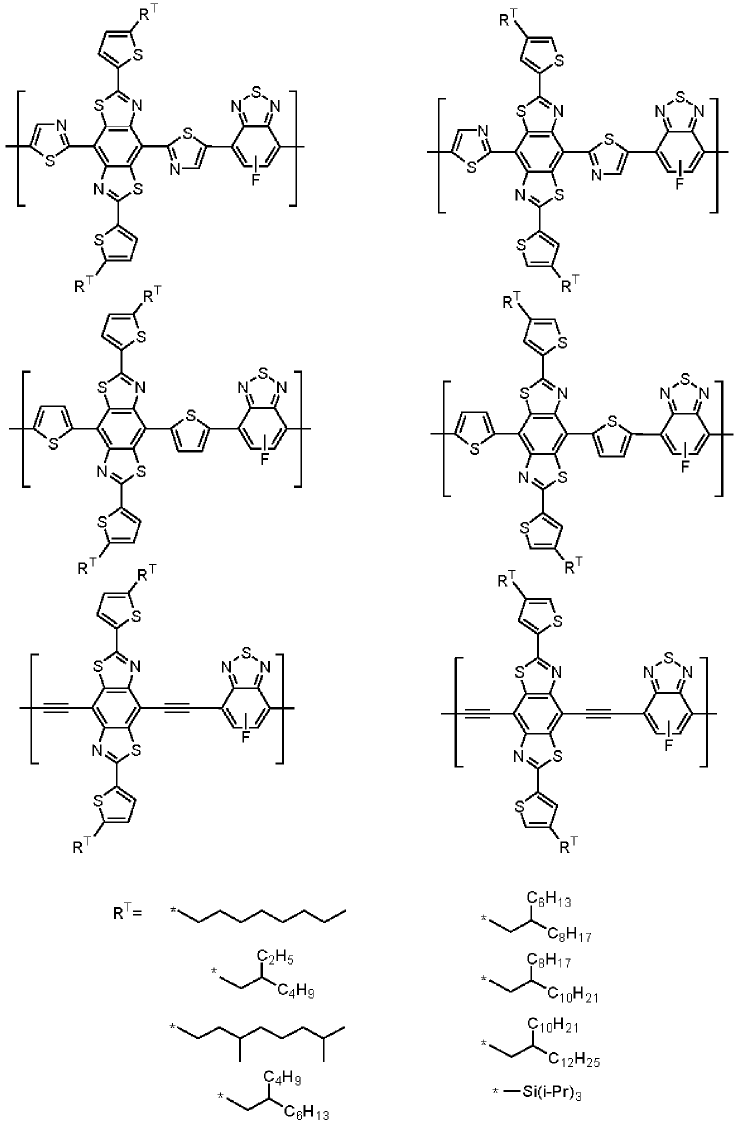

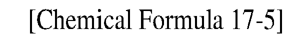

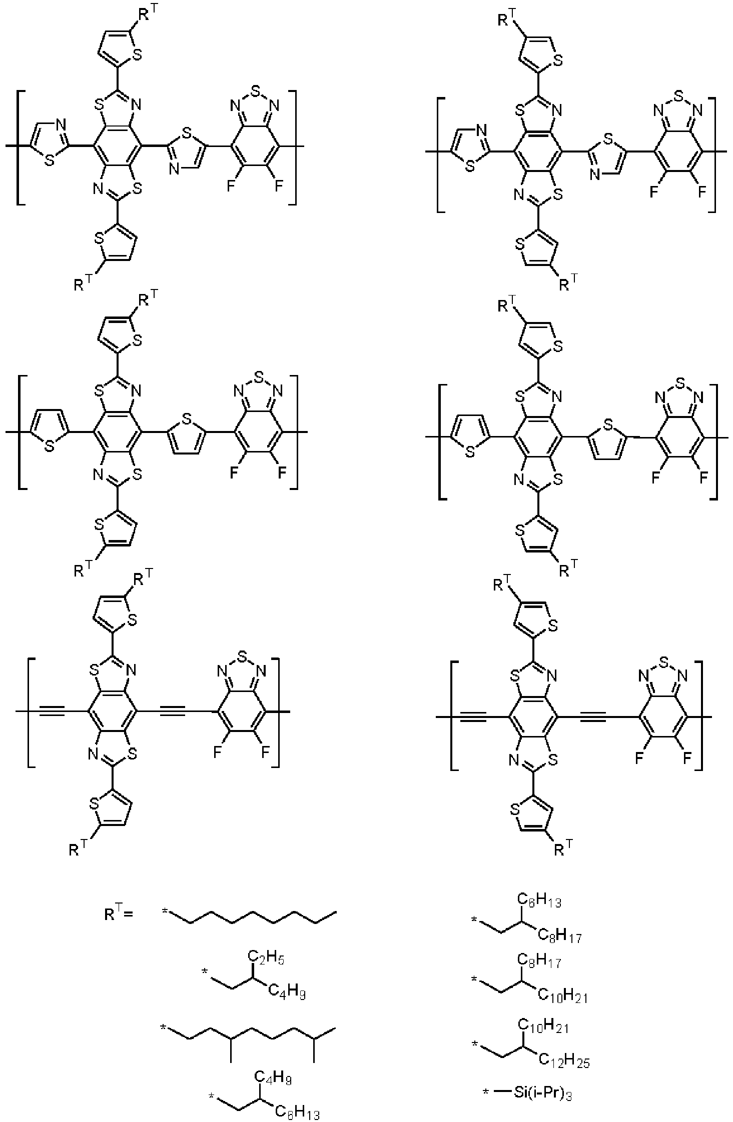

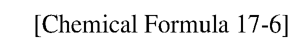

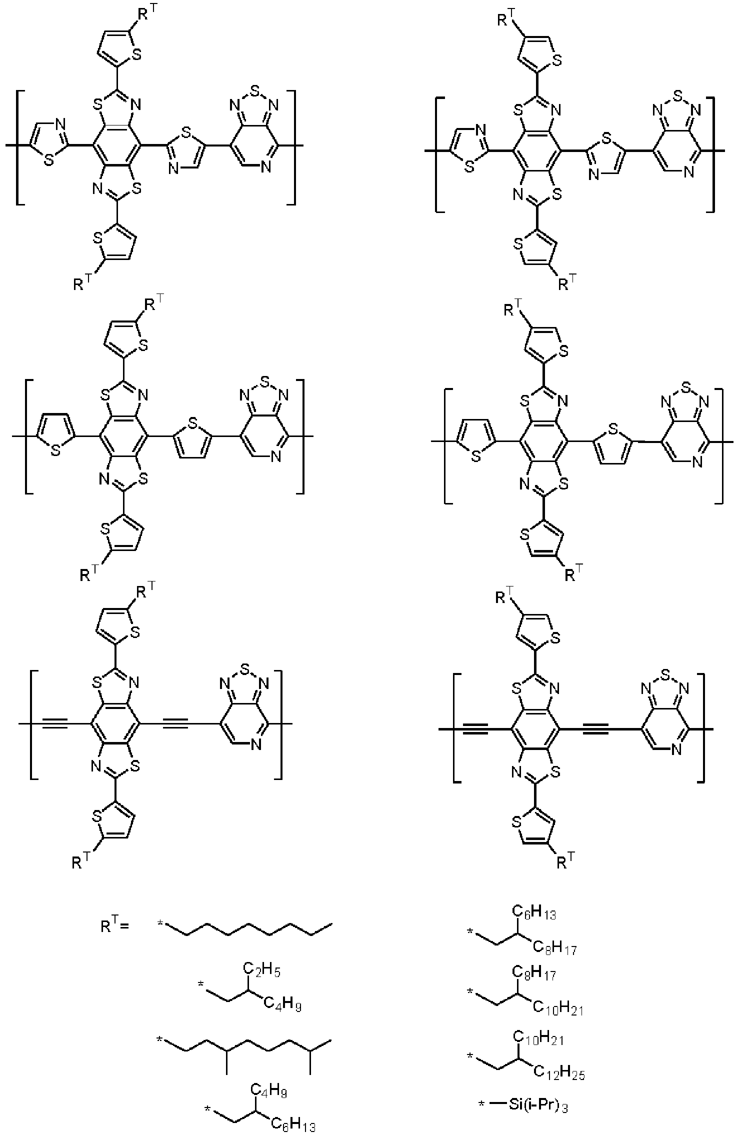

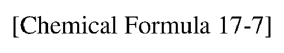

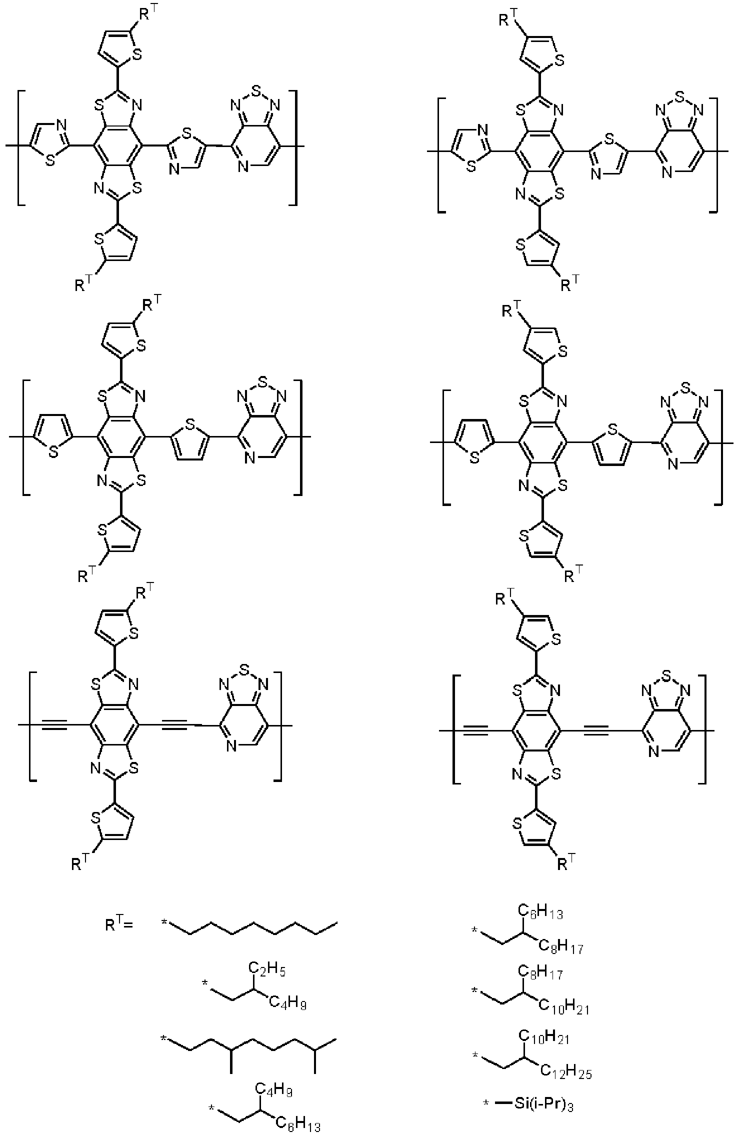

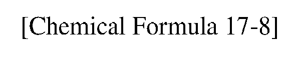

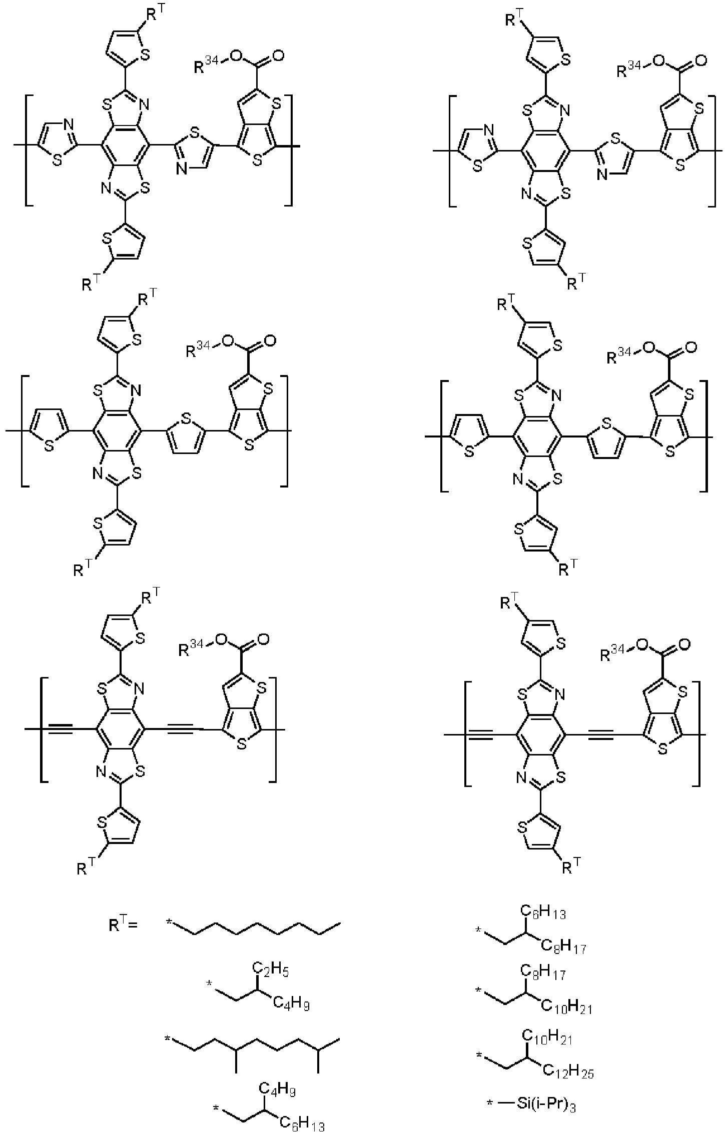

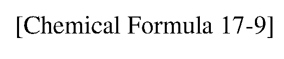

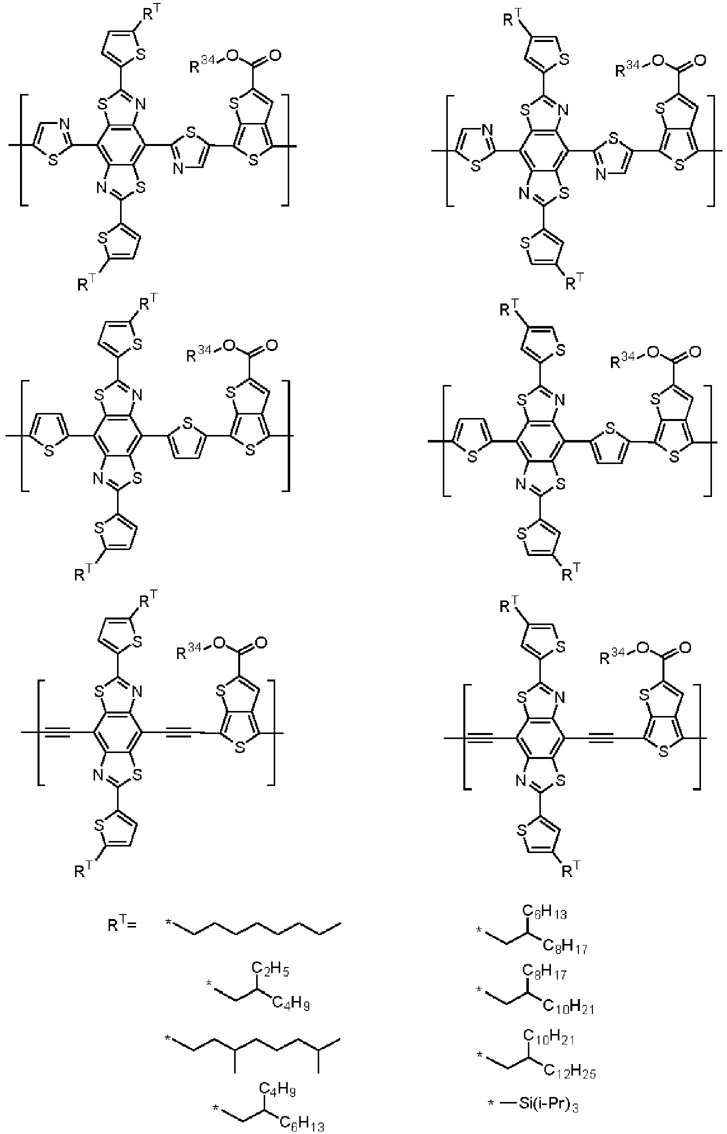

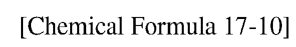

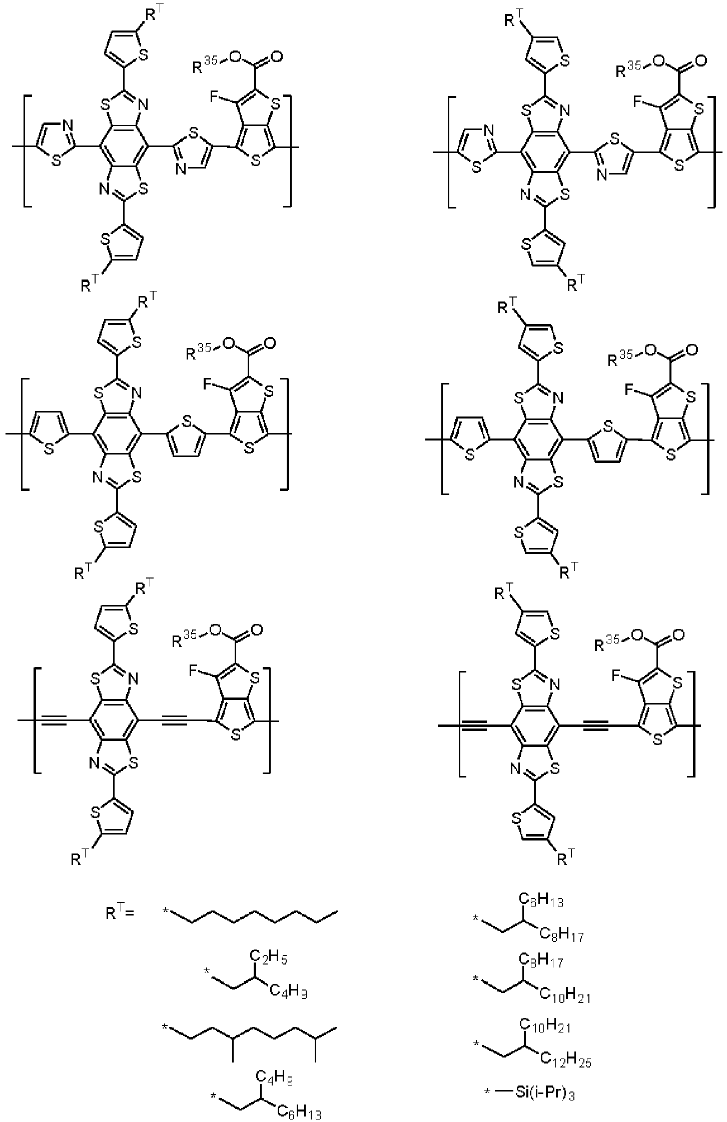

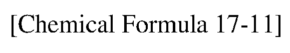

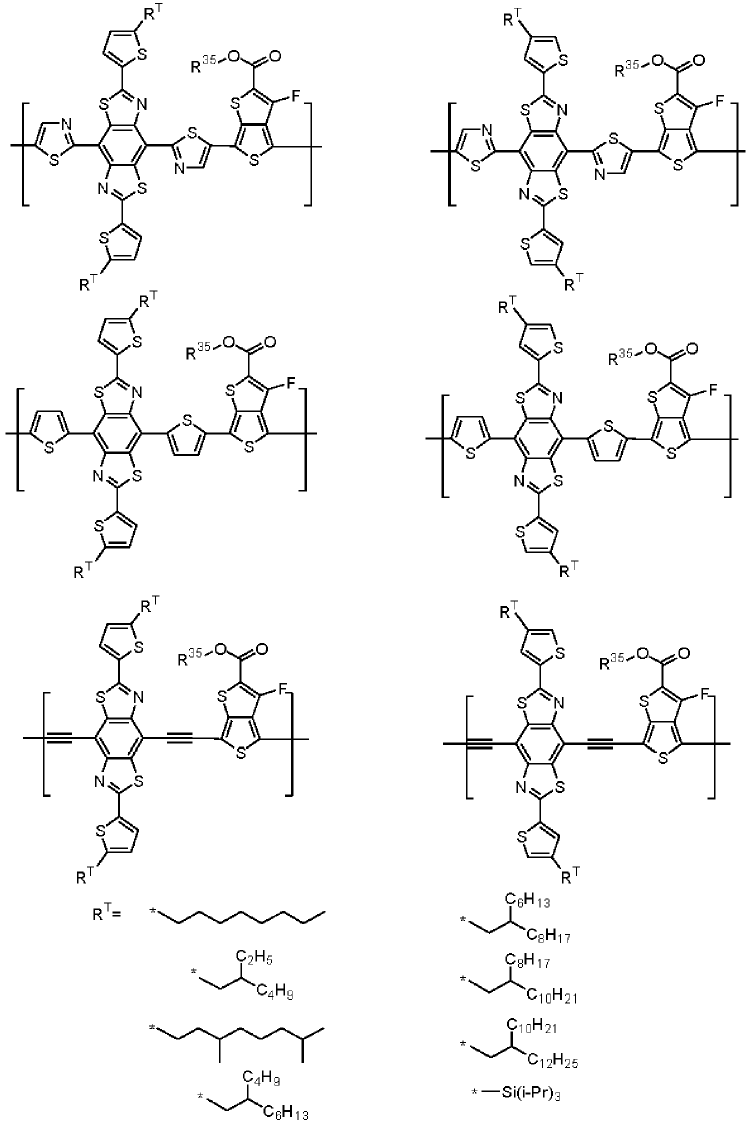

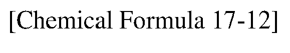

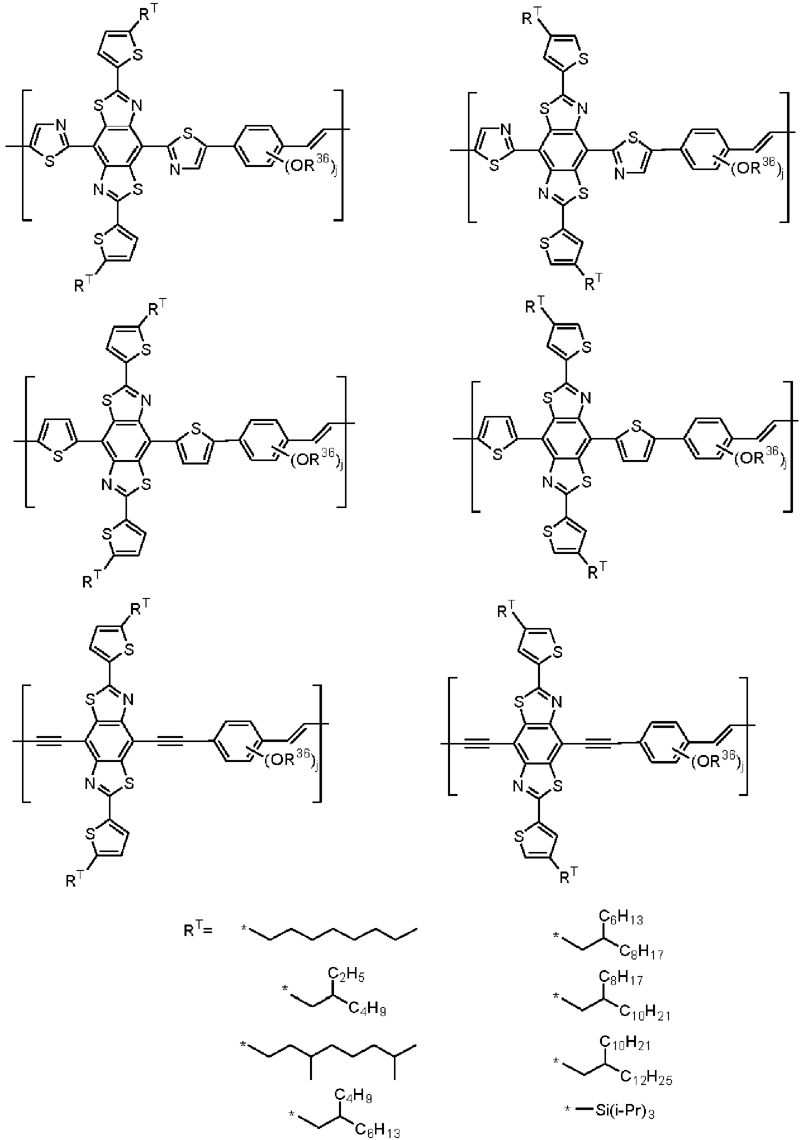

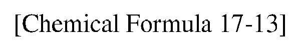

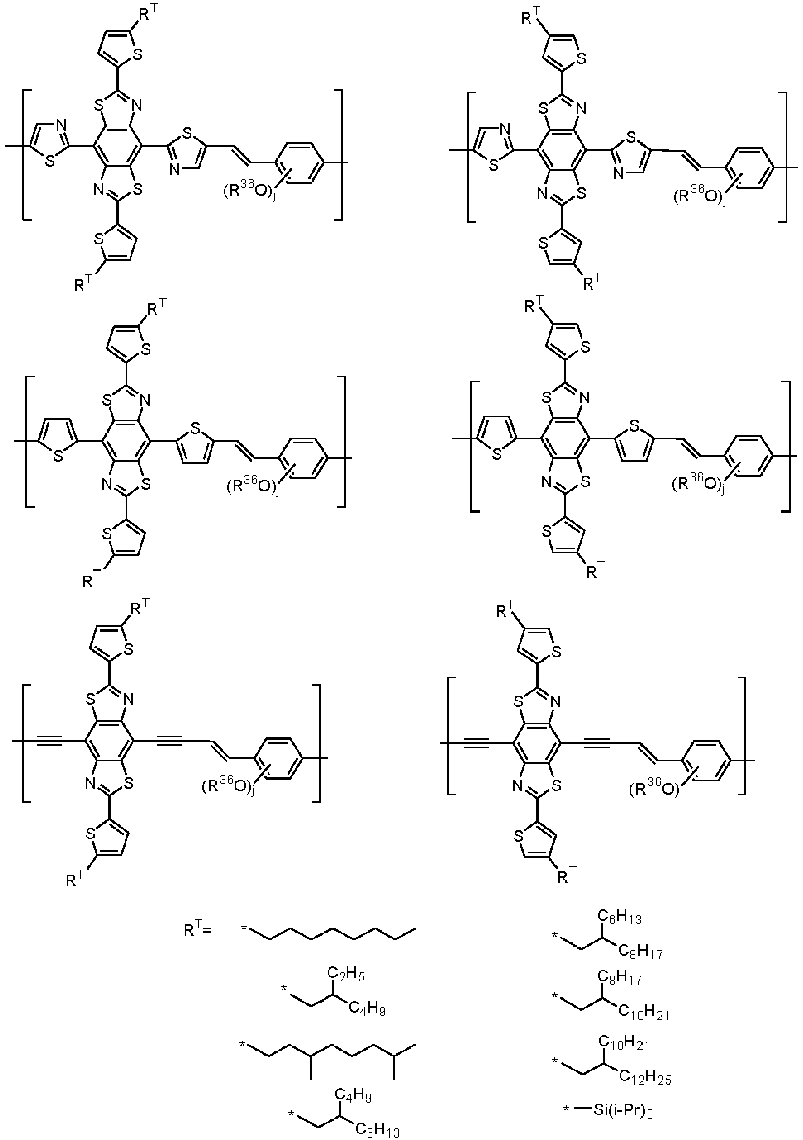

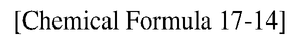

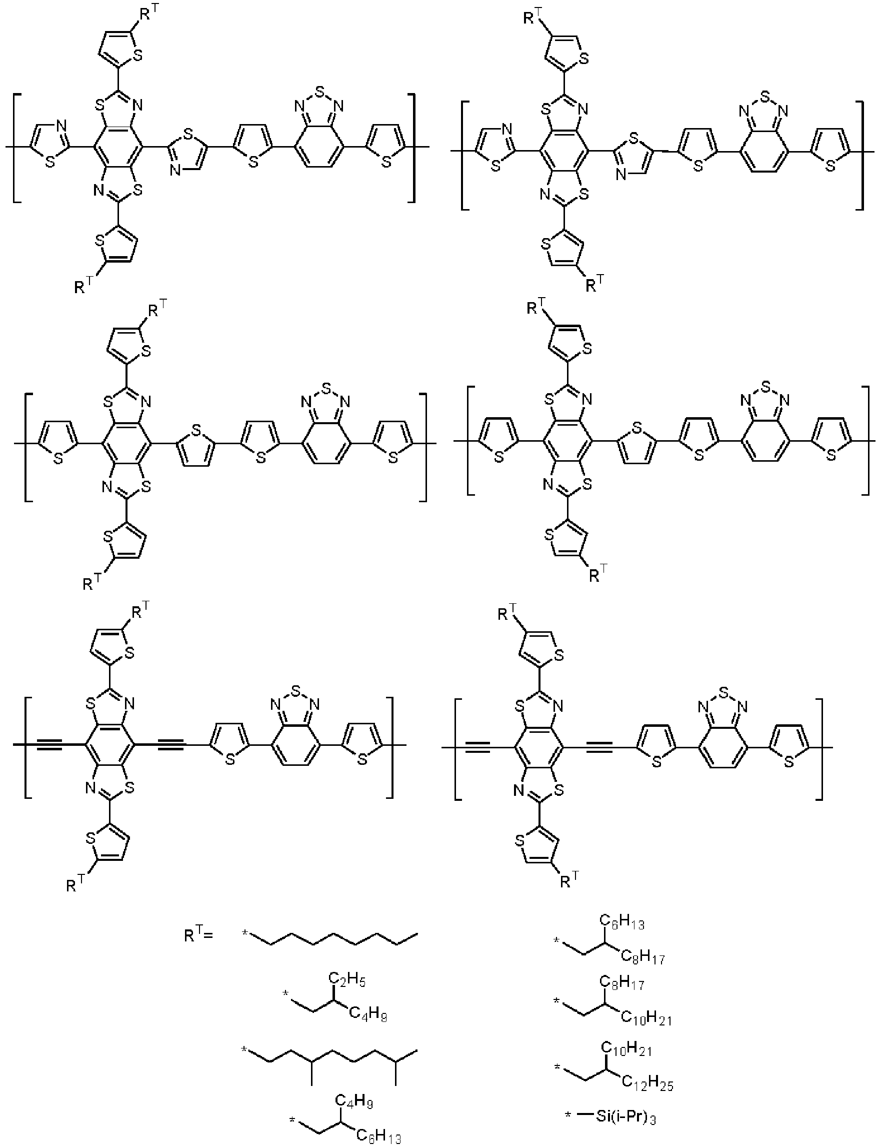

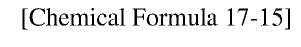

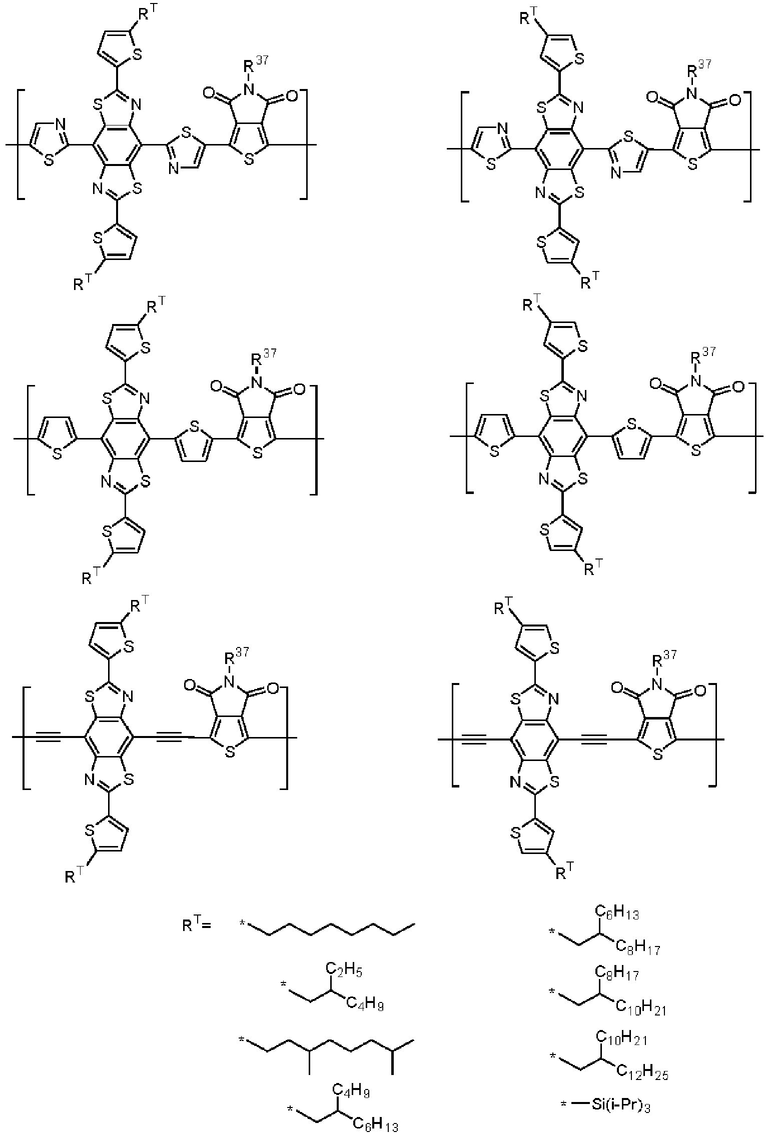

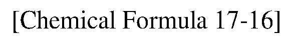

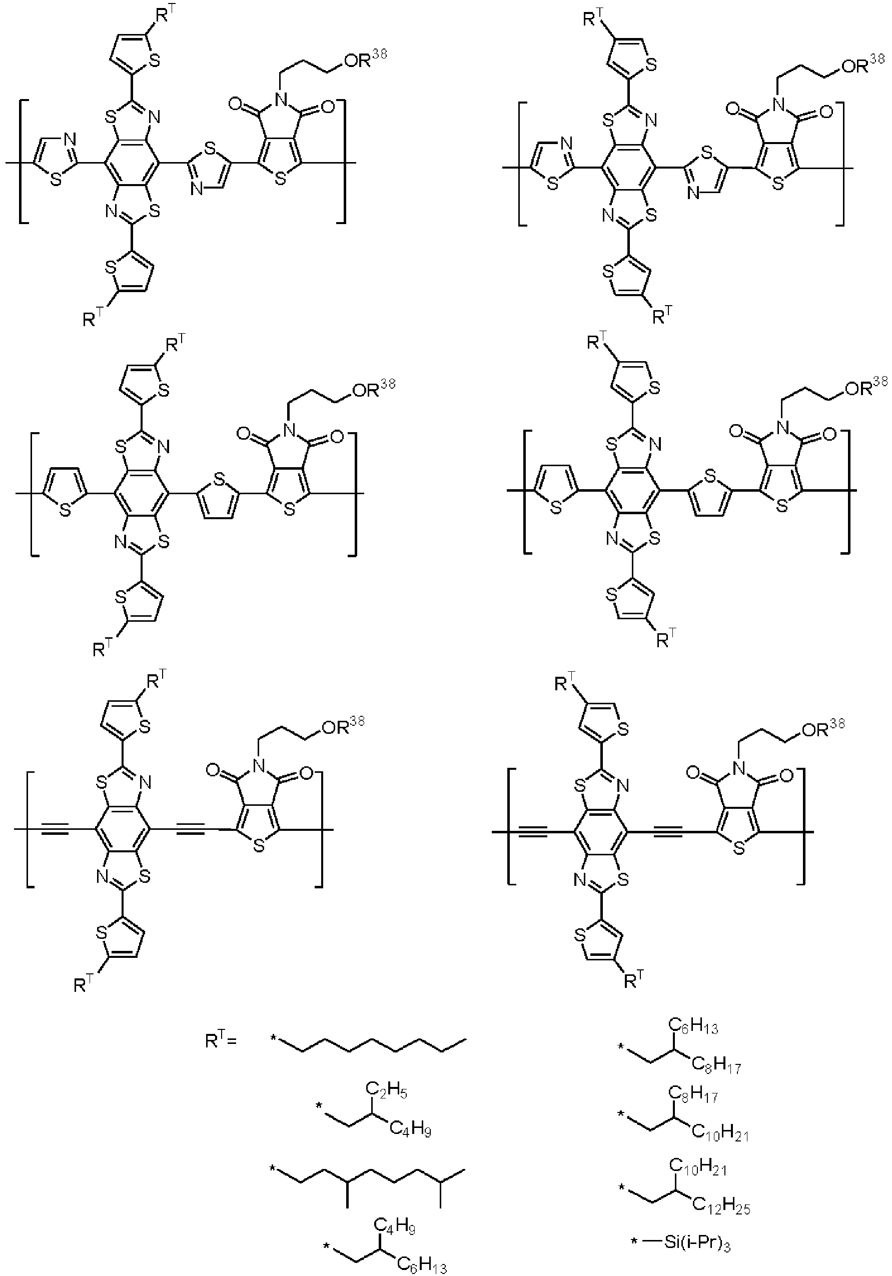

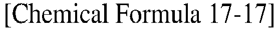

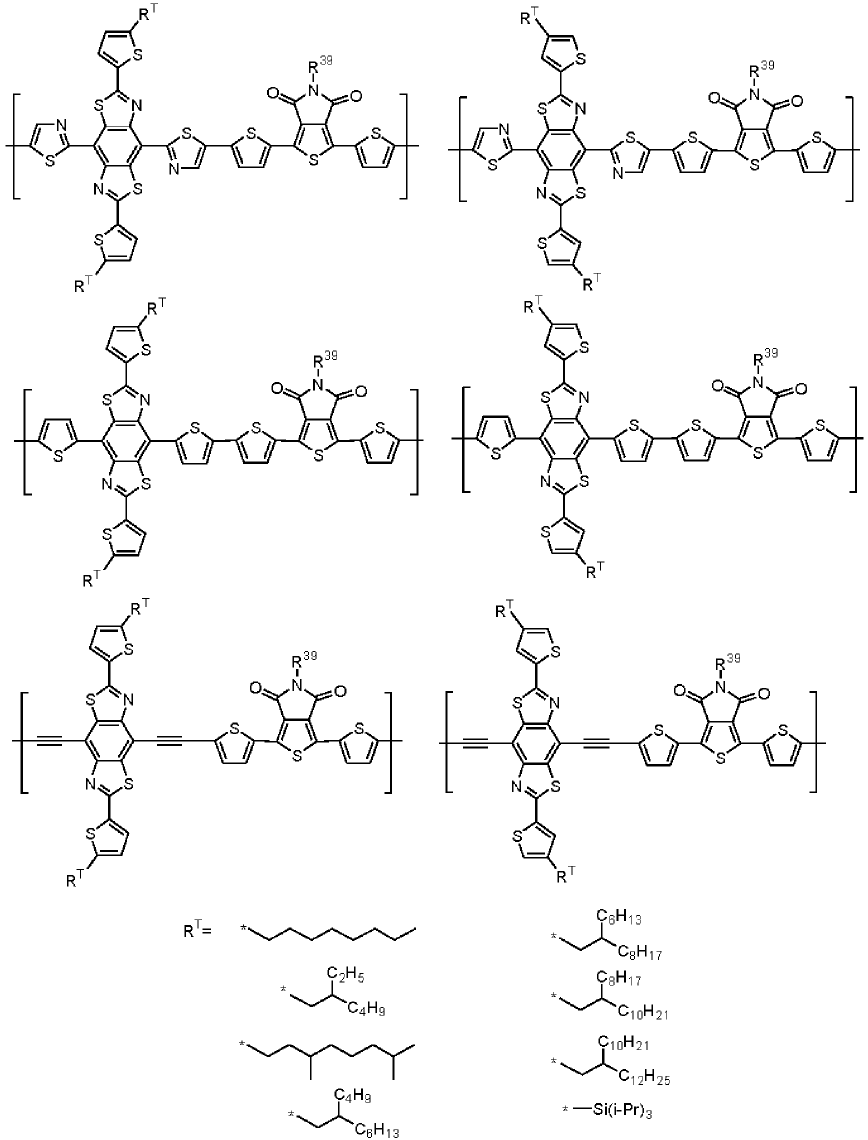

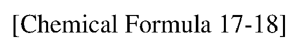

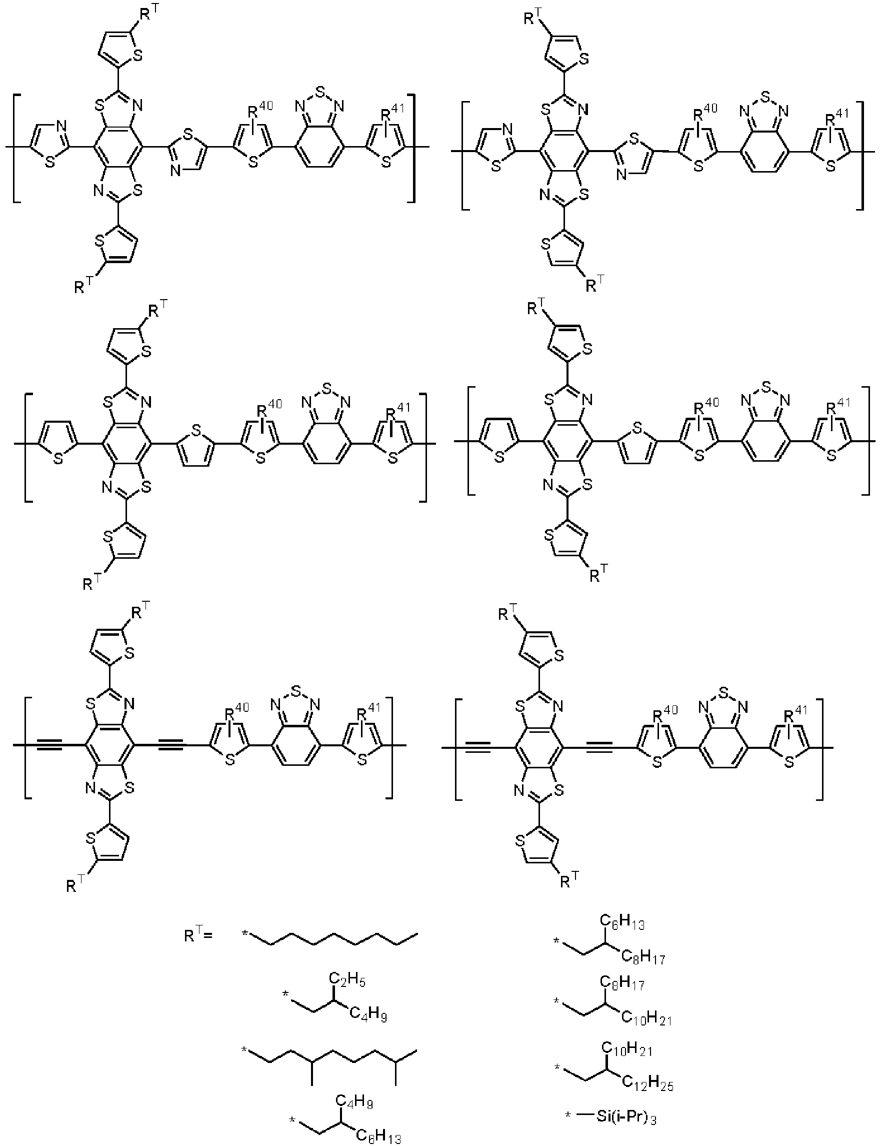

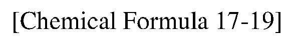

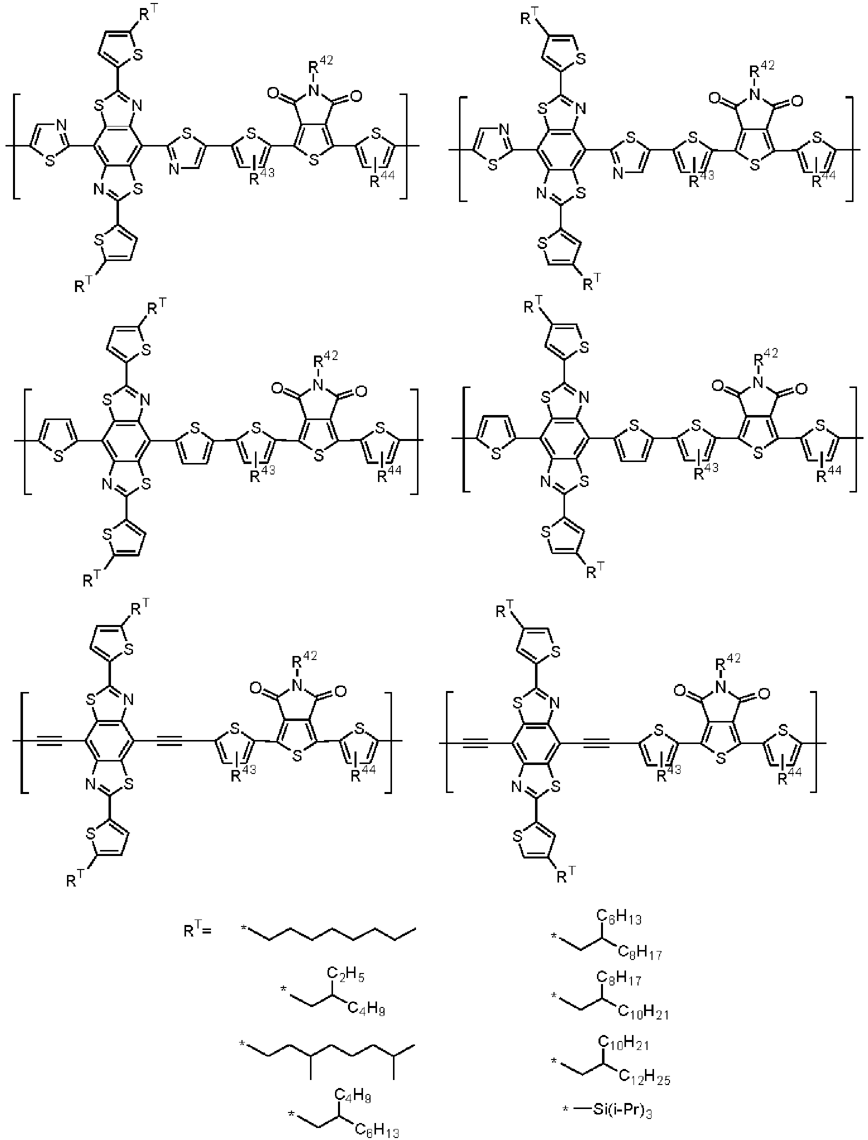

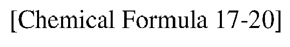

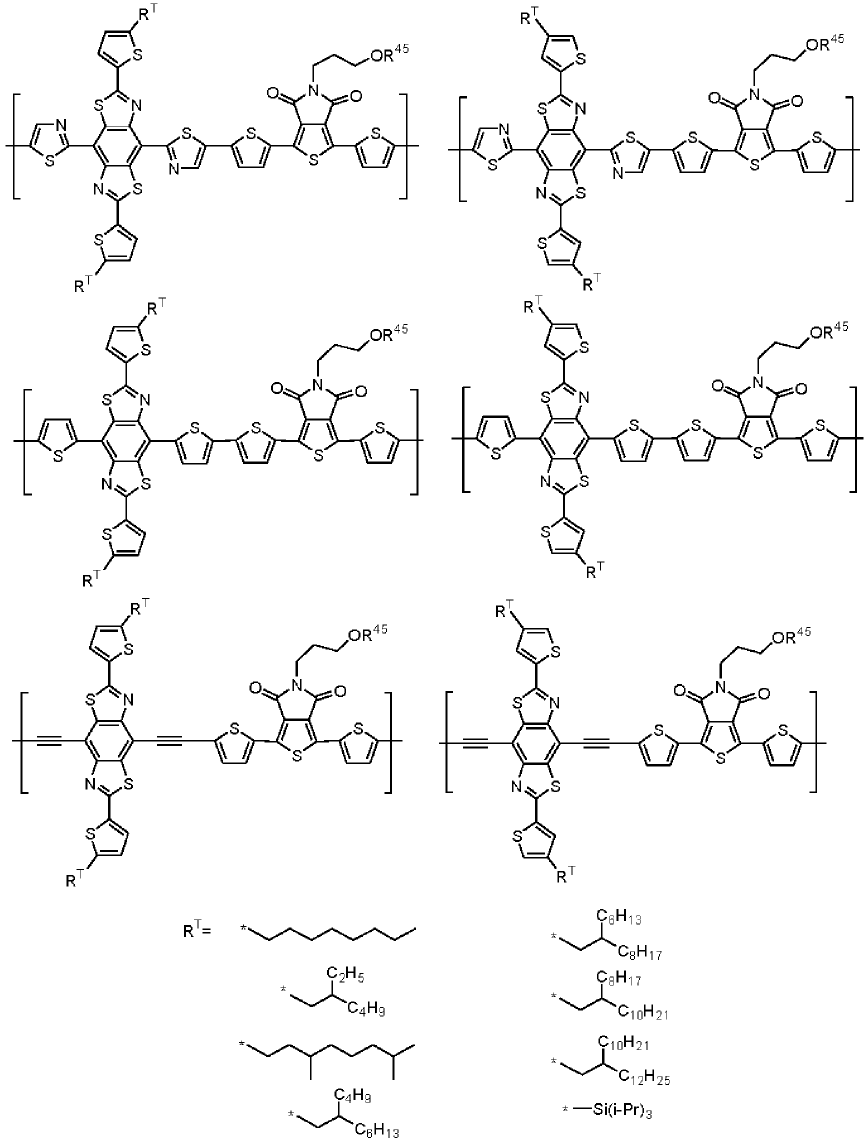

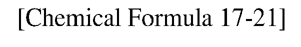

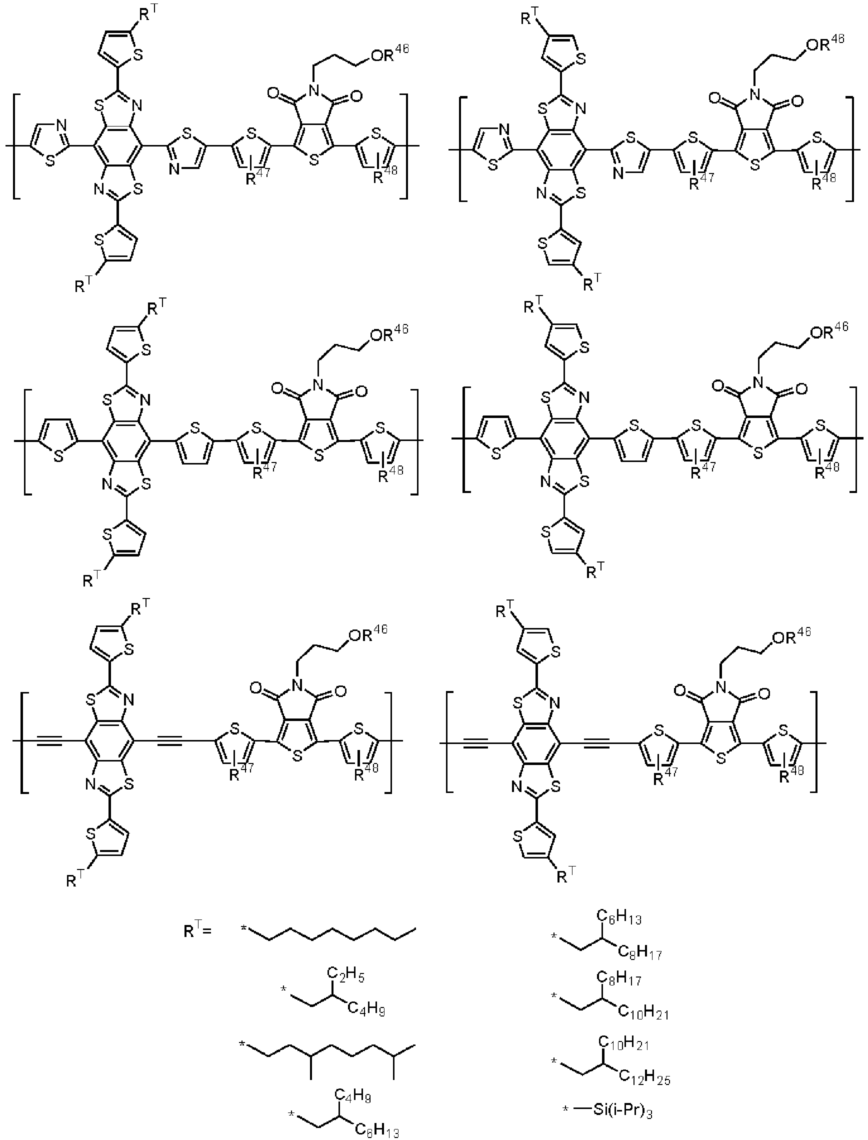

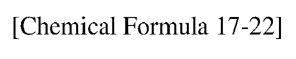

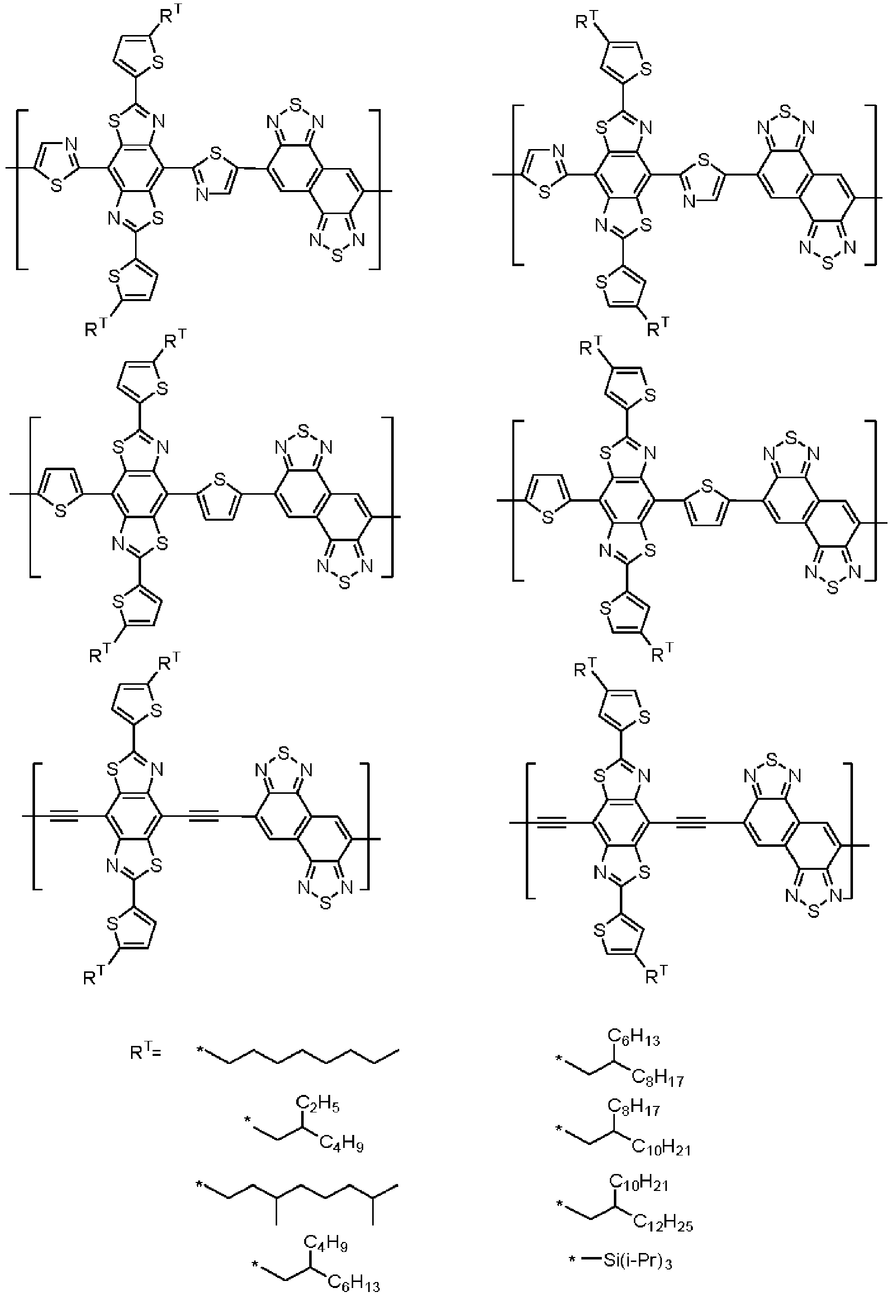

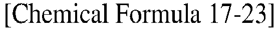

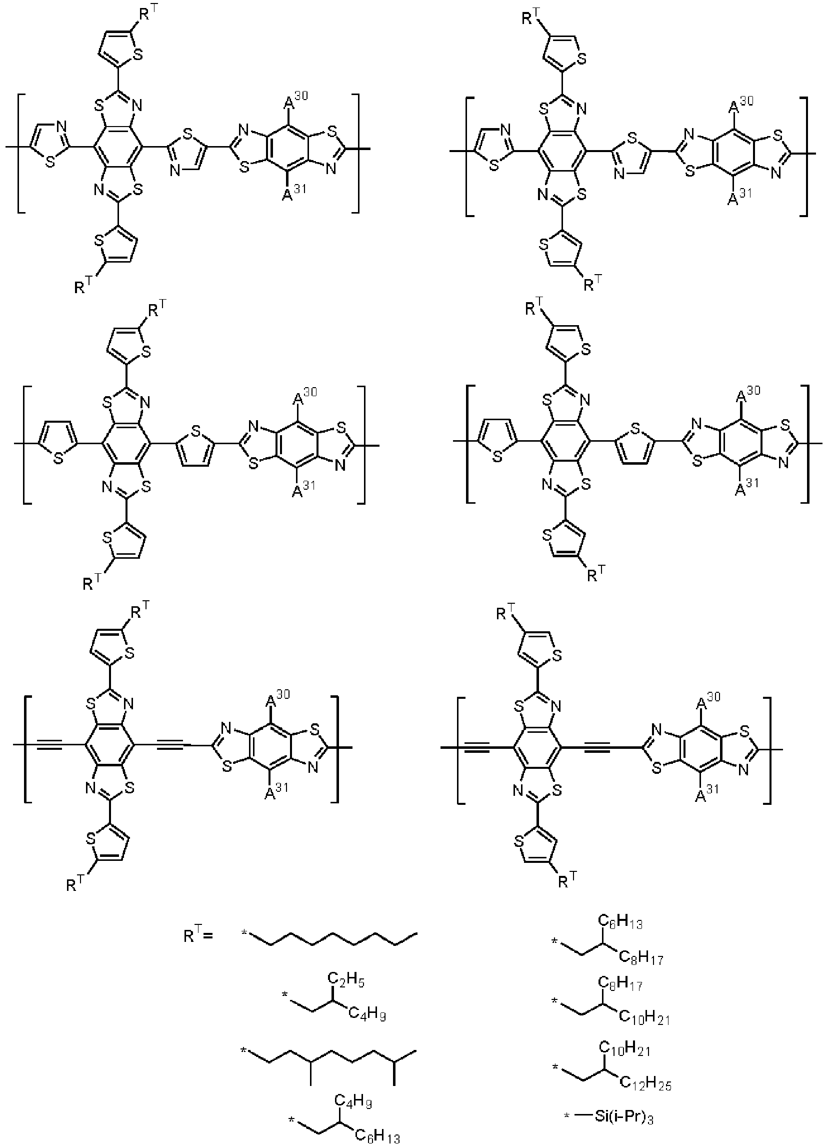

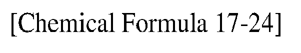

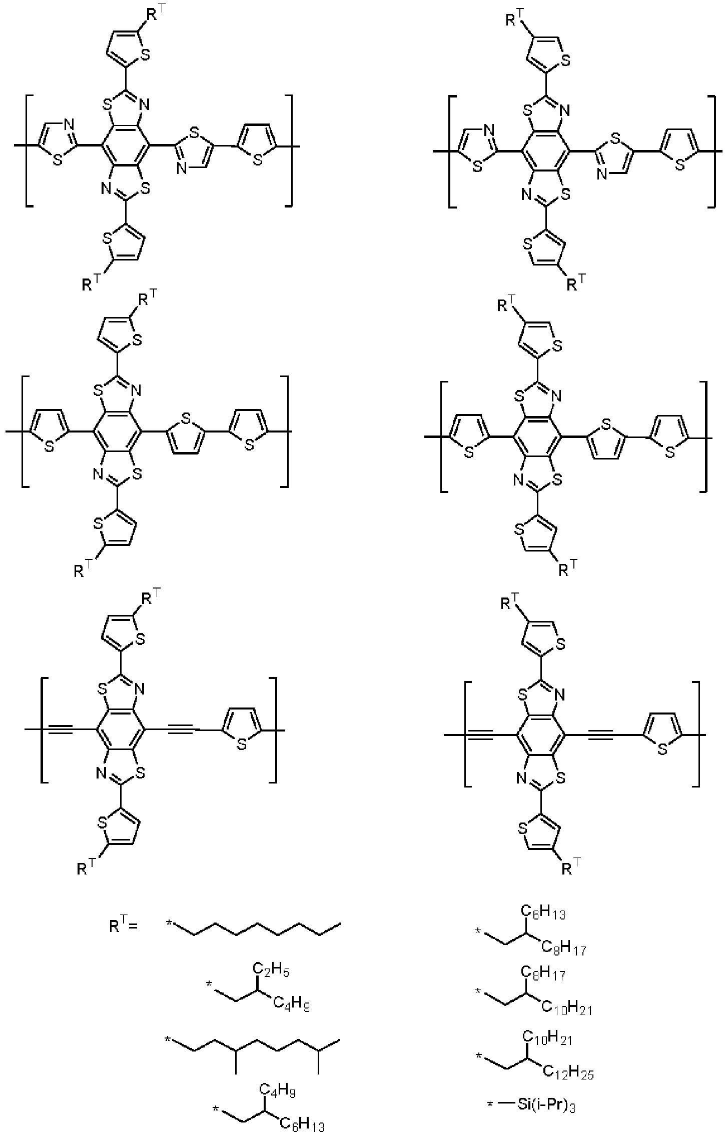

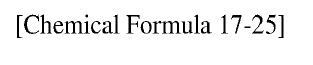

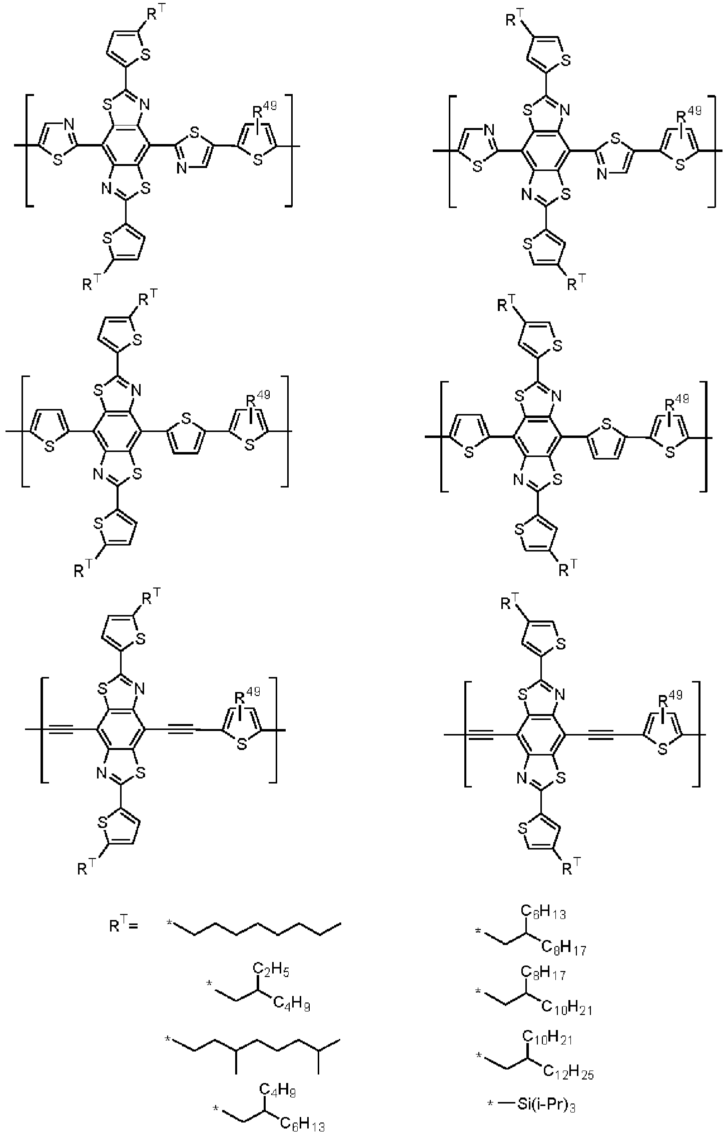

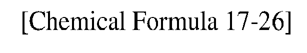

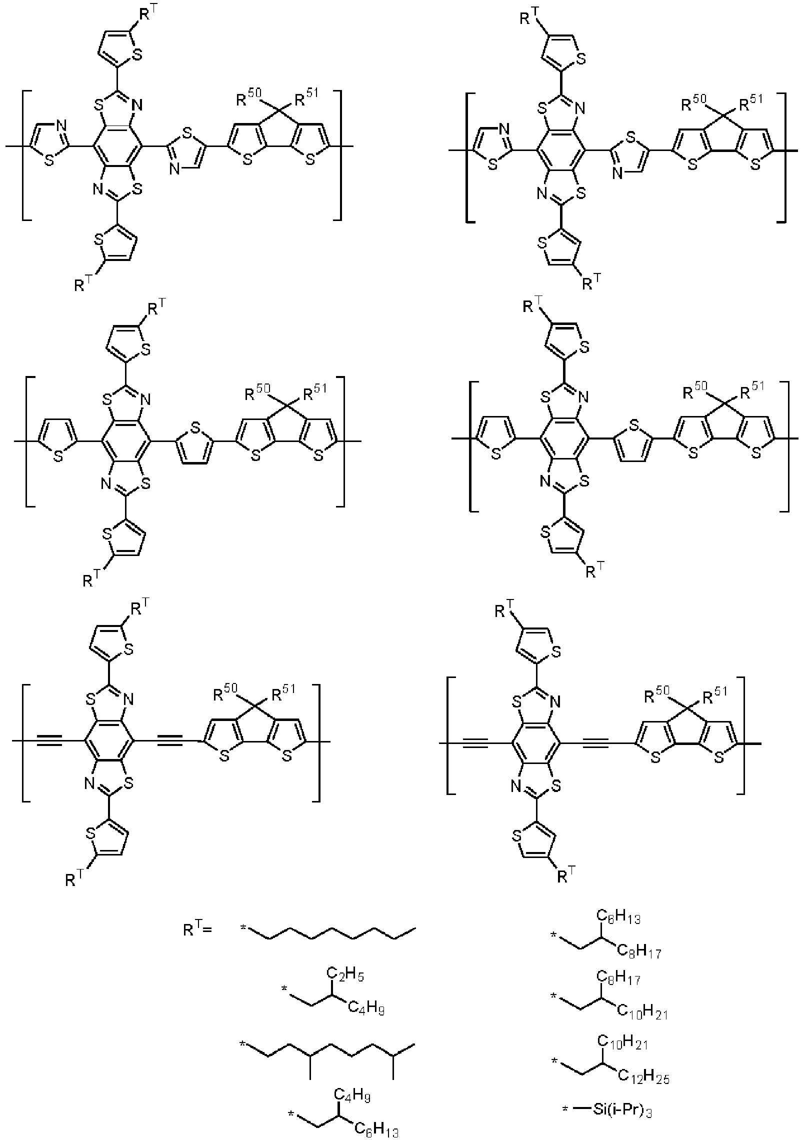

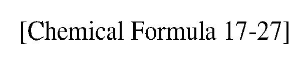

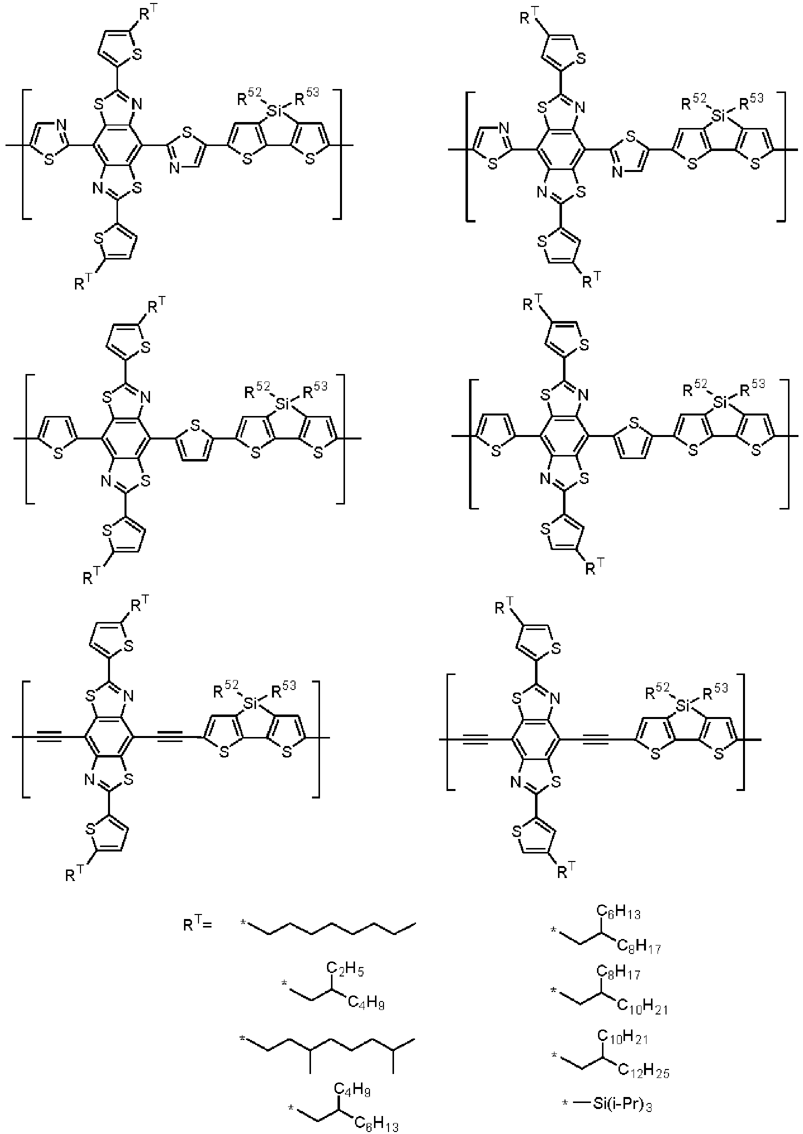

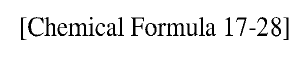

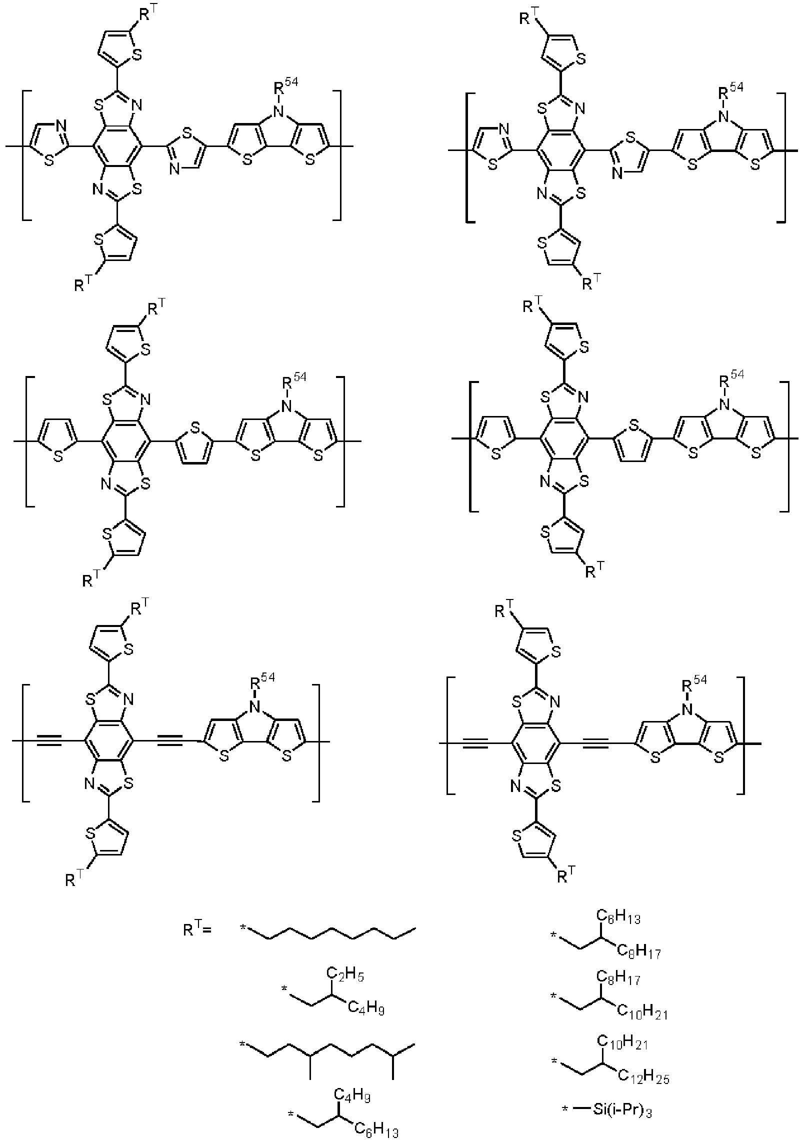

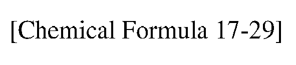

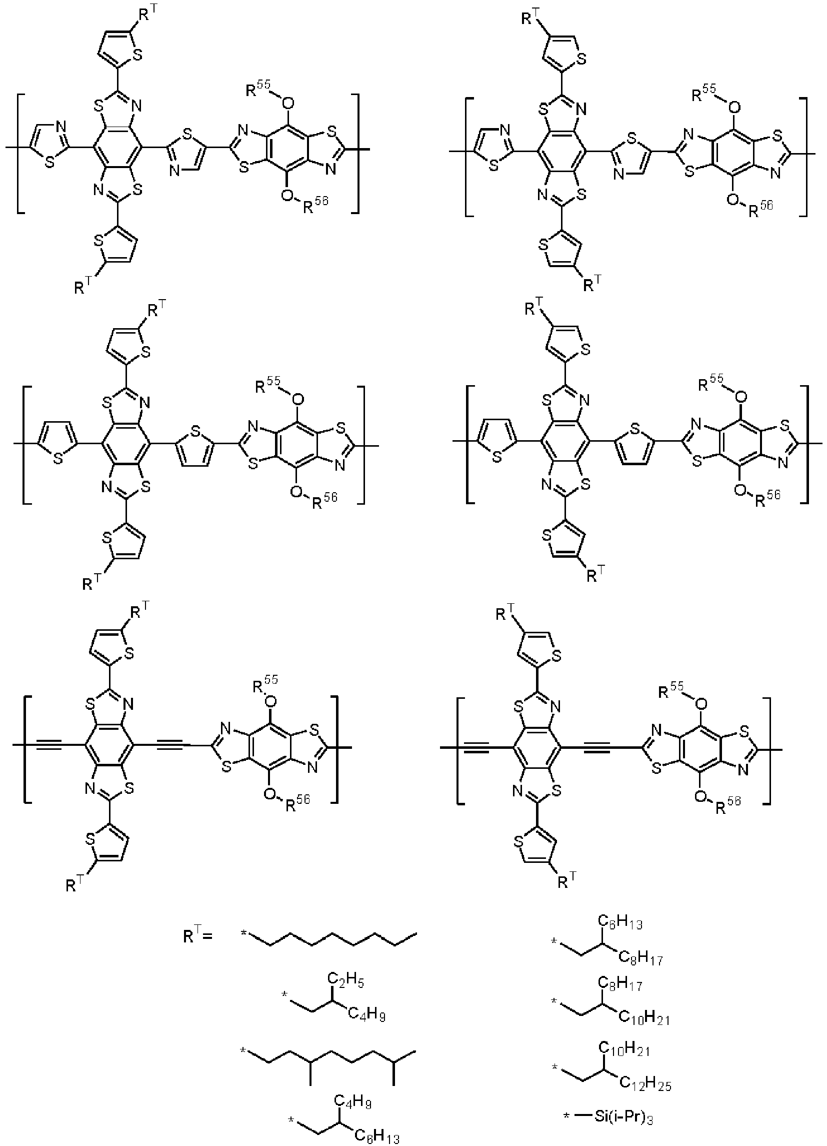

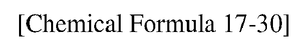

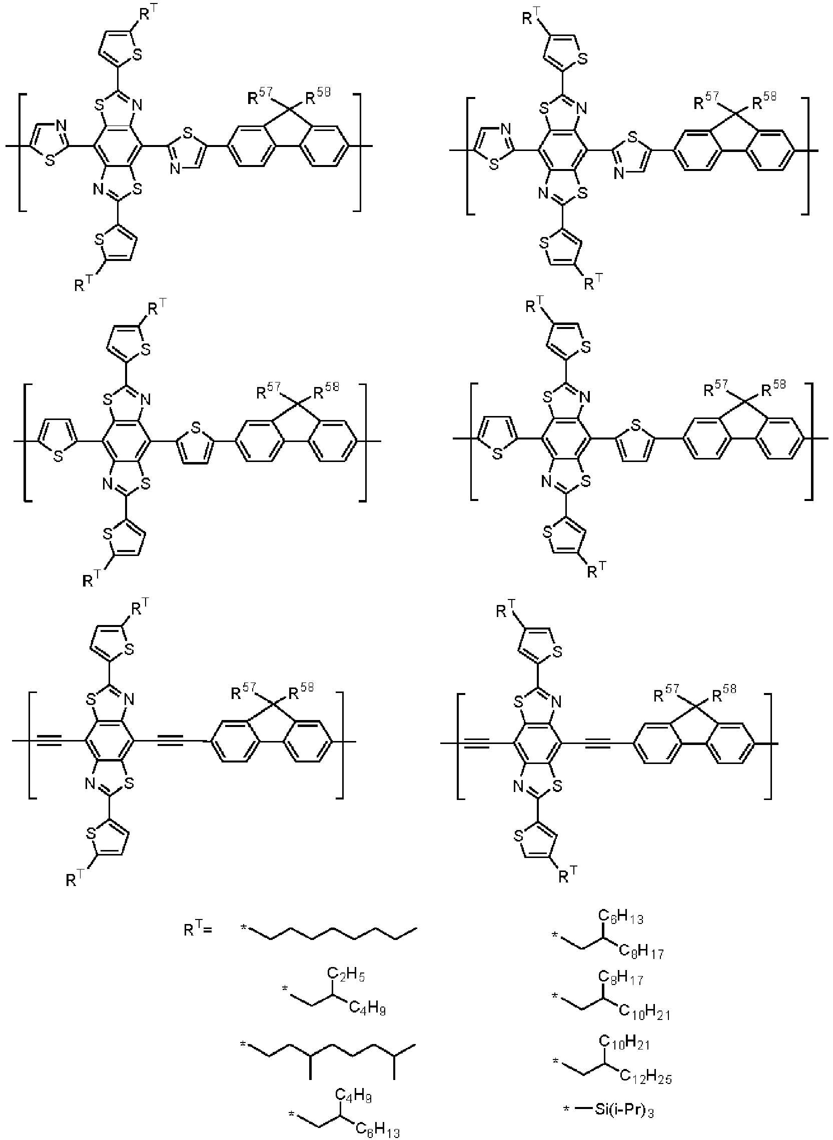

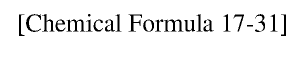

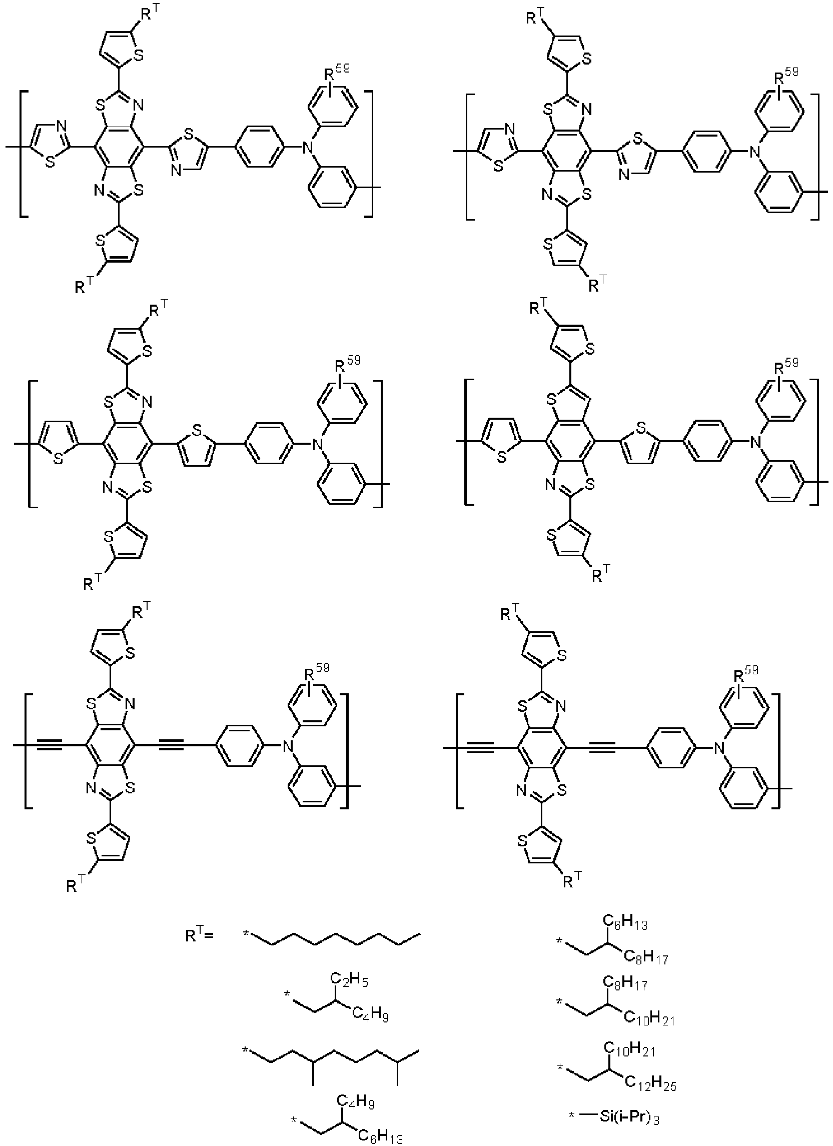

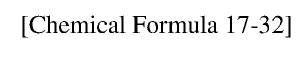

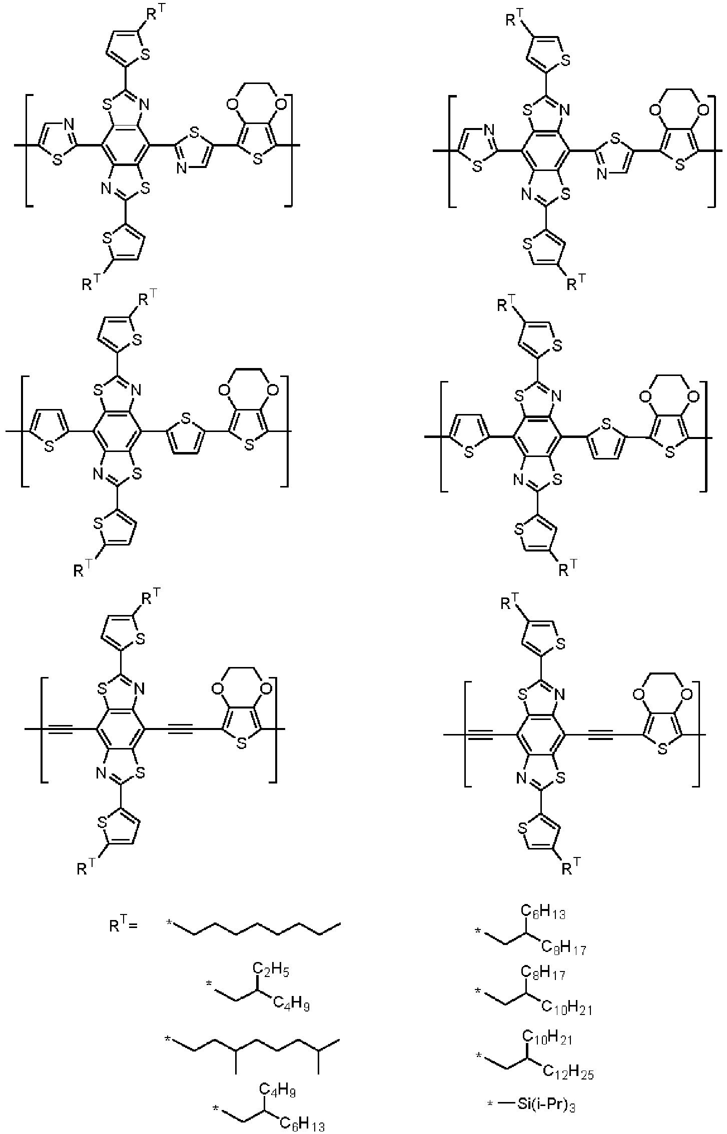

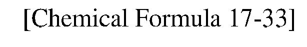

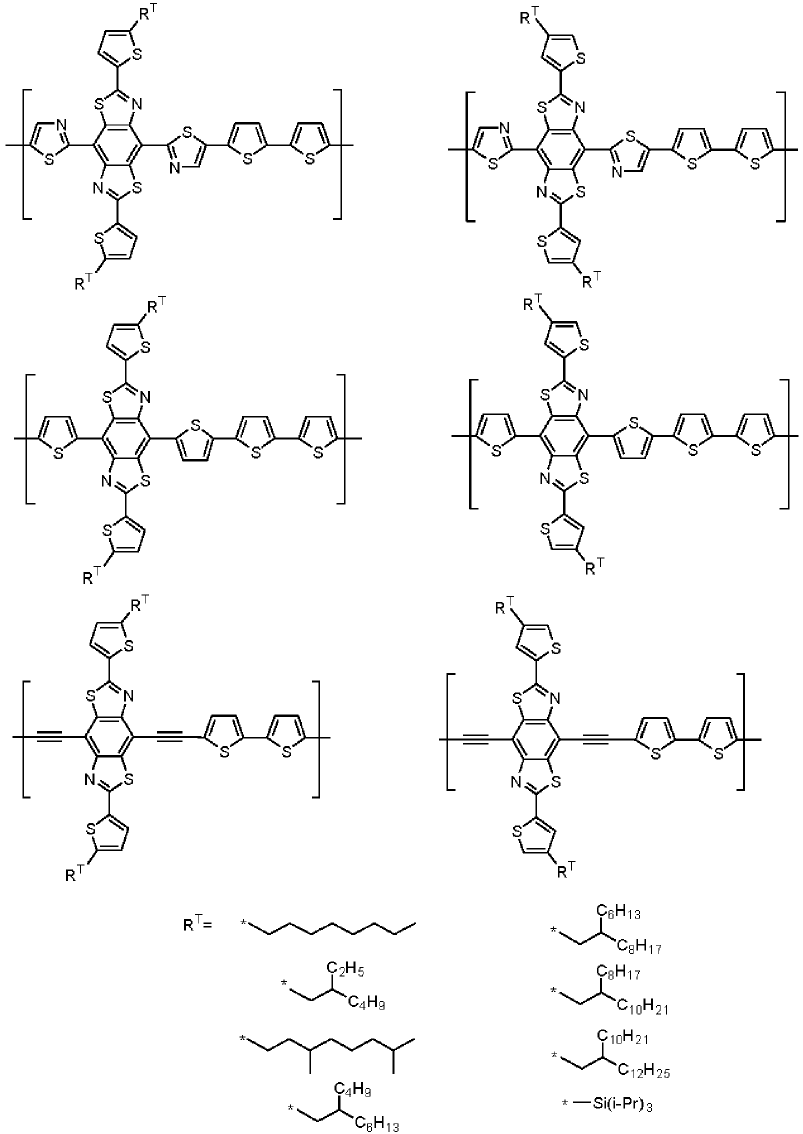

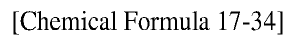

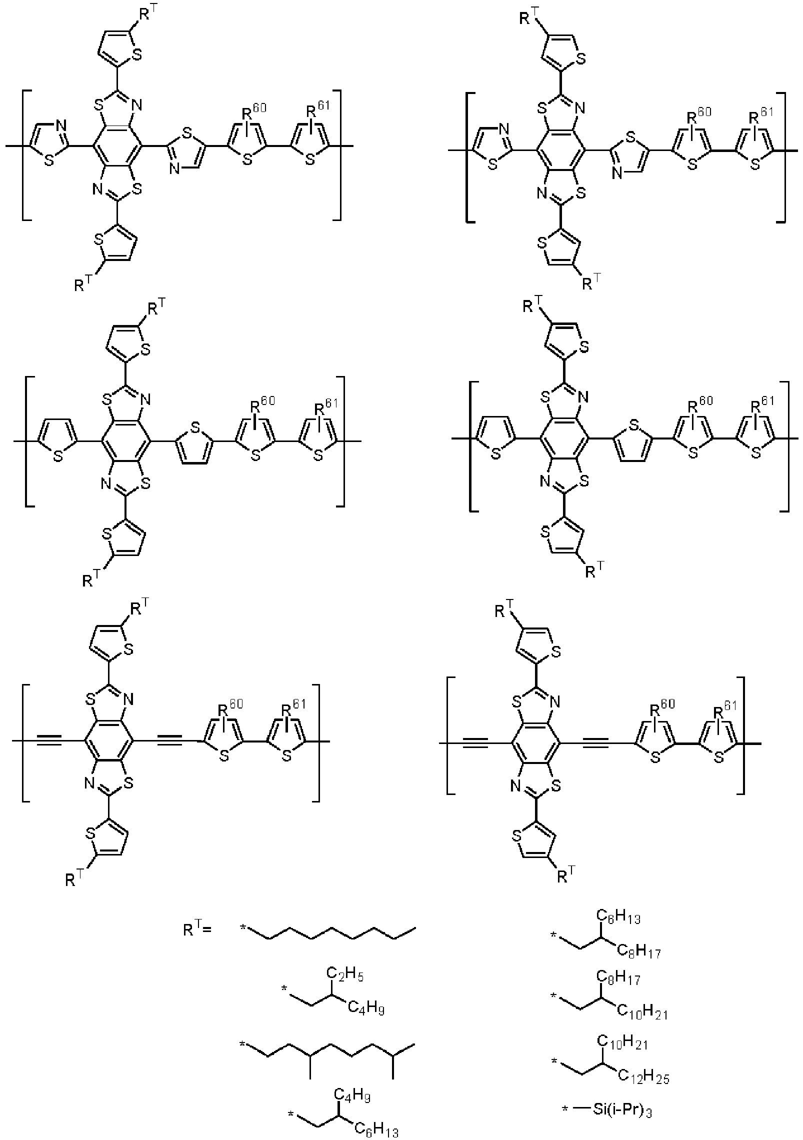

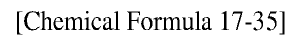

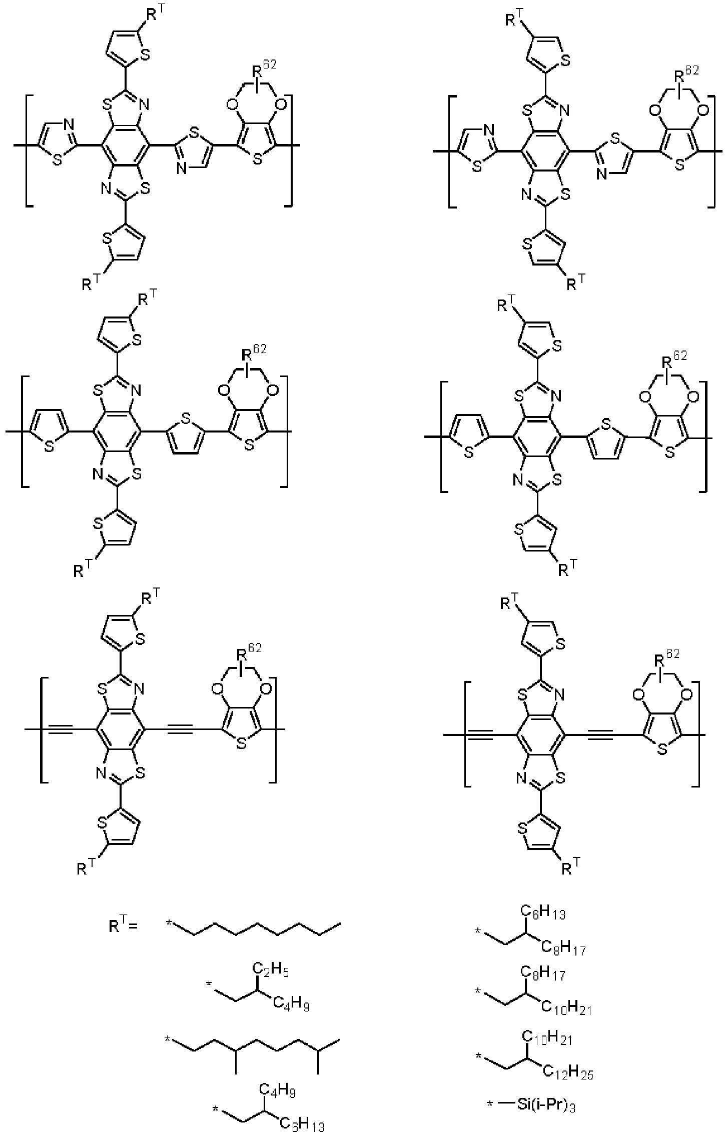

The polymer compound (1) used in the present invention has absorption in a long wavelength region (600 nm or more). The dark current is low in the organic photodetector in which the polymer compound (1) is used.

The dark current in the organic photodetector in which the polymer compound (1) is used is preferably 1×10⁻³ mA/cm² or lower when -2 V is applied. The dark current in the organic photodetector is more preferably 1×10⁻⁴ mA/cm² or lower and further preferably 1×10⁻⁵ mA/cm² or lower since minute light having a wider variety of wavelengths can be detected, and the organic photodetector becomes useful. The lower limit thereof is not particularly limited, but may be higher than 1×10⁻² mA/cm² from an industrial viewpoint, and may be 5×10⁻² mA/cm² or higher.

Photocurrent in the organic photodetector in which the polymer compound (1) is used is preferably 1×10⁻² mA/cm² or higher, more preferably 1×10⁻¹ mA/cm² or higher, and further preferably 1 mA/cm² or higher when -2 V is applied. The upper limit thereof is not particularly limited, but may be lower than 1×10⁻³ mA/cm² from an industrial viewpoint, and may be 5×10⁻³ mA/cm² or lower.

A ratio (photocurrent/dark current) of the photocurrent to the dark current in the organic photodetector in which the polymer compound (1) is used is preferably more than 1×10³ when -2 V is applied. The ratio is more preferably 1×10⁴ or more and further preferably 1×10⁵ or more since the power generation efficiency is enhanced. The upper limit of the ratio is not particularly limited, but may be 1×10¹⁰ or smaller from an industrial viewpoint, and may be 1×10⁸ or smaller.

Meanwhile, a ratio (photocurrent/dark current) of the photocurrent to the dark current in the organic photodetector disclosed in Non-Patent Literature 1 is about 1×10³ when -2 V is applied, and does not reach an industrially practical level and is not satisfactory.

The polymer compound (1) has high solubility with respect to a solvent, and is thus advantageous in that application and film formation are facilitated. Furthermore, since a solvent can be selected from a wider range of solvents for performing the application and film formation, a solvent that is more suitable to film formation can be selected, and the quality of the film formed in the active layer can be enhanced. This is also considered to be one factor of the low dark current in the organic photodetector in which the polymer compound (1) according to the present invention is used.

In general, each of the weight-average molecular weight and the number-average molecular weight of the polymer compound (1) of the present invention is preferably 2,000 or more and 500,000 or smaller and more preferably 3,000 or more and 200,000 or smaller. The weight-average molecular weight and the number-average molecular weight of the polymer compound (1) of the present invention can be calculated based on a calibration curve generated by using polystyrene as a standard sample with use of gel permeation chromatography.

The light absorption maximum wavelength (λmax) of the polymer compound (1) according to the present invention is preferably 400 nm or more and more preferably 450 nm or more, and usually 1200 nm or smaller, preferably 1000 nm or smaller, and more preferably 900 nm or smaller. The half-width is usually 10 nm or more and preferably 20 nm or more, and usually 300 nm or smaller. The polymer compound (1) according to the present invention in which the absorption wavelength range is closer to the wavelength range of visible light, is more preferable.

The solubility of the polymer compound (1) according to the present invention is not particularly limited. However, preferably, the solubility with respect to chlorobenzene at 25°C is usually 0.1 weight % or more, more preferably 0.4 weight % or more, and further preferably 0.8 weight % or more, and is usually 30 weight % or smaller and preferably 20 weight %. The high solubility is preferable in that film formation for a thicker active layer can be performed.

In the polymer compound (1) according to the present invention, molecules preferably interact with each other. In the present invention, that molecules interact with each other means that a distance between polymer chains is shortened by, for example, the π-π stacking interaction among molecules of the polymer compound. The stronger the interaction is, the higher carrier mobility and/or crystallinity of the polymer compound tend to be. That is, it is considered that, since charge transfer among molecules easily occurs in the polymer compound in which the molecules interact with each other, holes generated at the interface between the p-type semiconductor compound (the polymer compound (1)) and the n-type semiconductor compound in the active layer (IV) can be efficiently transported to the anode (VI).

### (Method for producing polymer compound (1))

The method for producing the polymer compound (1) used in the present invention is not particularly limited. For example, in the production method, one compound selected from the group consisting of 2,6-diiodobenzo[1,2-d:4,5-d']bisthiazole and 2,6-dibromobenzo[1,2-d:4,5-d']bisthiazole is used as a starting material, and the polymer compound is obtained through a compound represented by formula (3), 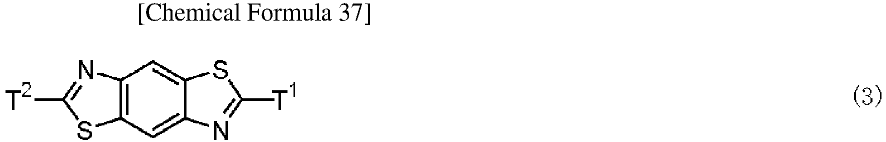
[in formula (3), T¹ and T² each independently represent an alkoxy group, a thioalkoxy group, a thiophene ring that is optionally substituted with a hydrocarbon group or an organosilyl group, a thiazole ring that is optionally substituted with a hydrocarbon group or an organosilyl group, or a phenyl group that is optionally substituted with a hydrocarbon group, an alkoxy group, a thioalkoxy group, an organosilyl group, a halogen atom, or a trifluoromethyl group.]
a compound represented by formula (4), and 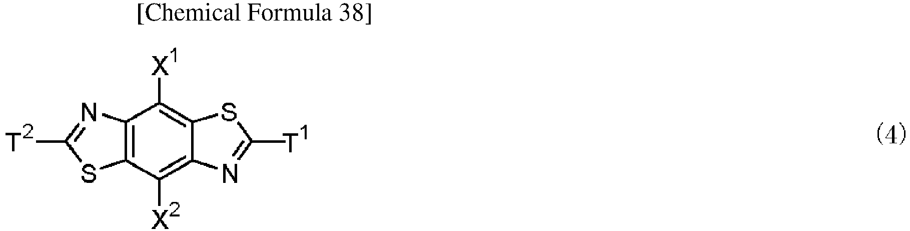 [in formula (4), T¹ and T² each represent a group that is the same as the above-described group. X¹ and X² each represent chlorine, bromine, or iodine.]
a compound represented by formula (5). 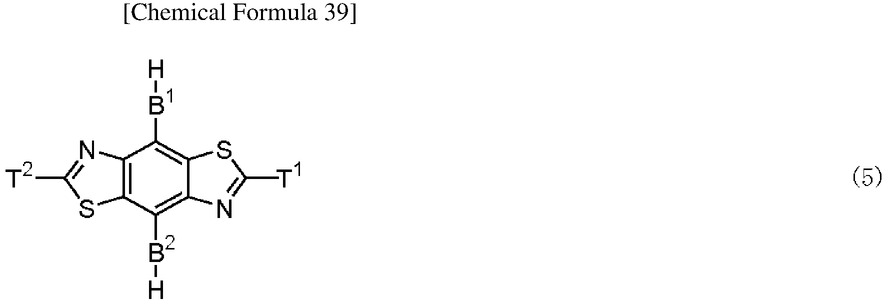 [in formula (5), T¹ and T² each represent a group that is the same as the above-described group. B¹ and B² each represent a thiophene ring that is optionally substituted with a hydrocarbon group, a thiazole ring that is optionally substituted with a hydrocarbon group, or an ethynylene group.].

In the method for producing the polymer compound (1) used in the present invention, the polymer compound (1) is more preferably produced through a compound represented by formula (6). 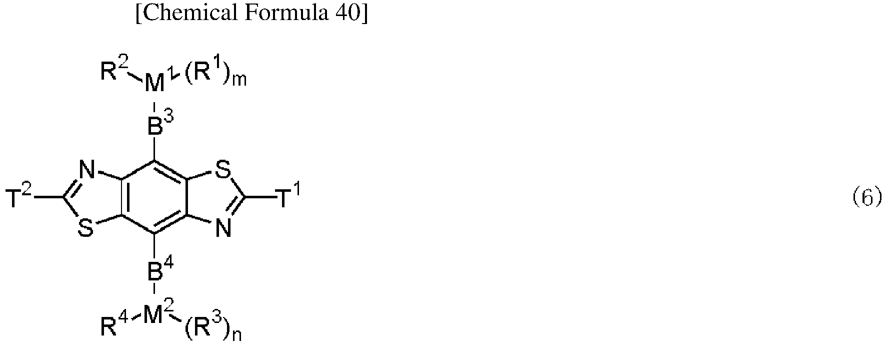 [in formula (6), T¹ and T² each represent a group that is the same as the above-described group. B³ and B⁴ represent groups that are same as the groups of B¹ and B². R¹ to R⁴ each independently represent a C1 to C6 aliphatic hydrocarbon group, a hydroxy group, a C1 to C6 alkoxy group, or a C6 to C10 aryloxy group. M¹ and M² each independently represent a boron atom or a tin atom. R¹ and R² may form a ring with M¹, and R³ and R⁴ may form a ring with M². m and n each represent an integer of 1 or 2. When m and n represent 2, the plurality of R¹s may be the same or different from each other and the plurality of R³s may be the same or different from each other.]

The compound represented by formula (6) can be produced, for example, as follows.
First step: causing 2,6-dihalogenated benzo(bis)thiazole and a compound represented by formula (7) and/or formula (8) to react with each other in the presence of a metal catalyst, and obtaining the compound represented by formula (3). 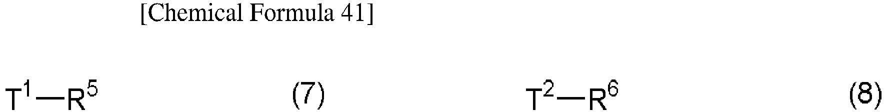 [in formulas (7) and (8), T¹ and T² each represent a group that is the same as the above-described group. R⁵ and R⁶ each independently represent a hydrogen atom or *-M³(R⁷)ₖR⁸. R⁷ and R⁸ each independently represent a C1 to C6 aliphatic hydrocarbon group, a hydroxy group, a C1 to C6 alkoxy group, or a C6 to C10 aryloxy group. M³ represents a boron atom or a tin atom. * represents a bond moiety. R⁷ and R⁸ may form a ring with M³. k represents an integer of 1 or 2. When k represents 2, the plurality of R⁷s may be the same or different from each other.]

Second step: causing the compound represented by formula (3) to react with a base and a halogenating reagent and thus obtaining the compound represented by formula (4).

Furthermore, in a step including the following third step, fourth step, and fifth step, the compound represented by formula (6) can be obtained.

Third step: causing the compound represented by formula (4) to react with a compound represented by the following formula (9) and/or formula (10) in the presence of a metal catalyst and thus obtaining the compound represented by formula (5). 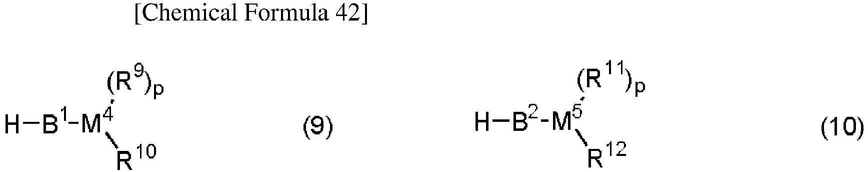 [In formulas (9) and (10), B¹ and B² each represent a group that is the same as the above-described group. R⁹ to R¹² each independently represent a C1 to C6 aliphatic hydrocarbon group, a hydroxy group, a C1 to C6 alkoxy group, a C6 to C10 aryl group, or a C6 to C10 aryloxy group. M⁴ and M⁵ each represent a boron atom, a tin atom, or a silicon atom. R⁹ and R¹⁰ may form a ring with M⁴, and R¹¹ and R¹² may form a ring with M⁵. p and q each represent an integer of 1 or 2. When p represents 2, the plurality of R⁹s may be the same or different from each other. When q represents 2, the plurality of R¹¹s may be the same or different from each other.]

Fourth step: causing the compound represented by formula (5) to react with a base and a tin halide compound and thus obtaining the compound represented by formula (6).

In the present invention, in a case where B¹ and B² of the compound represented by formula (5) represent a thiophene ring (preferably a group represented by formula (b1)) which is optionally substituted with a hydrocarbon group, or a thiazole ring (preferably a group represented by formula (b2)) which is optionally substituted with a hydrocarbon group, the fourth step is preferably included.

### Coupling reaction

Furthermore, the polymer compound (1) can be produced as a donor-acceptor polymer compound by alternately combining the structural unit represented by formula (1) and the copolymerization component (2) through a coupling reaction.

The coupling reaction can be performed by causing the compound represented by formula (6) to react with any of compounds represented by the following formulas (C1) to (C31) in the presence of a metal catalyst. 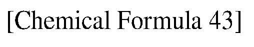 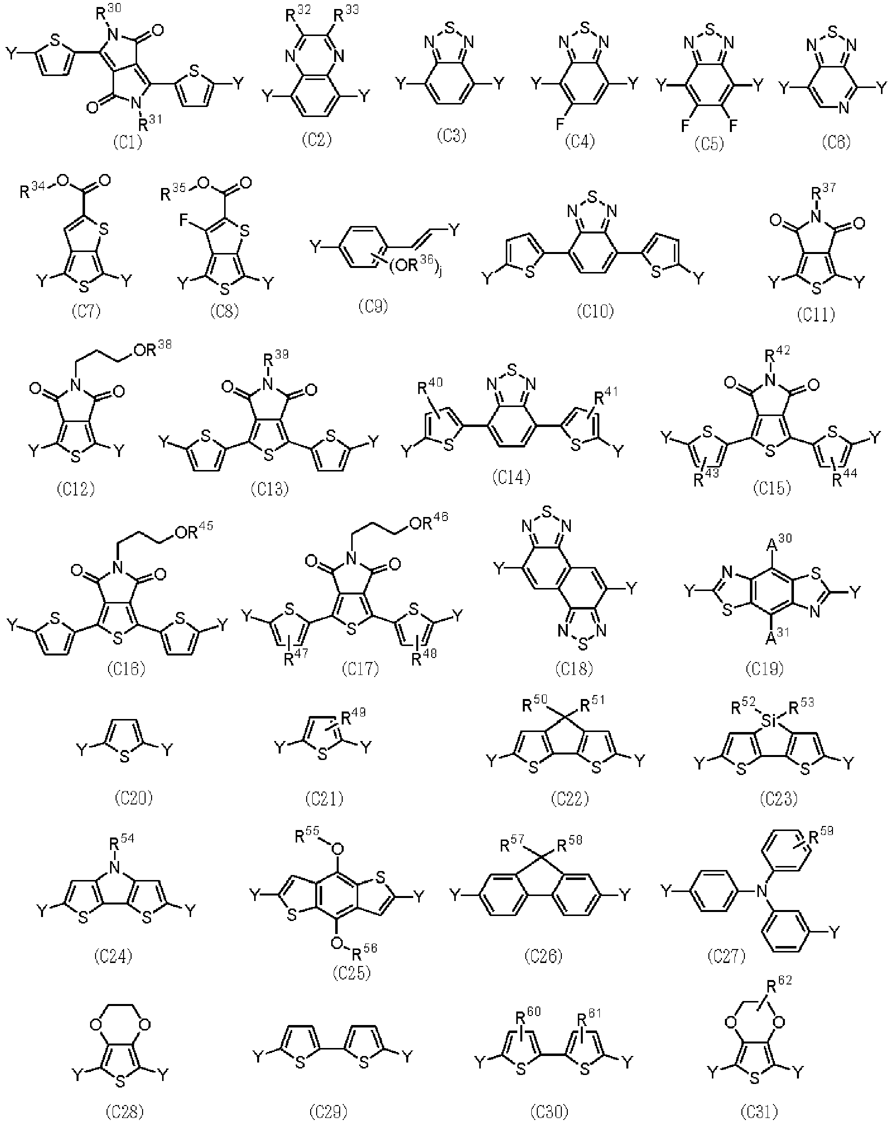 [in formulas (C1) to (C31), R³⁰ to R⁶² each independently represent a group that is the same as the C6 to C30 hydrocarbon group of R¹³ to R¹⁷, R¹⁹ to R²⁰, and R^{15'} and A³⁰ and A³¹ each independently represent a group that is the same as the group of T¹ and T². Y represents a halogen atom. j represents an integer of 1 to 4.]

### (Other p-type semiconductor compounds)

The active layer (IV) contains at least the polymer compound (1) according to the present invention as the p-type semiconductor compound. However, a p-type semiconductor compound different from the polymer compound (1) may be mixed and/or stacked with the polymer compound (1) and used in combination. Examples of the other p-type semiconductor compound to be used in combination include, but is not particularly limited to, an organic semiconductor compound (11). The organic semiconductor compound (11) will be described below. The organic semiconductor compound (11) may be a high molecular weight organic semiconductor compound or a low molecular weight organic semiconductor compound. However, the organic semiconductor compound (11) is preferably a high molecular weight organic semiconductor.

### (Organic semiconductor compound (11))

Examples of the organic semiconductor compound (11) include, but are not particularly limited to, conjugated copolymer semiconductor compounds such as polythiophene, polyfluorene, polyphenylenevinylene, polythienylenevinylene, polyacetylene, and polyaniline; and copolymer semiconductor compounds such as oligothiophene in which an alkyl group or another substituent is substituted, and further include a copolymer semiconductor compound in which two or more kinds of monomer units are copolymerized. As the conjugated copolymer, for example, copolymers described in a known document such as Handbook of Conducting Polymers, the 3rd Ed. (total two volumes), 2007, J. Polym. Sci. Part A: Polym. Chem. 2013, 51, 743-768, J. Am. Chem. Soc. 2009, 131, 13886-13887, Angew. Chem. Int. Ed. 2013, 52, 8341-8344, Adv. Mater. 2009, 21, 2093-2097, or derivatives thereof, and a copolymer that can be synthesized by a combination of the described monomers, can be used. The organic semiconductor compound (11) may be one kind of a compound or a mixture of a plurality of kinds of compounds. By using the organic semiconductor compound (11), for example, increase of an amount of absorbed light by addition of absorption wavelength bands can be expected.

Specific examples of the organic semiconductor compound (11) include, but are not limited to, the following compounds.

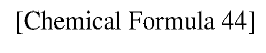

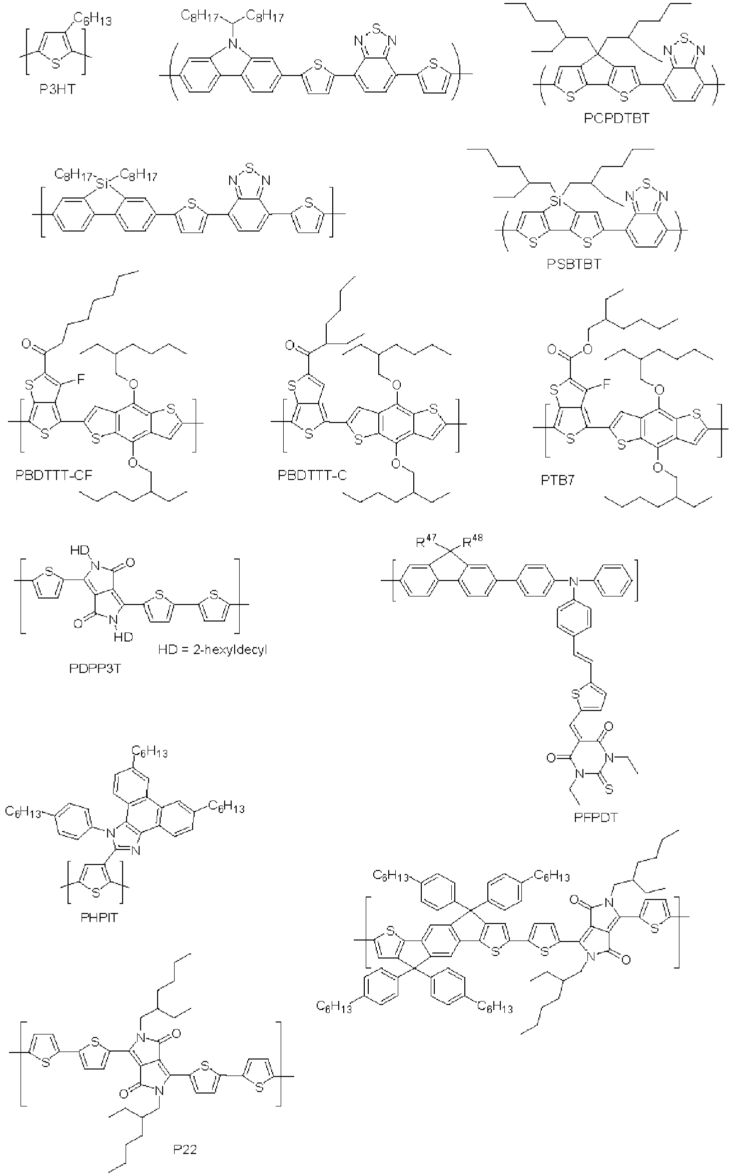

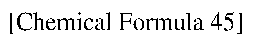

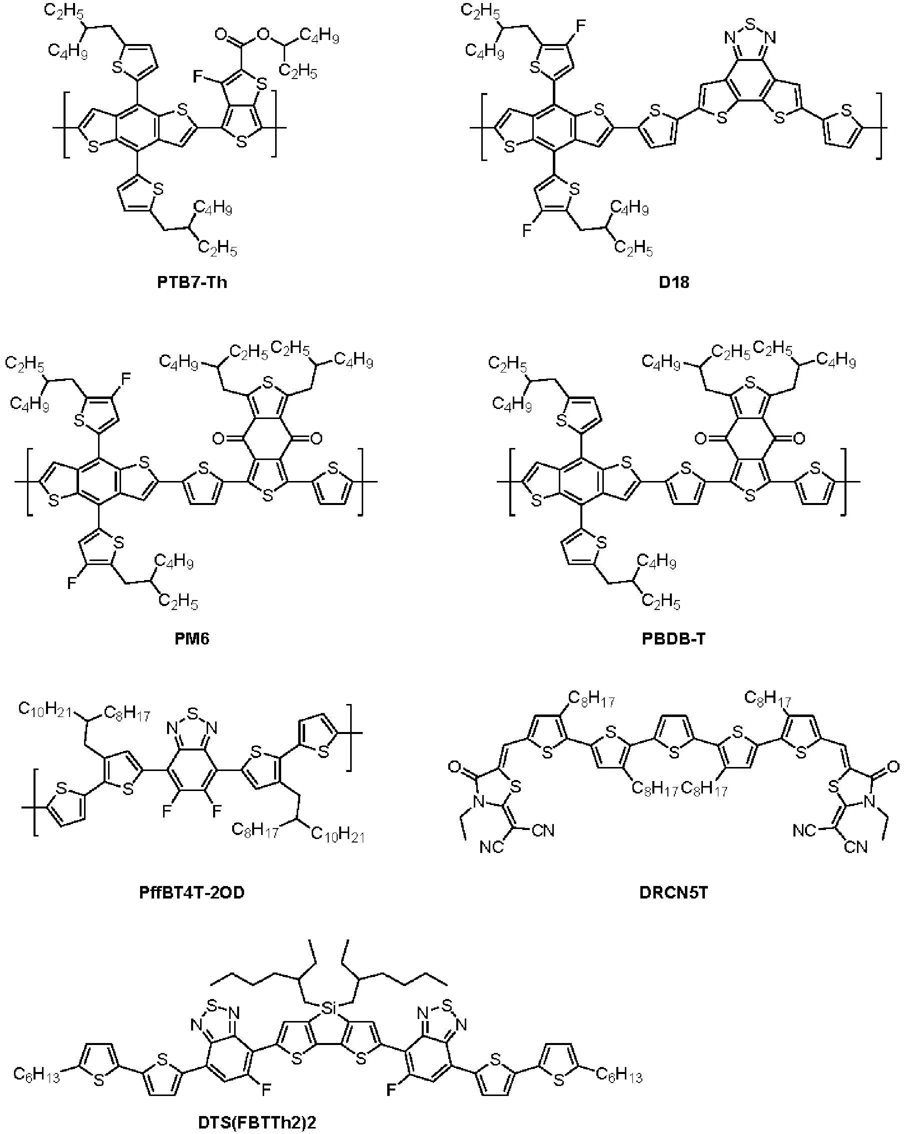

The HOMO (highest occupied molecular orbital) energy level of the p-type semiconductor compound is not particularly limited, and can be selected according to a kind of the n-type semiconductor compound as described below. Particularly, in a case where a fullerene compound is used as the n-type semiconductor compound, the lower limit of the HOMO energy level of the p-type semiconductor compound is usually -7 eV or more, more preferably -6.5 eV or more, and particularly preferably -6.2 eV or more. Meanwhile, the upper limit of the HOMO energy level is usually -4.0 eV or smaller, more preferably -4.5 eV or smaller, and particularly preferably -5.1 eV or smaller. In a case where the HOMO energy level of the p-type semiconductor compound is -6.2 eV or more, characteristics as a p-type semiconductor are enhanced. In a case where the HOMO energy level of the p-type semiconductor compound is -5.1 eV or smaller, stability of the p-type semiconductor compound is enhanced.

The LUMO (lowest unoccupied molecular orbital) energy level of the p-type semiconductor compound is not particularly limited, and can be selected according to a kind of the n-type semiconductor compound as described below. Particularly, in a case where a fullerene compound is used as the n-type semiconductor compound, the LUMO energy level of the p-type semiconductor compound is usually -4.5 eV or more and preferably -4.3 eV or more. Meanwhile, the LUMO energy level is usually -2.5 eV or smaller and preferably -2.7 eV or smaller. In a case where the LUMO energy level of the p-type semiconductor is -2.5 eV or smaller, a band gap is adjusted, long wavelength light energy can be effectively absorbed, and short-circuit current density is enhanced. In a case where the LUMO energy level of the p-type semiconductor compound is -4.5 eV or more, transfer of electrons to the n-type semiconductor compound easily occurs, and sensitivity is enhanced.

Examples of a method for calculating the LUMO energy level and the HOMO energy level include a method for theoretically obtaining the levels as calculated values and a method for actually measuring the levels. Examples of the method for theoretically obtaining the levels as calculated values include a semiempirical molecular orbital method and a non-empirical molecular orbital method. Examples of the method for actually measuring the levels include an ultraviolet-visible absorption spectrum measuring method, and a cyclic voltammetry, an ultraviolet photoelectron spectrometer, and a photoelectron yield spectrometer (ionization potential measuring apparatus) at ordinary temperature under ordinary pressure.

### [1.1.3 n-type semiconductor compound]

The n-type organic semiconductor compound is not particularly limited, and is a π electron conjugated compound in which the lowest unoccupied orbital (LUMO) level is 3.5 to 4.5 eV, in general. Examples of the n-type organic semiconductor compound include fullerenes and derivatives thereof, octaazaporphyrin, etc., a perfluoro compound (for example, perfluoropentacene or perfluorophthalocyanine) obtained by substituting a hydrogen atom of the p-type organic semiconductor compound with a fluorine atom, and a polymer compound which contains, as a backbone, aromatic carboxylic anhydrides and imidized products thereof such as naphthalenetetracarboxylic anhydride, naphthalenetetracarboxylic diimide, perylenetetracarboxylic anhydride, and perylenetetracarboxylic diimide.

Among these n-type organic semiconductor compounds, fullerenes or derivatives thereof are preferable since charge separation can be efficiently performed, at a high speed, with respect to the polymer compound (1) (p-type organic semiconductor compound) having the specific structural unit of the present invention.

Examples of the fullerenes and the derivatives thereof include C60 fullerene, C70 fullerene, C76 fullerene, C78 fullerene, C84 fullerene, C240 fullerene, C540 fullerene, mixed fullerene, fullerene nanotubes, and fullerene derivatives obtained by substituting a part of these fullerenes with a hydrogen atom, a halogen atom, or substituted or unsubstituted alkyl group, alkenyl group, alkynyl group, aryl group, heteroaryl group, cycloalkyl group, silyl group, ether group, thioether group, amino group, silyl group, or the like.

Preferable examples of the fullerene derivative include phenyl-C61-butyric acid ester, diphenyl-C62-bis(butyric acid ester), phenyl-C71-butyric acid ester, phenyl-C85-butyric acid ester, and thienyl-C61-butyric acid ester. The number of carbon atoms of an alcohol moiety of the above-described butyric acid ester is preferably 1 to 30, more preferably 1 to 8, further preferably 1 to 4, and most preferably 1.

Preferable examples of the fullerene derivatives include phenyl-C61-butyric acid methyl ester ([60]PCBM), phenyl-C61-butyric acid n-butyl ester ([60]PCBnB), phenyl-C61-butyric acid isobutyl ester ([60]PCBiB), phenyl-C61-butyric acid n-hexyl ester ([60]PCBH), phenyl-C61-butyric acid n-octyl ester ([60]PCBO), diphenyl-C62-bis(butyric acid methyl ester) (bis[60]PCBM), phenyl-C71-butyric acid methyl ester ([70]PCBM), phenyl-C85-butyric acid methyl ester ([84]PCBM), thienyl-C61-butyric acid methyl ester ([60]ThCBM), C60 pyrrolidine tris acid, C60 pyrrolidine tris acid ethyl ester, N-methylfulleropyrrolidine (MP-C60), (1,2-methanofullerene C60)-61-carboxylic acid, (1,2-methanofullerene C60)-61-carboxylic acid t-butyl ester, metallocene fullerene disclosed in Japanese Laid-Open Patent Publication No. 2008-130889, and a fullerene having a cyclic ether group as disclosed in US Patent No. 7,329,709.

As the n-type organic semiconductor compound, compounds other than fullerenes can also be used. Specific examples of the compounds include, but are not particularly limited to, the following compounds.

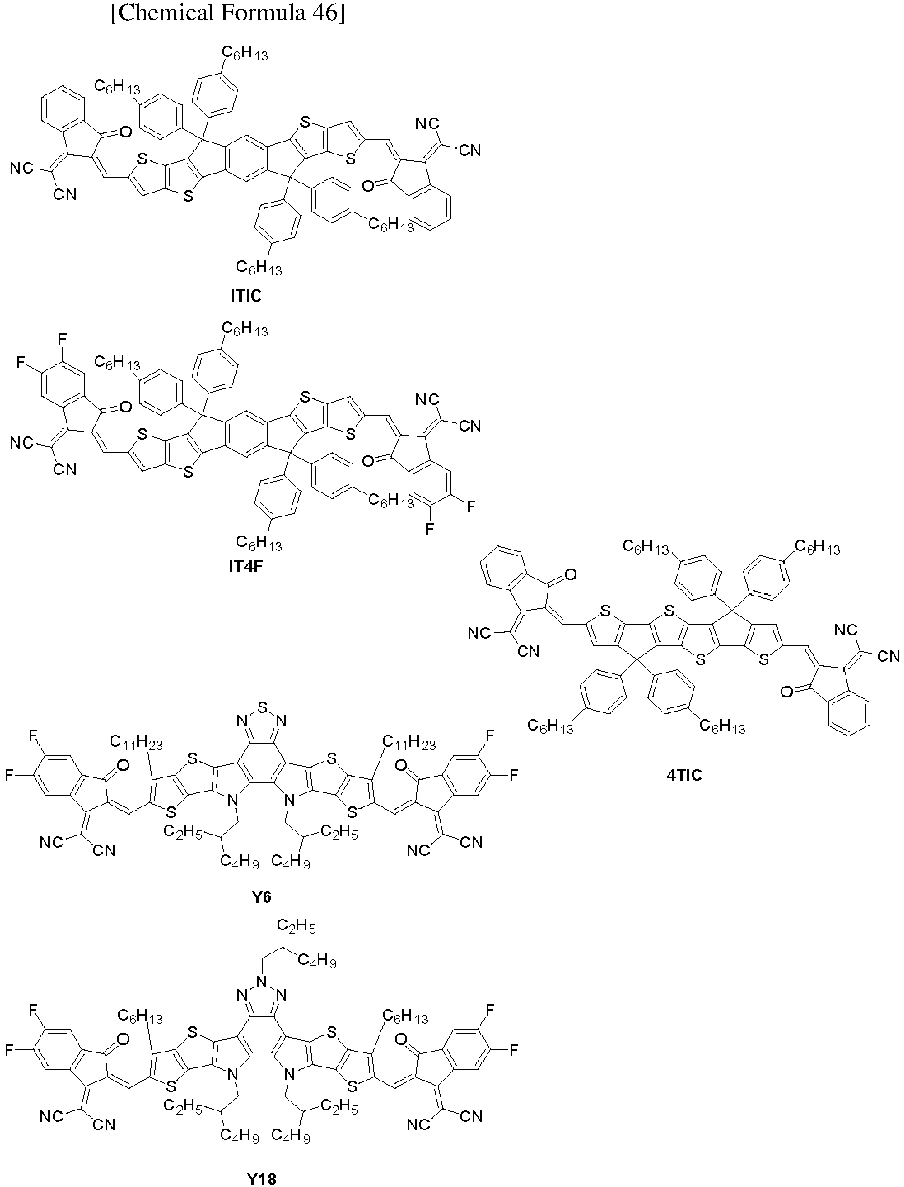

### <1.2 Cathode (II), Anode (VI)>

The cathode (II) and the anode (VI) function to collect holes and electrons generated by light absorption. Therefore, as a pair of electrodes, an electrode (II) (cathode) suitable for collecting electrons and an electrode (VI) (anode) suitable for collecting holes are preferably used. At least one of the paired electrodes may be translucent, and both of them may be translucent. Translucent means that 40% or more of sunlight is transmitted. A solar ray transmittance of a transparent electrode that is translucent is preferably 70% or more in order to allow light to be transmitted through the transparent electrode and to reach the active layer (IV). The transmittance of light can be measured by a standard spectrophotometer.

The cathode (II) is an electrode that is formed of an electrically conductive material in which a value of a work function is lower than that of an anode in general, and that has a function of smoothly extracting electrons generated at the active layer (IV).

Examples of the material of the cathode (II) include electrically conductive metal oxides such as nickel oxide, tin oxide, indium oxide, indium tin oxide (ITO), indium-zirconium oxide (IZO), titanium oxide, indium oxide, and zinc oxide; and metals such as gold, platinum, silver, chromium, and cobalt and alloys thereof. These substances are preferable since a value of the work function is low, and, furthermore, are preferable since an electrically conductive polymer material that is typified by PEDOT:PSS obtained by doping a polythiophene derivative with polystyrene sulfonate can be stacked. In a case where such an electrically conductive polymer is stacked, since a value of the work function of the electrically conductive polymer material is high, a wide range of metals such as aluminum and magnesium suitable for a cathode instead of a material having a small value of the work function as described above can be used.

In a case where the cathode (II) is a transparent electrode, an electrically conductive metal oxide, such as ITO, zinc oxide, or tin oxide, which is translucent is preferably used, and ITO is particularly preferably used.

The film thickness of the cathode (II) is, but is not particularly limited to, usually 10 nm or more, preferably 20 nm or more, and more preferably 50 nm or more. Meanwhile, the film thickness thereof is usually 10 µm or smaller, preferably 1 µm or smaller, and more preferably 500 nm or smaller. In a case where the film thickness of the cathode (II) is 10 nm or more, sheet resistance is reduced. In a case where the film thickness of the cathode (II) is 10 µm or smaller, light can be efficiently converted to electricity. In a case where the cathode (II) is a transparent electrode, a film thickness that can achieve both the transmittance of light and sheet resistance needs to be selected.

The sheet resistance of the cathode (II) is, but is not particularly limited to, usually 1 Ω/sq or higher, and 1000 Ω/sq or lower, preferably 500 Ω/sq or lower, and more preferably 100 Ω/sq or lower.

The anode (VI) is an electrode that is formed of an electrically conductive material in which a value of a work function is higher than that of a cathode in general, and that has a function of smoothly extracting holes generated at the active layer (IV).

Examples of the material of the anode (VI) include metals such as platinum, gold, silver, copper, iron, tin, zinc, aluminum, indium, chromium, lithium, sodium, potassium, cesium, calcium, and magnesium, and alloys thereof; inorganic salts such as lithium fluoride and cesium fluoride; and metal oxides such as nickel oxide, aluminum oxide, lithium oxide, and cesium oxide. These materials are preferable since a value of the work function is high. In a case where an n-type semiconductor compound such as zinc oxide having an electric conductivity is used as a material of the hole transport layer (V), a material such as indium tin oxide (ITO) in which a value of the work function is low can be used as the material of the anode (VI). From the viewpoint of protecting the electrode, the material of the cathode (II) is preferably a metal such as platinum, gold, silver, copper, iron, tin, aluminum, calcium, or indium, or an alloy using the metal.

The film thickness of the anode (VI) is, but is not particularly limited to, usually 10 nm or more, preferably 20 nm or more, and more preferably 50 nm or more. Meanwhile, the film thickness thereof is usually 10 µm or smaller, preferably 1 µm or smaller, and more preferably 500 nm or smaller. In a case where the film thickness of the anode (VI) is 10 nm or more, sheet resistance is reduced. In a case where the film thickness of the anode (VI) is 10 µm or smaller, light can be efficiently converted to electricity without reducing the transmittance of light. In a case where the anode (VI) is used as a transparent electrode, a film thickness that achieves both the transmittance of light and sheet resistance needs to be selected.

The sheet resistance of the anode (VI) is, but is not particularly limited to, usually 1000 Ω/sq or lower, preferably 500 Ω/sq or lower, and more preferably 100 Ω/sq or lower. The lower limit is, but is not limited to, usually 1 Ω/sq or higher.

Examples of a method for forming the anode (VI) include a vacuum film formation method such as a vapor deposition method and a sputtering method, and a wet application method for applying ink containing nanoparticles or a precursor and thus forming a film.

Furthermore, each of the cathode (II) and the anode (VI) may have a laminate structure formed of two or more layers. The cathode (II) and the anode (VI) may have characteristics (electric characteristics, wetting characteristics, or the like) improved through surface treatment.

### <1.3 Base material (I)>

The organic photodetector (VII) usually has the base material (I) serving as a support body. That is, the electrodes (II), (VI) and the active layer (IV) are formed on the base material.

A material of the base material (I) is not particularly limited as long as the effect of the present invention is not significantly impaired. Preferable examples of the material of the base material (I) include inorganic materials such as quartz, glass, sapphire, and titania; organic materials such as polyethylene terephthalate, polyethylene naphthalate, polyether sulfone, polyimide, nylon, polystyrene, polyvinyl alcohol, ethylene-vinylalcohol copolymers, fluororesin films, vinyl chloride, polyolefin like polyethylene, cellulose, polyvinylidene chloride, aramid, polyphenylene sulfide, polyurethane, polycarbonate, polyarylate, polynorbornene, and epoxy resin; paper materials such as paper and synthetic paper; and composite materials in which, for example, the surface of a metal such as stainless steel, titanium, or aluminum is subjected to coating or lamination in order to impart insulating properties.

Examples of glass include soda glass, blue plate glass, and alkali-free glass. Among them, alkali-free glass is preferable since an amount of ions eluted from glass is small.

The shape of the base material (I) is not limited, and may be, for example, a platelike shape, a film-like shape, or a sheet-like shape. The film thickness of the base material (I) is, but is not limited to, usually 5 µm or more and preferably 20 µm or more, and is usually 20 mm or smaller and preferably 10 mm or smaller. In a case where the film thickness of the base material (I) is 5 µm or more, strength of the organic photodetector is unlikely to become insufficient, and it is thus preferable. In a case where the film thickness of the base material (I) is 20 mm or smaller, cost is reduced and the weight is not increased, and it is thus preferable. In a case where the material of the base material (I) is glass, the film thickness is usually 0.01 mm or more and preferably 0.1 mm or more, and is usually 1 cm or smaller and preferably 0.5 cm or smaller. In a case where the film thickness of the glass base material (I) is 0.01 mm or more, mechanical strength is increased and breakage can be inhibited, and it is thus preferable. In a case where the film thickness of the glass base material (I) is 0.5 cm or smaller, the weight is not increased, and it is thus preferable.

### <1.4 Buffer layer (III, V)>

The organic photodetector (VII) preferably has the buffer layer (III) between the active layer (IV) and the cathode (II) (hereinafter, may also be referred to as "electrode (II)") and has the buffer (V) between the active layer (IV) and the anode (VI) (hereinafter, may also be referred to as "electrode (VI)"). The buffer layers can be classified into the hole transport layer (V) and the electron transport layer (III). In a case where the buffer layer is disposed, electrons or holes are easily transferred between the active layer (IV) and the cathode (II), and, furthermore, shortcircuiting between the electrodes can be prevented. However, in the present invention, the buffer layers (III) and (V) may not necessarily be disposed.

The hole transport layer (V) and the electron transport layer (III) are disposed between the pair of electrodes (II) and (VI) such that the active layer (IV) is disposed between the hole transport layer (V) and the electron transport layer (III). That is, in a case where the organic photodetector (VII) according to the present invention includes both the hole transport layer (V) and the electron transport layer (III), the anode (VI), the hole transport layer (V), the active layer (IV), the electron transport layer (III), and the cathode (II) are disposed in order, respectively. In a case where the organic photodetector (VII) according to the present invention includes the hole transport layer (V) and does not include the electron transport layer (III), the anode (VI), the hole transport layer (V), the active layer (IV), and the cathode (II) are disposed in order, respectively.

### [1.4.1 Electron transport layer (III)]

The electron transport layer (III) is a layer at which electrons are extracted from the active layer (IV) into the cathode (II), is not particularly limited as long as the electron transport layer (III) is formed of an electron-transporting material for enhancing the electron extracting efficiency, and may be formed of an organic compound or an inorganic compound. However, an inorganic compound is preferable.

Preferable examples of the inorganic compound material include salts of alkali metals such as lithium, sodium, potassium, and cesium, and metal oxides. Among them, fluoride salts such as lithium fluoride, sodium fluoride, potassium fluoride, and cesium fluoride are preferably used as salts of alkali metals, and metal oxides, such as titanium oxide (TiOx) and zinc oxide (ZnO), which have n-type semiconductor characteristics are preferably used as metal oxides. The inorganic compound material is more preferably a metal oxide such as titanium oxide (TiOx) or zinc oxide (ZnO) which has n-type semiconductor characteristics. Titanium oxide (TiOx) is particularly preferable. The operation mechanism of such a material is unclear. However, it is considered that, when the material is used in combination with the cathode (VI), the value of a work function is reduced, and a voltage to be applied into a solar cell element is enhanced.

The LUMO energy level of the material of the electron transport layer (III) is, but is not particularly limited to, usually -4.0 eV or more and preferably -3.9 eV or more, and is usually -1.9 eV or smaller and preferably -2.0 eV or smaller. It is preferable that the LUMO energy level of the material of the electron transport layer (III) is -1. 9 eV or smaller since charge transfer is promoted. It is preferable that the LUMO energy level of the material of the electron transport layer (III) is -4.0 eV or more since reverse electron transfer to the n-type semiconductor compound can be prevented.

The HOMO energy level of the material of the electron transport layer (III) is, but is not particularly limited to, usually -9.0 eV or more and preferably -8.0 eV or more, and is usually -5.0 eV or smaller and preferably -5.5 eV or smaller. It is preferable that the HOMO energy level of the material of the electron transport layer (III) is -5.0 eV or smaller since the holes can be inhibited from being transferred. Examples of a method for calculating the LUMO energy level and the HOMO energy level of the material of the electron transport layer (III) include cyclic voltammogram, an ultraviolet photoelectron spectrometer, and a photoelectron yield spectrometer (ionization potential measuring apparatus).

The film thickness of the electron transport layer (III) is, but is not particularly limited to, usually 0.1 nm or more, preferably 0.5 nm or more, and more preferably 1.0 nm or more, and is usually 100 nm or smaller, preferably 70 nm or smaller, more preferably 40 nm or smaller, and particularly preferably 20 nm or smaller. In a case where the film thickness of the electron transport layer (III) is 0.1 nm or more, a function as a buffer material is exhibited. In a case where the film thickness of the electron transport layer (III) is 100 nm or smaller, electrons can be easily extracted, and photoelectric conversion efficiency can be enhanced.

### [1.4.2 Hole transport layer (V)]

The hole transport layer (V) is a layer at which holes are extracted from the active layer (IV) into the anode (VI), and is not particularly limited as long as the hole transport layer (V) is formed of a hole-transporting material capable of enhancing the hole extracting efficiency. Specifically, examples of the material include: electrically conductive organic compounds such as an electrically conductive polymer obtained by doping polythiophene, polypyrrole, polyacetylene, triphenylenediamine, polyaniline, etc., with sulfonic acid and/or iodine, etc., a polythiophene derivative having a sulfonyl group as a substituent, and arylamine; metal oxides such as molybdenum trioxide, vanadium pentoxide, or nickel oxide which have p-type semiconductor characteristics; and the above-described p-type semiconductor compounds. Among them, an electrically conductive polymer obtained by doping with sulfonic acid is preferable, and poly(3,4-ethylenedioxythiophene)poly(styrenesulfonic acid) (PEDOT:PSS) obtained by doping a polythiophene derivative with polystyrene sulfonate, and metal oxides such as molybdenum oxide and vanadium oxide are more preferable. A thin film formed of, for example, a metal such as gold, indium, silver, or palladium can also be used. The thin film formed of a metal or the like may be formed alone, or may be formed in combination with the above-described organic material.

The film thickness of the hole transport layer (V) is, but is not particularly limited to, usually 0.2 nm or more, preferably 0.5 nm or more, and more preferably 1.0 nm or more, and is usually 400 nm or smaller, preferably 200 nm or smaller, more preferably 100 nm or smaller, and particularly preferably 70 nm or smaller. In a case where the film thickness of the hole transport layer (V) is 0.2 nm or more, a function as a buffer material is exhibited. In a case where the film thickness of the hole transport layer (V) is 400 nm or smaller, holes can be easily extracted, and photoelectric conversion efficiency can be enhanced.

A method for forming the electron transport layer (III) and the hole transport layer (V) is not limited. For example, in a case where a sublimable material is used, the electron transport layer (III) and the hole transport layer (V) can be formed by a vacuum vapor deposition method or the like. For example, in a case where a material that is soluble in a solvent is used, the electron transport layer (III) and the hole transport layer (V) can be formed by, for example, a wet application method such as a spin coating method and an ink jet method. In a case where a semiconductor compound is used for the electron transport layer (III), a layer containing a semiconductor compound precursor is formed, and the precursor may be thereafter converted to a semiconductor compound, similarly to the active layer (IV).

### <1.5 Method for producing organic photodetector>

A method for producing the organic photodetector (VII) is not particularly limited, and the organic photodetector (VII) can be formed by stacking the base material (I), the cathode (II), the electron transport layer (III), the active layer (IV), the hole transport layer (V), and the anode (VI) in order, respectively, according to the following method. For example, a glass substrate (manufactured by GEOMATEC Co., Ltd.) patterned with an electrically conductive transparent film (cathode) formed of indium tin oxide (ITO) is ultrasonically washed with acetone and then ultrasonically washed with ethanol, is thereafter dried by nitrogen blowing, and is subjected to UV-ozone treatment, whereby a base material having an anode is obtained. Subsequently, 0.5 M of zinc acetate·0.5 M of aminoethanol/2-methoxyethanol solution which is used as an electron transport layer is applied (at 3000 rpm for 40 seconds) by a spin coater, and is thereafter annealed at 175°C for 30 minutes to be converted to zinc oxide, whereby an electron transport layer formed of the zinc oxide can be formed. Thereafter, the obtained product is put into a glove box, spin-coating of a mixture solution of a donor material and an acceptor material is performed in an inert gas atmosphere, and the obtained product is annealed or dried under reduced pressure on a hot plate, whereby an active layer can be formed. Subsequently, molybdenum oxide is deposited under reduced pressure, whereby a hole transport layer can be formed. Finally, silver as an electrode is deposited to form an anode, whereby the organic photodetector can be obtained.

An organic photodetector having a different structure, for example, an organic photodetector which does not have at least one of the electron transport layer (III) and the hole transport layer (V) can also be formed in the same method.

### <1.6 Photoelectric conversion characteristics>

The photoelectric conversion characteristics of the organic photodetector (VII) can be obtained as follows. The organic photodetector (VII) is irradiated with light at an irradiation intensity of 100 mW/cm2 by a solar simulator in the condition that AM is 1.5 G, whereby current-voltage characteristics are measured. The photoelectric conversion characteristics such as the photoelectric conversion efficiency (PCE), a short-circuit current density (Jd), an open circuit voltage (Voc), a fill factor (FF), series resistance, and shunt resistance can be obtained from the obtained current-voltage curve.

### <2. Organic thin-film solar cell according to the present invention>

The organic photodetector (VII) according to the present invention is preferably used as a solar cell element of a solar cell, in particular, an organic thin-film solar cell.

The usage of the organic thin-film solar cell according to the present invention is not limited, and the organic thin-film solar cell can be used for any usage. Examples of the field to which the organic thin-film solar cell according to the present invention can be applied include solar cells for construction materials, solar cells for automobiles, solar cells for interior, solar cells for railroads, solar cells for ships, solar cells for airplanes, solar cells for spacecrafts, solar cells for home appliances, solar cells for mobile phones, and solar cells for toys.

The organic thin-film solar cell according to the present invention may be used as it is, or may be used as a solar cell module by setting a solar cell on the base material (I). Specifically, for example, in a case where a plate material for construction is used as a base material, a thin-film solar cell is disposed on the surface of the plate material, whereby a solar cell panel can be produced as a solar cell module.

### EXAMPLES

### A measurement method used in synthesis examples was as follows.

### (NMR spectrum measurement)

For the benzo(bis)thiazole compound, an NMR spectrum measurement device ("400MR" manufactured by Agilent and "AVANCE NEO 600" manufactured by Bruker) was used to perform NMR spectrum measurement.

One example of synthesis of the polymer compound (1) used in the present invention will be described below. It is needless to say that the polymer compound (1) used in the present invention is not limited by the following synthesis examples, and the synthesis methods themselves can also be appropriately modified and combined without departing from the gist described above and below. In the following description, unless otherwise specified, "part" means "parts by mass", and "%" means "mass%".

### (Synthesis example 1)

Synthesis of 2,6-bis[5-(2-hexyldecyl)thiophene-2-yl]benzo[1,2-d;4,5-d']bisthiazole (DBTH-HDTH)

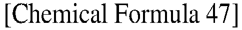

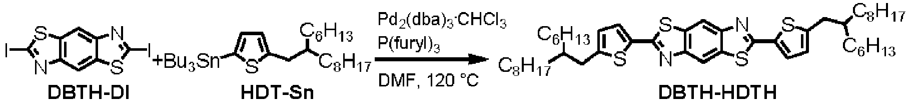

In a 300 mL flask, 2,6-diiodobenzo[1,2-d;4,5-d']bisthiazole (DBTH-DI, 5.2 g, 11.7 mmol), tributyl[5-(2-hexyldecyl)thiophene-2-yl]stannane (HDT-Sn, 23.2 g, 38.6 mmol), tris(2-furyl)phosphine (443 mg, 1.87 mmol), a tris(dibenzylideneacetone)dipalladium (0)-chloroform adduct (490 mg, 0.47 mol), and N,N-dimethylformamide (115 mL) were added and caused to react at 120°C for 23 hours. After the end of the reaction, the obtained product was cooled to room temperature, water was then added, extraction was performed twice with chloroform, and an organic layer was washed with water, and thereafter dried with anhydrous magnesium sulfate. Subsequently, the organic layer was filtered and concentrated to obtain a crude product, and the obtained crude product was purified by column chromatography (silica gel, chloroform/hexane=1/1), to obtain 5.62 g of 2,6-bis[5-(2-hexyldecyl)thiophene-2-yl]benzo[1,2-d;4,5-d']bisthiazole (DBTH-HDTH) as a light yellow solid (the yield was 60%).

Generation of the target compound was confirmed by ¹H-NMR measurement.

### (Synthesis examples 2 to 6)

Similarly to Synthesis example 1, 2,6-bis[5-(3,7-dimethyloctyl)thiophene-2-yl]benzo[1,2-d;4,5-d']bisthiazole (DBTH-DMOTH) (Synthesis example 2), 2,6-bis[5-(2-ethylhexyl)thiophene-2-yl]benzo[1,2-d;4,5-d']bisthiazole (DBTH-EHTH) (Synthesis example 3), 2,6-bis[5-(2-butyloctyl)thiophene-2-yl]benzo[1,2-d;4,5-d']bisthiazole(DBTH-BOTH) (Synthesis example 4), 2,6-bis[5-(2-decyltetradecyl)thiophene-2-yl]benzo[1,2-d;4,5-d']bisthiazole (DBTH-TDTH) (Synthesis example 5), and 2,6-bis(5-triisopropylsilanylthiophene-2-yl)-benzo[1,2-d;4,5-d']bisthiazole (DBTH-TIPSTH) (Synthesis example 6) were obtained. The yield was 38 to 51%.

### (Synthesis example 7)

Synthesis of 2,6-bis[5-(2-hexyldecyl)thiophene-2-yl]-4,8-diiodobenzo[1,2-d;4,5-d']bisthiazole (DI-DBTH-HDTH)

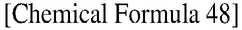

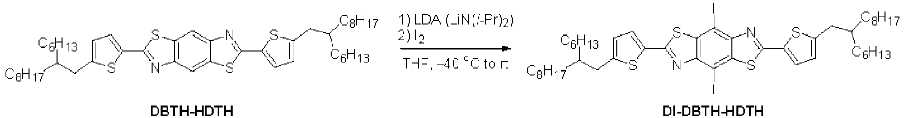

In a 100 mL flask, 2,6-bis[5-(2-hexyldecyl)thiophene-2-yl]benzo[1,2-d;4,5-d']bisthiazole (DBTH-HDTH, 4 g, 4.97 mmol) and tetrahydrofuran (80 mL) were added and cooled to -40°C, lithium diisopropylamide (2 M solution, 5.5 mL, 10.9 mmol) was thereafter dropped, and the obtained product was stirred for 30 minutes. Subsequently, iodine (3.8 g, 14.9 mol) was added, and reaction was thereafter made at room temperature for two hours. After the end of the reaction, 10% sodium hydrogen sulfite was added, extraction was performed with chloroform, and the obtained organic layer was washed with saturated sodium bicarbonate water and then washed with saturated saline solution, and was dried by using anhydrous magnesium sulfate. Subsequently, the organic layer was filtered and concentrated to obtain a crude product, and the obtained crude product was purified by column chromatography (silica gel, chloroform/hexane=1/1), to obtain 2.66 g of 2,6-bis[5-(2-hexyldecyl)thiophene-2-yl]-4,8-diiodobenzo[1,2-d;4,5-d']bisthiazole (DI-DBTH-HDTH) as a yellow solid (the yield was 51%).

Generation of the target compound was confirmed by ¹H-NMR measurement.

### (Synthesis examples 8 to 12)

Similarly to Synthesis example 7, 2,6-bis[5-(3,7-dimethyloctyl)thiophene-2-yl]-4,8-diiodobenzo[1,2-d;4,5-d']bisthiazole (DI-DBTH-DMOTH) (Synthesis example 8), 2,6-bis[5-(2-ethylhexyl)thiophene-2-yl]-4,8-diiodobenzo[1,2-d;4,5-d']bisthiazole (DI-DBTH-EHTH) (Synthesis example 9), 2,6-bis[5-(2-butyloctyl)thiophene-2-yl]-4,8-diiodobenzo[1,2-d;4,5-d']bisthiazole (DI-DBTH-BOTH) (Synthesis example 10), 2,6-bis[5-(2-decyltetradecyl)thiophene-2-yl]-4,8-diiodobenzo[1,2-d;4,5-d']bisthiazole (DI-DBTH-TDTH) (Synthesis example 11), and 4,8-diiodo-2,6-bis-(5-triisopropylsilanylthiophene-2-yl)-benzo[1,2-d;4,5-d']bisthiazole (DI-DBTH-TIPSTH) (Synthesis example 12) were obtained. The yield was 36 to 70%.

### (Synthesis example 13)

Synthesis of 2,6-bis[5-(2-hexyldecyl)thiophene-2-yl]-4,8-dithiophene-2-yl-benzo[1,2-d;4,5-d']bisthiazole (DTH-DBTH-HDTH)

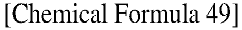

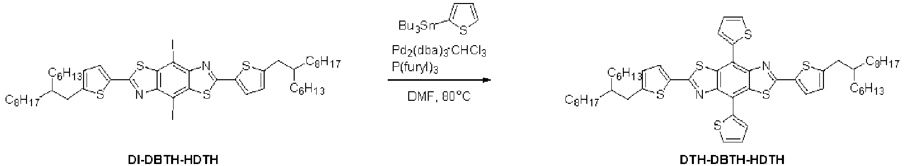

In a 50 mL flask, 2,6-bis[5-(2-hexyldecyl)thiophene-2-yl]-4,8-diiodobenzo[1,2-d;4,5-d']bisthiazole (DI-DBTH-HDTH, 1.1 g, 1.04 mmol), tributylthiophene-2-yl-stannane (830 µL, 2.60 mmol), tris(2-furyl)phosphine (40 mg, 0.17 mmol), a tris(dibenzylideneacetone)dipalladium (0)-chloroform adduct (45 mg, 0.04 mmol), and N,N-dimethylformamide (22 mL) were added and caused to react at 80°C for 19 hours. After the end of the reaction, the obtained product was cooled to room temperature, water was then added, extraction was performed twice with chloroform, and an organic layer was washed with water and thereafter dried with anhydrous magnesium sulfate. Subsequently, the organic layer was filtered and concentrated to obtain a crude product, and the obtained crude product was purified by column chromatography (silica gel, chloroform/hexane=1/1-chloroform), to obtain 1.01 g of 2,6-bis[5-(2-hexyldecyl)thiophene-2-yl]-4,8-dithiophene-2-yl-benzo[1,2-d;4,5-d']bisthiazole (DTH-DBTH-HDTH) as a yellow solid (the yield was 100%).

Generation of the target compound was confirmed by ¹H-NMR measurement.

### (Synthesis examples 14 to 18)

Similarly to Synthesis example 13, 2,6-bis[5-(3,7-dimethyloctyl)thiophene-2-yl]-4,8-dithiophene-2-yl-benzo[1,2-d;4,5-d']bisthiazole (DTH-DBTH-DMOTH) (Synthesis example 14), 2,6-bis[5-(2-ethylhexyl)thiophene-2-yl]-4,8-dithiophene-2-yl-benzo[1,2-d;4,5-d']bisthiazole (DTH-DBTH-EHTH) (Synthesis example 15), 2,6-bis[5-(2-butyloctyl)thiophene-2-yl]-4,8-dithiophene-2-yl-benzo[1,2-d;4,5-d']bisthiazole (DTH-DBTH-BOTH) (Synthesis example 16), 2,6-bis[5-(2-decyltetradecyl)thiophene-2-yl]-4,8-dithiophene-2-yl-benzo[1,2-d;4,5-d']bisthiazole (DTH-DBTH-TDTH) (Synthesis example 17), and 4,8-bis-(thiophene-2-yl)-2,6-bis-(5-triisopropylsilanylthiophene-2-yl)-benzo[1,2-d;4, 5-d']bisthiazole (DBTH-TIPSTH-THA) (Synthesis example 18) were obtained. The yield was 45 to 99%.

### (Synthesis example 19)

### 2,6-bis[5-(2-hexyldecyl)thiophene-2-yl]-4,8-bis(5-tributylstannylthiophene-2-yl)-benzo[1,2-d;4,5-d']bisthiazole (DTH-DBTH-HDTH-DSB)

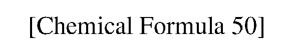

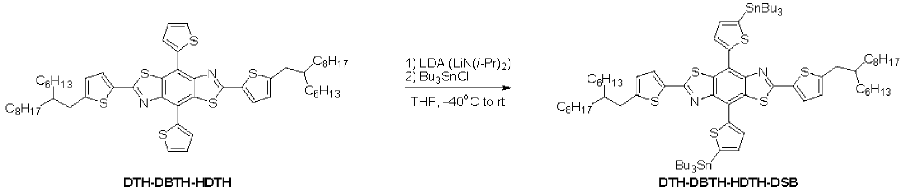

In a 50 mL flask, 2,6-bis[5-(2-hexyldecyl)thiophene-2-yl]-4,8-dithiophene-2-yl-benzo[1,2-d;4,5-d']bisthiazole (DTH-DBTH-HDTH, 602 mg, 0.62 mmol) and tetrahydrofuran (18 mL) were added and cooled to -40°C, lithium diisopropylamide (2 M solution, 0.65 mL, 1.30 mmol) was dropped, and the obtained product was stirred for 30 minutes. Thereafter, tributyltin chloride (352 µL, 1.30 mmol) was added, the temperature was increased to room temperature, and the obtained product was stirred for two hours. After the end of the reaction, water was added, extraction was performed twice with toluene, and an organic layer was washed with water and thereafter dried with anhydrous magnesium sulfate. Subsequently, the organic layer was filtered and concentrated to obtain a crude product, and the obtained crude product was purified by GPC-HPLC (JAIGEL-1H, 2H, chloroform), to obtain 634 mg of 2,6-bis[5-(2-hexyldecyl)thiophene-2-yl]-4,8-bis(5-tributylstannylthiophene-2-yl)-benzo[1,2-d;4,5-d']bisthiazole (DTH-DBTH-HDTH-DSB) as a light brown oil-like product (the yield was 66%).

Generation of the target compound was confirmed by ¹H-NMR measurement.

### (Synthesis examples 20 to 21)

Similarly to Synthesis example 19, 2,6-bis[5-(3,7-dimethyloctyl)thiophene-2-yl]-4,8-bis(5-tributylstannylthiophene-2-yl)-benzo[1,2-d;4,5-d']bisthiazole (DTH-DBTH-DMOTH-DSB) (Synthesis example 20), and 4,8-bis(5-tributylstannylthiophene-2-yl)-2,6-bis(5-triisopropylsilanylthiophene-2-yl)-benzo[1,2-d;4,5-d']bisthiazole (DBTH-TIPSTH-THA-DSB) (Synthesis example 21) were obtained. The yield was 49 to 63%.

### (Synthesis examples 22 to 25)

Similarly to Synthesis example 19, by using trimethyltin chloride instead of tributyltin chloride, 2,6-bis[5-(2-ethylhexyl)thiophene-2-yl]-4,8-bis(5-trimethylstannylthiophene-2-yl)-benzo[1,2-d;4,5-d']bisthiazole (DTH-DBTH-EHTH-DSM) (Synthesis example 22), 2,6-bis[5-(2-butyloctyl)thiophene-2-yl]-4,8-bis(5-trimethylstannylthiophene-2-yl)-benzo[1,2-d;4,5-d']bisthiazole (DTH-DBTH-BOTH-DSM) (Synthesis example 23), 2,6-bis[5-(2-hexyldecyl)thiophene-2-yl]-4,8-bis(5-trimethylstannylthiophene-2-yl)-benzo[1,2-d;4,5-d']bisthiazole (DTH-DBTH-HDTH-DSM) (Synthesis example 24), and 2,6-bis[5-(2-decyltetradecyl)thiophene-2-yl]-4,8-bis(5-trimethylstannylthiophene-2-yl)-benzo[1,2-d;4,5-d']bisthiazole (DTH-DBTH-TDTH-DSM) (Synthesis example 25) were obtained. The yield was 27 to 67%.

### (Synthesis example 26)

### Synthesis of P-THDT-DBTH-EH-IMTH

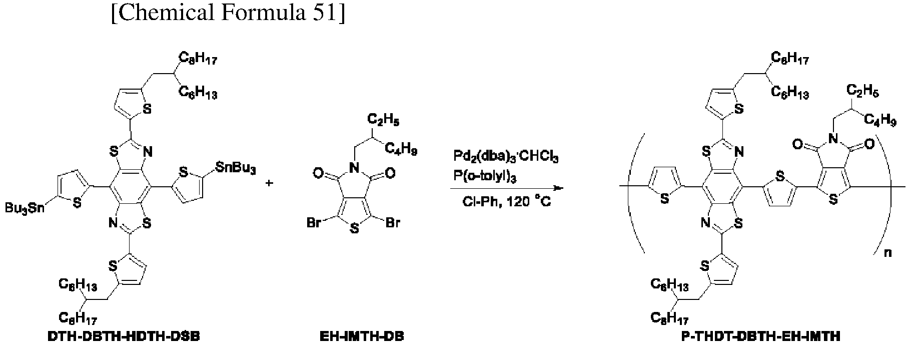

In a 20 mL flask, 2,6-bis[5-(2-hexyldecyl)thiophene-2-yl]-4,8-bis(5-tributylstannylthiophene-2-yl)-benzo[1,2-d;4,5-d']bisthiazole (DTH-DBTH-HDTH-DSB, 150 mg, 0.10 mmol), 1,3-dibromo-5-(2-ethylhexyl)thieno[3,4-c]pyrrolo-4,6-dione (EH-IMTH-DB, 41 mg, 0.10 mmol), a tris(dibenzylideneacetone)dipalladium (0)-chloroform adduct (4 mg, 3.9 µmol), tris(o-tolyl)phosphine (5 mg, 15.5 µmol), and chlorobenzene (12 mL) were added and caused to react at 120°C for 22 hours. After the end of the reaction, methanol (60 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 109 mg (91%) of P-THDT-DBTH-EH-IMTH as a black solid.

### (Synthesis example 27)

### Synthesis of P-THDT-DBTH-O-IMTH

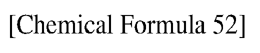

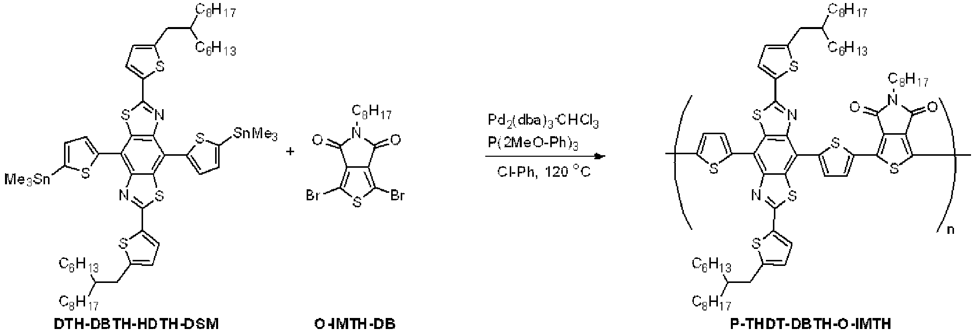

In a 20 mL flask, 2,6-bis[5-(2-hexyldecyl)thiophene-2-yl]-4,8-bis(5-trimethylstannylthiophene-2-yl)-benzo[1,2-d;4,5-d']bisthiazole (DTH-DBTH-HDTH-DSM, 90 mg, 0.07 mmol), 1,3-dibromo-5-octylthieno[3,4-c]pyrrolo-4,6-dione (O-IMTH-DB, 30 mg, 0.07 mmol), a tris(dibenzylideneacetone)dipalladium (0)-chloroform adduct (3 mg, 2.8 µmol), tris(2-methoxyphenyl)phosphine (4 mg, 11.1 µmol), and chlorobenzene (7 mL) were added and caused to react at 120°C for 24 hours. After the end of the reaction, methanol (50 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 74 mg (87%) of P-THDT-DBTH-O-IMTH as a black solid.

### (Synthesis example 28)

### Synthesis of P-TEHT-DBTH-HD-IMTH

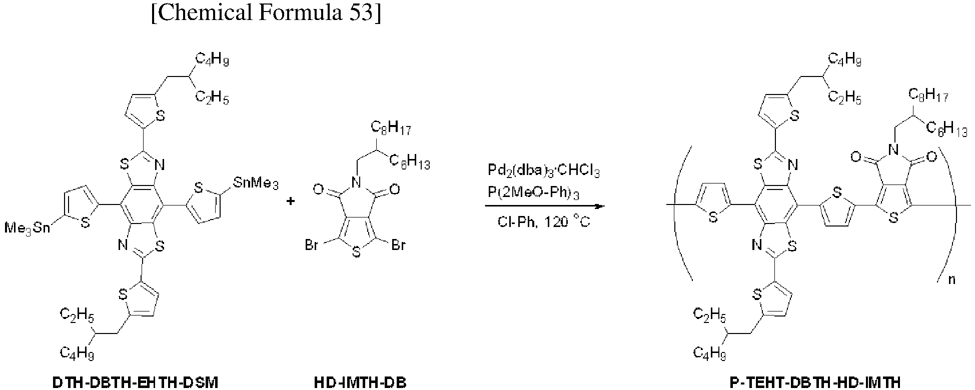

In a 20 mL flask, 2,6-bis[5-(2-hexyldecyl)thiophene-2-yl]-4,8-bis(5-trimethylstannylthiophene-2-yl)-benzo[1,2-d;4,5-d']bisthiazole (DTH-DBTH-EHTH-DSM, 100 mg, 0.09 mmol), 1,3-dibromo-5-(2-lhexyldecyl)thieno[3,4-c]pyrrolo-4,6-dione (HD-IMTH-DB, 50 mg, 0.09 mmol), a tris(dibenzylideneacetone)dipalladium (0)-chloroform adduct (4 mg, 3.7 µmol), tris(2-methoxyphenyl)phosphine (6 mg, 14.9 µmol), and chlorobenzene (7 mL) were added and caused to react at 120°C for 24 hours. After the end of the reaction, methanol (40 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 39 mg (37%) of P-TEHT-DBTH-HD-IMTH as a black solid.

### (Synthesis example 29)

### Synthesis of P-TBOT-DBTH-DMO-IMTH

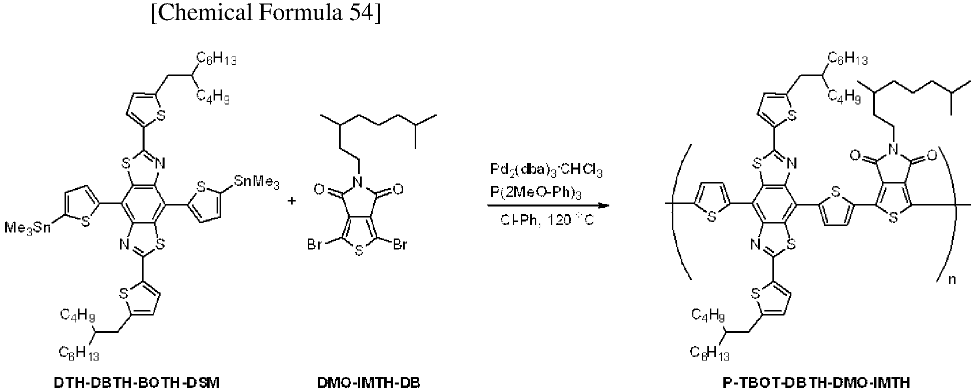

In a 20 mL flask, 2,6-bis[5-(2-butyloctyl)thiophene-2-yl]-4,8-bis(5-trimethylstannylthiophene-2-yl)-benzo[1,2-d;4,5-d']bisthiazole (DTH-DBTH-BOTH-DSM, 100 mg, 0.09 mmol), 1,3-dibromo-5-(3,7-dimethyloctyl)thieno[3,4-c]pyrrolo-4,6-dione (DMO-IMTH-DB, 38 mg, 0.09 mmol), a tris(dibenzylideneacetone)dipalladium (0)-chloroform adduct (4 mg, 3.6 µmol), tris(2-methoxyphenyl)phosphine (5 mg, 14.4 µmol), and chlorobenzene (8 mL) were added and caused to react at 120°C for 24 hours. After the end of the reaction, methanol (40 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 26 mg (27%) of P-TBOT-DBTH-DMO-IMTH as a black solid.

### (Synthesis example 30)

### Synthesis of P-THDT-DBTH-DMO-DPP

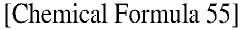

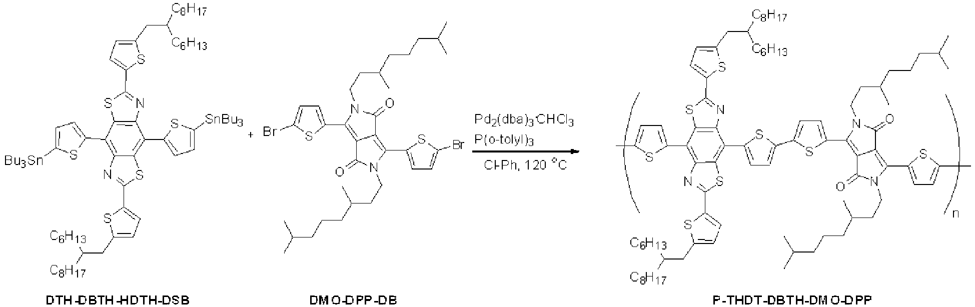

In a 20 mL flask, 2,6-bis[5-(2-hexyldecyl)thiophene-2-yl]-4,8-bis(5-tributylstannylthiophene-2-yl)-benzo[1,2-d;4,5-d']bisthiazole (DTH-DBTH-HDTH-DSB, 100 mg, 0.06 mmol), 3,6-bis (5-bromothiophen-2-yl)-2,5-(3,7-dimethyloctyl)-2,5-dihydropyrrolo [3,4-c]pyrrole-1,4-dione (DMO-DPP-DB, 49 mg, 0.06 mmol), a tris(dibenzylideneacetone)dipalladium (0)-chloroform adduct (3 mg, 2.6 µmol), tris(o-tolyl)phosphine (3 mg, 10.4 µmol), and chlorobenzene (10 mL) were added and caused to react at 120°C for 23 hours. After the end of the reaction, methanol (60 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 26 mg (26%) of P-THDT-DBTH-DMO-DPP as a black solid.

### (Synthesis example 31)

### Synthesis of P-THHDT-DBTH-HTT

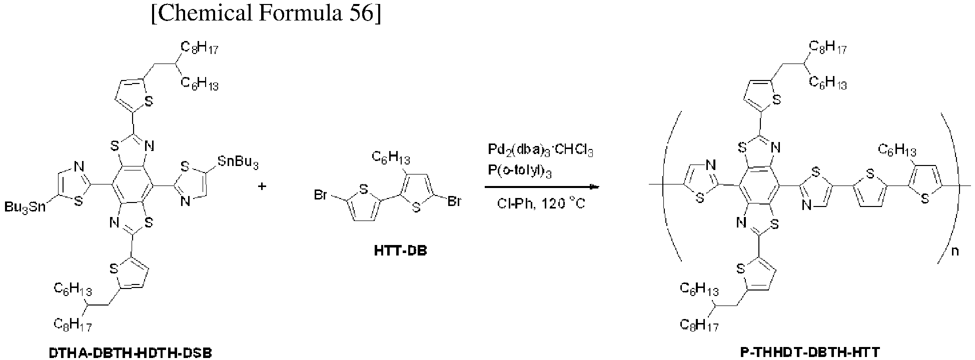

In a 20 mL flask, 2,6-bis[5-(2-hexyldecyl)thiophene-2-yl]-4,8-bis(5-tributylstannylthiazole-2-yl)-benzo[1,2-d;4,5-d']bisthiazole (DTHA-DBTH-HDTH-DSB, 120 mg, 0.08 mmol), 5,5'-Dibromo-3-hexyl[2,2']bithiophenyl (HTT-DB, 32 mg, 0.08 mmol), a tris(dibenzylideneacetone)dipalladium (0)-chloroform adduct (3 mg, 3.1 µmol), tris(o-tolyl)phosphine (4 mg, 12.3 µmol), and chlorobenzene (10 mL) were added and caused to react at 120°C for 24 hours. After the end of the reaction, methanol (60 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 72 mg (77%) of P-THHDT-DBTH-HTT as a black solid.

### (Synthesis example 32)

### Synthesis of P-THHDT-DBTH-EH-BDT

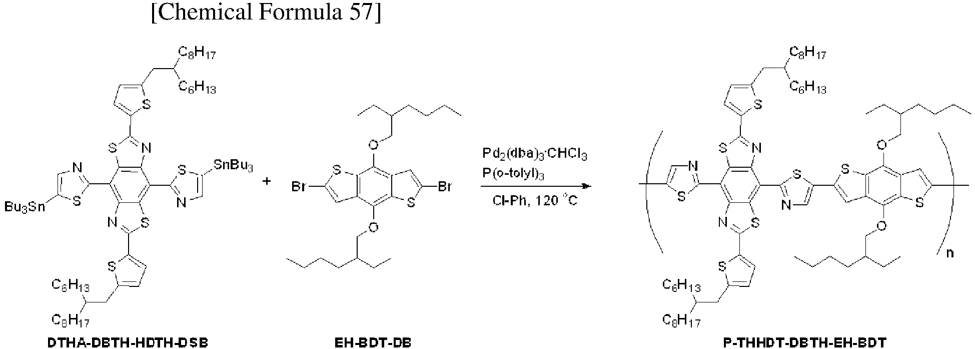

In a 20 mL flask, 2,6-bis[5-(2-hexyldecyl)thiophene-2-yl]-4,8-bis(5-tributylstannylthiazole-2-yl)-benzo[1,2-d;4,5-d']bisthiazole (DTHA-DBTH-HDTH-DSB, 120 mg, 0. 08 mmol), 2,6-Dibromo-4,8-bis[(2-ethylhexyloxy)-1,5-dithia-s-indacene (EH-BDT-DB, 47 mg, 0.08 mmol), a tris(dibenzylideneacetone)dipalladium (0)-chloroform adduct (3 mg, 3.1 µmol), tris(o-tolyl)phosphine (4 mg, 12.3 µmol), and chlorobenzene (10 mL) were added and caused to react at 120°C for 25 hours. After the end of the reaction, methanol (50 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 70 mg (64%) of P-THHDT-DBTH-EH-BDT as a dark red solid.

### (Synthesis example 33)

### Synthesis of P-THTIPSTH-DBTH-O-IMTH

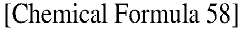

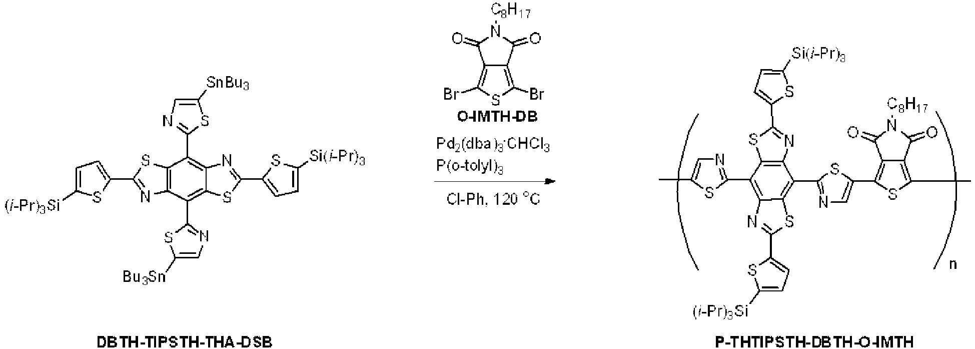

In a 20 mL flask, 4,8-bis(5-tributylstannylthiophene-2-yl)-2,6-bis(5-triisopropylsilanylthiophene-2-yl)-benzo[1,2-d;4,5-d']bisthiazole (DBTH-TIPSTH-THA-DSB, 88 mg, 0.06 mmol), 1,3-dibromo-5-octylthieno[3,4-c]pyrrolo-4,6-dione (O-IMTH-DB, 26 mg, 0.06 mmol), a tris(dibenzylideneacetone)dipalladium (0)-chloroform adduct (3 mg, 2.5 µmol), tris(o-tolyl)phosphine (4 mg, 10 µmol), and chlorobenzene (8 mL) were added and caused to react at 120°C for 24 hours. After the end of the reaction, methanol (50 mL) was added to the reaction solution, a precipitated solid was filtered and collected, and the obtained solid was subjected to Soxhlet washing (methanol, acetone, hexane). Subsequently, the obtained product was subjected to Soxhlet extraction (chloroform) to obtain 34 mg (50%) of P- THTIPSTH-DBTH-O-IMTH as a black solid.

### (Evaluation of absorption wavelength)

The obtained compound was dissolved in chlorobenzene, and was applied to glass (spin coating, coater, or the like) and then dried. The obtained dried product was subjected to ultraviolet-visible light absorption spectrum measurement by using an ultraviolet-visible spectrophotometer (manufactured by SHIMADZU CORPORATION, "UV-2450", "UV-3600iPlus").

### (Method for evaluating organic photodetecting element)

A 0.05027 mm square metal mask was adhered to an organic photodetecting element, and a solar simulator (OTENTO-SUN III, AM 1.5 G filter, radiation intensity of 100 mW/cm², manufactured by Bunkoukeiki Co., Ltd.) was used as an irradiation light source, and current-voltage characteristics between the ITO electrode and the aluminum electrode were measured by SourceMeter (manufactured by Keithley, Type 2400). A current density (dark current) Jd (mA/cm²) obtained when reverse voltage (V) was applied in a dark place, was calculated.

### <Example 1>

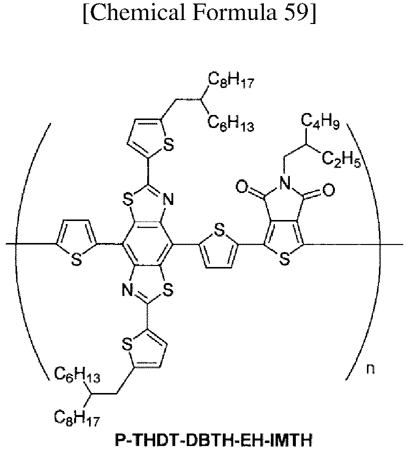

### (Preparation of mixture solution of p-type semiconductor compound and n-type semiconductor compound)

As the p-type semiconductor compound, a polymer compound having a structure of P-THDT-DBTH-EH-IMTH [Chemical Formula 59] was used.

As the n-type semiconductor compound, PC61BM (phenyl C61 butyric acid methyl ester, manufactured by Frontier Carbon Corporation, NS-E100H) was used. The p-type semiconductor compound and the n-type semiconductor compound at a weight ratio of 1:1.5 (a total concentration of 2.4 wt%), and 1,8-diiodooctane (0.03 mL/mL) were dissolved in chlorobenzene. The obtained solution was stirred and mixed on a hot stirrer at the temperature of 100°C for two hours or longer. The stirred and mixed solution was filtered through a 0.45 µm filter, and thus, a mixture solution of the p-type semiconductor compound and the n-type semiconductor compound was obtained.

### (Production of organic photodetector element)

A glass substrate (manufactured by GEOMATEC Co., Ltd.) patterned with an electrically conductive transparent film (anode) formed of indium tin oxide (ITO) was ultrasonically washed with acetone and then ultrasonically washed with ethanol, and was thereafter dried by nitrogen blowing.

After UV-ozone treatment was performed, 0.5 M of zinc acetate·0.5 M of aminoethanol/2-methoxyethanol solution used as an electron transport layer was applied (at 3000 rpm for 40 seconds) by a spin coater, and the obtained product was thereafter annealed at 175°C for 30 minutes.

The obtained product was put in a glove box, spin-coating of the mixture solution of the p-type semiconductor compound and the n-type semiconductor compound was performed in an inert gas atmosphere, and the obtained product was annealed or dried under reduced pressure on a hot plate.

Molybdenum oxide as a hole transport layer was deposited by a deposition machine. Thereafter, silver as an electrode was deposited, to obtain a reverse-structure-type device. The obtained device was evaluated for the organic photodetecting element as described above. As a result, a current density (dark current) Jd was 1×10⁻⁵ (mA/cm²) at a reverse voltage of -2 V (end absorption wavelength of 715 nm). A current density (photocurrent) Jd was 1 (mA/cm²) at the same voltage as that in light application. Therefore, the photocurrent/dark current at the reverse voltage of -2 V was 1×10⁵. At this time, the film thickness of the active layer was 547 nm.

### <Example 2>

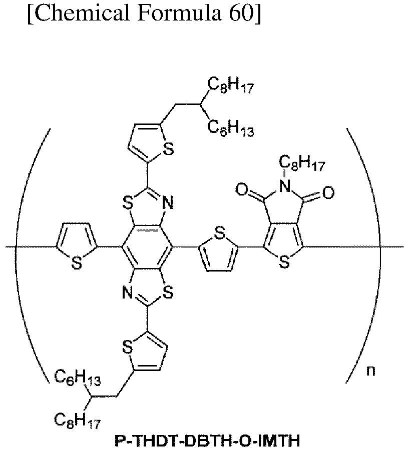

As the p-type semiconductor compound, a polymer compound having a structure of P-THDT-DBTH-O-IMTH [Chemical Formula 60] was used.

As the n-type semiconductor compound, PCBM(C61) was used. The p-type semiconductor compound and the n-type semiconductor compound at a weight ratio of 1:2 (a total concentration of 2.4 wt%), and 1,8-diiodooctane (0.03 mL/mL) were dissolved in chlorobenzene, and the obtained product was filtered through a 0.45 µm filter, to obtain a mixture solution. The obtained mixture solution was used to produce a reverse-structure-type device as in Example 1. The obtained device was evaluated for the organic photodetecting element as described above. As a result, a current density (dark current) Jd was 1×10⁻⁴ (mA/cm²) at a reverse voltage of -2 V (end absorption wavelength of 720 nm). A current density (photocurrent) Jd was 1×10¹ (mA/cm²) at the same voltage as that in light application. Therefore, the photocurrent/dark current at the reverse voltage of -2 V was 1×10⁵. At this time, the film thickness of the active layer was 547 nm.

### <Example 3>

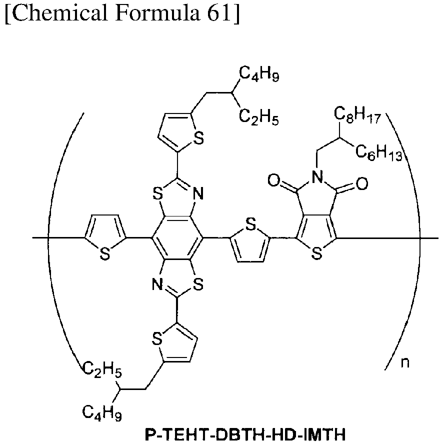

As the p-type semiconductor compound, a polymer compound having a structure of P-TEHT-DBTH-HD-IMTH [Chemical Formula 61] was used.

As the n-type semiconductor compound, PCBM(C61) was used. The p-type semiconductor compound and the n-type semiconductor compound at a weight ratio of 1:2 (a total concentration of 2.4 wt%), and 1,8-diiodooctane (0.03 mL/mL) were dissolved in chlorobenzene, and the obtained product was filtered through a 0.45 µm filter, to obtain a mixture solution. The obtained mixture solution was used to produce a reverse-structure-type device as in Example 1. The obtained device was evaluated for the organic photodetecting element as described above. As a result, a current density (dark current) Jd was 1×10⁻⁴ (mA/cm²) at a reverse voltage of -2 V (end absorption wavelength of 720 nm). A current density (photocurrent) Jd was 1×10¹ (mA/cm²) at the same voltage as that in light application. Therefore, the photocurrent/dark current at the reverse voltage of -2 V was 1×10⁵. At this time, the film thickness of the active layer was 547 nm.

### <Example 4>

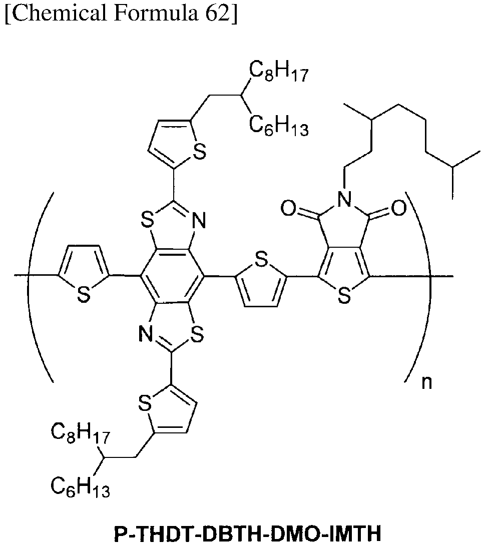

As the p-type semiconductor compound, a polymer compound having a structure of P-THDT-DBTH-DMO-IMTH [Chemical Formula 62] was used.

As the n-type semiconductor compound, PCBM(C61) was used. The p-type semiconductor compound and the n-type semiconductor compound at a weight ratio of 1:1 (a total concentration of 2.4 wt%), and 1,8-diiodooctane (0.03 mL/mL) were dissolved in chlorobenzene, and the obtained product was filtered through a 0.45 µm filter, to obtain a mixture solution. The obtained mixture solution was used to produce a reverse-structure-type device as in Example 1. The obtained device was evaluated for the organic photodetecting element as described above. As a result, a current density (dark current) Jd was 1×10⁻⁶ (mA/cm²) at a reverse voltage of -2 V (end absorption wavelength of 720 nm). A current density (photocurrent) Jd was 1×10⁻¹ (mA/cm²) at the same voltage as that in light application. Therefore, the photocurrent/dark current at the reverse voltage of -2 V was 1×10⁵. At this time, the film thickness of the active layer was 547 nm.

### <Example 5>

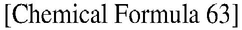

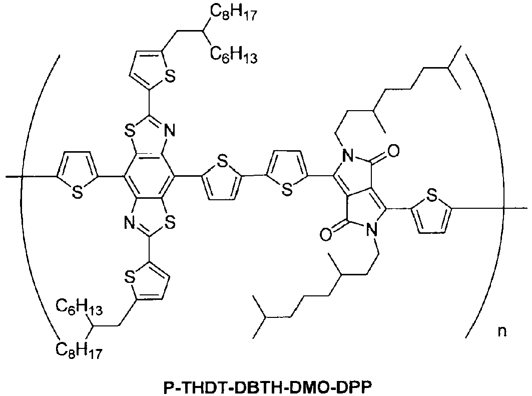

As the p-type semiconductor compound, a polymer compound having a structure of P-THDT-DBTH-DMO-DPP [Chemical Formula 63] was used.

As the n-type semiconductor compound, PCBM(C61) was used. The p-type semiconductor compound and the n-type semiconductor compound at a weight ratio of 1:2 (a total concentration of 2.4 wt%), and 1,8-diiodooctane (0.03 mL/mL) were dissolved in ortho-dichlorobenzene, and the obtained product was filtered through a 0.45 µm filter, to obtain a mixture solution. The obtained mixture solution was used to produce a reverse-structure-type device as in Example 1. The obtained device was evaluated for the organic photodetecting element as described above. As a result, a current density (dark current) Jd was 1×10⁻⁴ (mA/cm²) at a reverse voltage of -2 V (end absorption wavelength of 880 nm). A current density (photocurrent) Jd was 1×10¹ (mA/cm²) at the same voltage as that in light application. Therefore, the photocurrent/dark current at the reverse voltage of -2 V was 1×10⁵. At this time, the film thickness of the active layer was 547 nm.

### <Example 6>

According to the technique of Example 1, a mixture solution of the p-type semiconductor compound and the n-type semiconductor compound was used to produce a reverse-structure-type device. When the reverse-structure-type device was produced, the number of revolutions in spin-coating was made higher than that in Example 1, and the film thickness of the active layer was thus reduced. A current density (dark current) Jd was 1×10⁻³ (mA/cm²) at a reverse voltage of -2 V (end absorption wavelength of 880 nm). A current density (photocurrent) Jd was 1×10¹ (mA/cm²) at the same voltage as that in light application. Therefore, the photocurrent/dark current at the reverse voltage of -2 V was 1×10⁴. At this time, the film thickness of the active layer was 214 nm.

As described above, the organic photodetecting element produced by using the polymer compound used in the present invention allows a current amount (Jd) to be extremely lowered when a reverse voltage is applied in a dark place. The organic photodetecting element of the present invention is useful for health care (sensing in blood, etc.), security (various authentications), logistics (article management, etc.), and the like.

### DESCRIPTION OF THE REFERENCE CHARACTERS

(VII) organic photodetector
(VI) anode
(V) hole transport layer
(IV) active layer
(III) electron transport layer
(II) cathode
(I) base material

## Claims

1. An organic photodetector comprising
a structure in which a base material, a cathode, an active layer, and an anode are disposed in order, respectively, wherein
the active layer includes a polymer compound having a benzo(bis)thiazole structural unit represented by chemical formula (1), 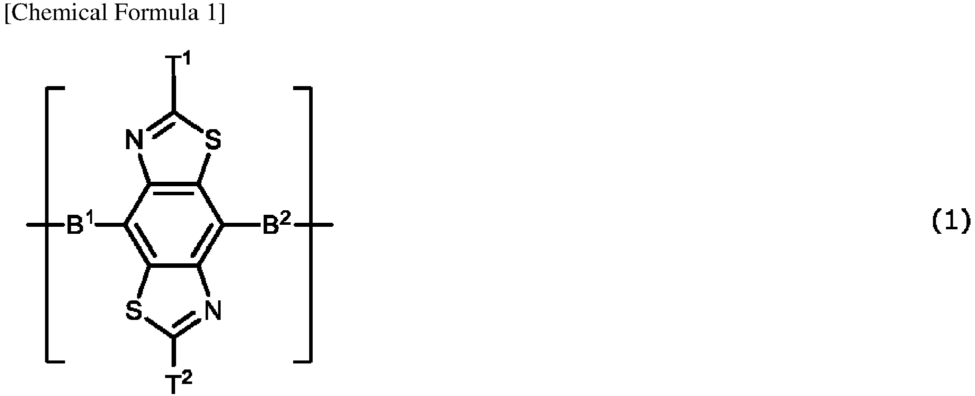 [in formula (1),
T¹ and T² each independently represent an alkoxy group, a thioalkoxy group, a thiophene ring that is optionally substituted with a hydrocarbon group or an organosilyl group, a thiazole ring that is optionally substituted with a hydrocarbon group or an organosilyl group, or a phenyl group that is optionally substituted with a hydrocarbon group, an alkoxy group, a thioalkoxy group, an organosilyl group, a halogen atom, or a trifluoromethyl group, and
B¹ and B² each represent a thiophene ring that is optionally substituted with a hydrocarbon group, a thiazole ring that is optionally substituted with a hydrocarbon group, or an ethynylene group.].

2. The organic photodetector according to claim 1, wherein T¹ and T² of the polymer compound each represent a group represented by one of the following formulas (t1) to (t5), 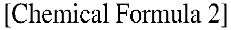 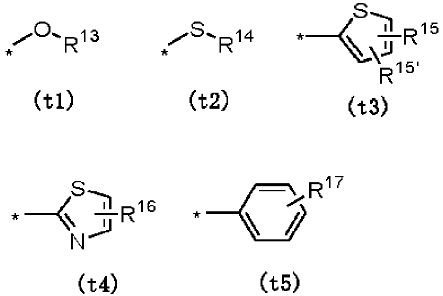 [in formulas (t1) to (t5),
R¹³ to R¹⁴ each independently represent a C6 to C30 hydrocarbon group,
R¹⁵ to R¹⁶ each independently represent a C6 to C30 hydrocarbon group or a group represented by *-Si(R¹⁸)₃, and R^{15'} represents a hydrogen atom, a C6 to C30 hydrocarbon group, or a group represented by *-Si(R¹⁸)₃,
R¹⁷ each independently represent a C6 to C30 hydrocarbon group, *-O-R¹⁹, *-S-R²⁰, *-Si(R¹⁸)₃, or *-CF₃,
R¹⁸ each independently represent a C1 to C20 aliphatic hydrocarbon group or a C6 to C10 aromatic hydrocarbon group, and a plurality of R¹⁸s may be the same or different from each other,
R¹⁹ to R²⁰ each represent a C6 to C30 hydrocarbon group, and
* represents a bond moiety for bonding to a thiazole ring of benzo(bis)thiazole.].

3. The organic photodetector according to claim 1 or 2, wherein B¹ and B² each represent a group represented by one of the following formulas (b1) to (b3), 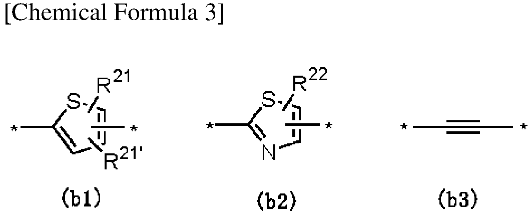 [in formulas (b1) to (b3), R²¹, R²², and R^{21'} each represent a hydrogen atom or a C6 to C30 hydrocarbon group, * represents a bond moiety, and, in particular, * on a left side represents a bond moiety for bonding to a benzene ring of a benzo(bis)thiazole compound.].

4. The organic photodetector according to any one of claims 1 to 3, wherein the polymer compound is a donor-acceptor semiconductor polymer.

5. The organic photodetector according to any one of claims 1 to 4, wherein the active layer further includes an n-type organic semiconductor compound.

6. The organic photodetector according to claim 5, wherein the n-type organic semiconductor compound is a fullerene or a derivative thereof.

7. The organic photodetector according to any one of claims 1 to 6, wherein a hole transport layer is disposed between the anode and the active layer.

8. The organic photodetector according to any one of claims 1 to 7, wherein an electron transport layer is disposed between the cathode and the active layer.

9. The organic photodetector according to any one of claims 1 to 8, wherein the cathode is a transparent electrode.

10. The organic photodetector according to any one of claims 1 to 9, wherein the anode is a metal electrode.
